(19) **Europäisches Patentamt / European Patent Office / Office européen des brevets**

(11) **EP 4 440 690 B1**

(12) **EUROPEAN PATENT SPECIFICATION**

(45) Date of publication and mention
of the grant of the patent:
**29.04.2026 Bulletin 2026/18**

(21) Application number: **22843971.7**

(22) Date of filing: **02.12.2022**

(51) International Patent Classification (IPC):
**A61N 1/39** *(2006.01)*    **A61N 1/362** *(2006.01)*
**A61N 1/37** *(2006.01)*    **A61N 1/378** *(2006.01)*

(52) Cooperative Patent Classification (CPC):
**A61N 1/3904; A61N 1/3912; A61N 1/3937;**
**A61N 1/3981;** A61N 1/3621; A61N 1/3625;
A61N 1/3706; A61N 1/378

(86) International application number:
**PCT/US2022/080792**

(87) International publication number:
**WO 2023/102509 (08.06.2023 Gazette 2023/23)**

(54) **ELECTROTHERAPEUTIC WAVEFORM AND PULSE GENERATOR**

GENERATOR FÜR ELEKTROTHERAPEUTISCHE WELLENFORMEN UND PULSEN

GÉNÉRATEUR DE FORMES D'ONDES ET D'IMPULSIONS ÉLECTROTHÉRAPEUTIQUES

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **03.12.2021 US 202163264875 P**

(43) Date of publication of application:
**09.10.2024 Bulletin 2024/41**

(73) Proprietor: **Zoll Medical Corporation**
**Chelmsford, MA 01824 (US)**

(72) Inventor: **BUTLER, Gideon D.H.**
**Portsmouth, NH 03801 (US)**

(74) Representative: **Potter Clarkson**
**Chapel Quarter**
**Mount Street**
**Nottingham NG1 6HQ (GB)**

(56) References cited:
WO-A1-2021/242882    US-A1- 2016 352 231
US-A1- 2017 252 571    US-A1- 2019 282 822
US-A1- 2020 246 628    US-A1- 2021 361 965
US-B1- 6 208 896

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## EP 4 440 690 B1

**Description**

**BACKGROUND**

Electrotherapies, which can include delivery of electrical energy or current to a patient, may be used, for example, in connection with treatments relating to organs such as the human heart. Normally, electro-chemical activity within a human heart causes the organ's muscle fibers to contract and relax in a synchronized manner. This synchronized action of the heart's musculature results in the effective pumping of blood from the ventricles throughout the body, including to the vital organs. Certain conditions can disrupt this normal electrochemical activity, synchronized action and effective pumping of the heart. These conditions include cardiac arrhythmias, which can cause the heart to beat irregularly or abnormally. Electrotherapies, including defibrillation and pacing, for example, can be useful or lifesaving in connection with conditions such as cardiac arrhythmias.

In particular, one of the most deadly cardiac arrhythmias is ventricular fibrillation, during which abnormal electrical activity within the heart causes the individual muscle fibers to contract in an unsynchronized and chaotic way. As a result of this loss of synchronization, the heart can rapidly lose its ability to effectively pump blood.

Defibrillation is a type of electrotherapy that may be used to treat conditions including ventricular fibrillation. A defibrillator may produce a large shock for delivery to a patient that disrupts the chaotic electrical activity of the heart associated with ventricular fibrillation, which can allow the heart's electro-chemical system to re-synchronize itself. Once organized electrical activity is restored, synchronized muscle contractions often follow, leading to the restoration of effective cardiac pumping. WO2021/242882A1 discloses a system for generating an electrotherapeutic pulse to be delivered to a patient. The system includes: an energy storage capacitor for providing electrotherapeutic current to the patient; a therapeutic current control network, electrically coupled to the energy storage capacitor, for controlling the electrotherapeutic current to be delivered to the patient, the therapeutic current control network comprising a resonant electrical circuit and at least one current control switch, wherein energy provided by the energy storage capacitor flows through the resonant electrical circuit; and a controller electrically coupled to the energy storage capacitor and the therapeutic current control network, wherein the controller is configured to, in connection with the energy provided by the energy storage capacitor that flows through the resonant electrical circuit, control operation of the at least one current control switch of the therapeutic current control network in delivering an electrotherapeutic waveform to the patient to correspond with a specified waveform.

**SUMMARY**

The invention is defined in claim 1. Further aspects and preferred embodiments are defined in the appended claims. The disclosure relates to electrotherapy waveform and pulse generation and delivery systems are described, such as for generation and delivery of defibrillation or pacing electrotherapeutic waveforms to patients, using open or closed loop current control. An example system includes a power supply, a therapeutic current control network including a resonant tank and at least one current control switch, and a controller. The controller may adjust operation of at least one current control switch in adjusting delivery of an electrotherapeutic waveform to the patient to correspond with a specified waveform. Therapeutic current control networks are described that are quasi-resonant and boost a voltage of an electrotherapeutic waveform delivered to the patient. Therapeutic current control networks are also described that may switch between a parallel resonance mode and a series resonance mode. Systems are described that may utilize one or more of soft switching, wide bandgap materials and a bidirectional power supply.

One aspect of the disclosure provides a system for generating an electrotherapeutic pulse to be delivered to a patient comprising: an energy storage capacitor for providing electrotherapeutic current to the patient; a therapeutic current control network, electrically coupled to the energy storage capacitor, for controlling the electrotherapeutic current to be delivered to the patient, the therapeutic current control network comprising a resonant electrical circuit and at least one current control switch, wherein energy provided by the energy storage capacitor flows through the resonant electrical circuit, the therapeutic current control network being configured to be operable in an operation mode of two modes of operation, the two modes comprising: a first mode in which the resonant electrical circuit is connected with the patient in a parallel configuration, and a second mode in which the resonant electrical circuit is connected with the patient in a series configuration; and a controller electrically coupled to the energy storage capacitor and the therapeutic current control network, wherein the controller is configured to, in connection with the energy provided by the energy storage capacitor that flows through the resonant electrical circuit, control operation of the at least one current control switch of the therapeutic current control network in delivering an electrotherapeutic waveform to the patient to correspond with a specified waveform.

In some examples, the resonant electrical circuit boosts a voltage of the electrotherapeutic pulse to be delivered to the patient. In some examples, electrical isolation of the patient from the energy storage capacitor is not included. In some examples, a transformer electrically connected between the energy storage capacitor and the patient is not required.

In some examples, the therapeutic current control network is configured such that the operation mode can be changed between the two modes of operation. In some examples, the controller is configured to be capable of changing of the operation mode between the two modes of operation. In some examples, the controller is configured to select the operation mode to be the first mode or the second mode. In some examples, the therapeutic current control network comprises at least one mode control switch. A configuration of the at least one mode control switch may determine the operation mode. In some examples, the controller is configured to select the operation mode to be the first mode or the second mode by controlling the configuration of the at least one mode control switch as open or closed. In some examples, the at least one mode control switch comprises two mode control switches. A configuration of each of the two mode control switch as open or closed my determine the operation mode. In some examples, the controller is configured to select the operation mode to be the first mode or the second mode by controlling the configuration of each of the at least two mode control switches as open or closed. In some examples, the controller is configured to select the operation mode to be the first mode or the second mode based at least in part on an impedance of the patient. In some examples, the controller is configured to select the operation mode as being the first mode or the second mode, based at least in part on the impedance of the patient. The first mode may be selected based at least in part on the impedance of the patient being at or above a threshold impedance. The second mode may be selected based at least in part on the impedance of the patient being below the threshold impedance.

In some examples, the controller is configured to select the operation mode. The threshold impedance may be a value between 40 ohms and 60 ohms or 50 ohms. In some examples, the controller is configured to select the operation mode to be the first mode or the second mode based at least in part on a voltage of the electrotherapeutic pulse to be delivered to the patient. In some examples, the controller is configured to select the operation mode to be the first mode or the second mode based at least in part on a voltage of the electrotherapeutic pulse to be delivered to the patient. The first mode may be selected based at least in part on the voltage of the electrotherapeutic pulse to be delivered to the patient being at or above a threshold voltage. The second mode may be selected based at least in part on the voltage of the electrotherapeutic pulse to be delivered to the patient being below the threshold voltage. In some examples, the system is configured such that, when the controller selects the operation mode to be the first mode, the configuration of the at least one mode control switch as open or closed causes the resonant electrical circuit to be connected with the patient in the parallel configuration, and, when the controller selects the operation mode to be the second mode, the configuration of the at least one mode control switch as open or closed causes the resonant electrical circuit to be connected with the patient in the series configuration.

In some examples, the energy storage capacitor and a first portion of the therapeutic current control network comprising the at least one current control switch and the resonant electrical circuit are connected via a first node and a second node; the first portion of the therapeutic current control network and a second portion of the therapeutic current control network comprising at least one mode control switch are connected via a third node and a fourth node. A configuration of the at least one mode control switch may determine the operation mode; the second portion of the therapeutic current control network and a third portion of the therapeutic current control network comprising a rectifier are connected via a fifth node and a sixth node; the third portion of the therapeutic current control network and a fourth portion of the therapeutic current control network comprising a filter are connected via a seventh node and an eighth node; and the fourth portion of the therapeutic current control network and a fifth portion of the therapeutic current control network, comprising at least one polarity control switch, a patient load, and at least one sensor configured to sense at least one electrical parameter from which current flow to the patient can be determined or estimated, are connected via a ninth node and a tenth node.

In some examples, the at least one current control switch comprises two switches. In some examples, the at least one current control switch comprises four switches. In some examples, the first portion of the therapeutic current control network comprises at least one circuit component configured to reduce common-mode noise. In some examples, the at least one circuit component configured to reduce common-mode noise comprises at least one inductor and at least one capacitor. In some examples, the at least one sensor is configured to sense a current parameter. In some examples, the at least one sensor is configured to sense a voltage parameter. In some examples, the controller comprises at least one processor.

In some examples, the resonant electrical circuit comprises a resonant tank. In some examples, the resonant tank is a two element resonant tank. In some examples, the two element resonant tank comprises an inductor and a capacitor. In some examples, resonant tank is a three element resonant tank. In some examples, the resonant tank is an LCC resonant tank. In some examples, the resonant tank is a CLL resonant tank. In some examples, the resonant tank is an LLC resonant tank. In some examples, the resonant tank is a four element resonant tank. In some examples, the resonant electrical circuit is an LCLC resonant tank.

In some examples, the rectifier comprises a switching network configured to be controlled to perform synchronous rectification. In some examples, the rectifier comprises a diode network configured to be controlled to perform passive rectification. In some examples, the at least one polarity control switch is configured for use in controlling direction of current flow. In some examples, the at least one polarity control switch comprises an H-bridge. In some examples, the sensed at least one electrical parameter comprises a current parameter. In some examples, the sensed at least one electrical parameter comprises a voltage parameter. In some examples, the rectifier is configured for use in conversion of alternating current to direct current. In some examples, the filter is configured for use in electrical signal frequency filtering.

In some examples, the system includes at least one sensor configured to sense at least one electrical parameter from which current flow to the patient can be determined or estimated. The controller may be configured to: process a signal associated with the sensed at least one electrical parameter; compare the processed signal with a second signal associated with the specified waveform; and control operation of the at least one current control switch of the therapeutic current control network in adjusting delivery of the electrotherapeutic waveform to the patient to correspond with the specified waveform.

In some examples, the at least one current control switch comprises a plurality of switches. The control of the operation of the at least one current control switch of the therapeutic current control network in the adjusting of the delivery of the electrotherapeutic waveform to the patient to correspond with the specified waveform may comprise controlling a configuration of each of the plurality of switches as open or closed.

In some examples, the system includes a battery and a bidirectional charging control network. The battery and the bidirectional charging control network may be connected via a first node and a second node. The bidirectional charging control network and the energy storage capacitor may be connected via a third node and a fourth node. The bidirectional charging control network may be for controlling energy flow from the battery to the energy storage capacitor for storage by the energy storage capacitor, and from the energy storage capacitor to the battery for storage by the battery. In some examples, the bidirectional charging control network comprises a second resonant electrical circuit.

In some examples, a computerized mobile device comprises the system. The computerized mobile device may be configured to deliver the electrotherapeutic pulse to the patient. In some examples, the computerized mobile device is configured to deliver the electrotherapeutic pulse to the patient. The electrotherapeutic pulse may have an energy of between 1 - 400 joules. In some examples, the computerized mobile device is configured to deliver the electrotherapeutic pulse to the patient. The electrotherapeutic pulse may have an energy of between 50 - 200 joules. In some examples, the computerized mobile device has an energy density, given by an energy of the electrotherapeutic pulse to be delivered to the patient relative to a volume defined by a housing of the mobile device, of between 0.5 - 1.00 joules per cubic centimeter. In some examples, the computerized mobile device has an energy density, given by an energy of the electrotherapeutic pulse to be delivered to the patient relative to a volume defined by a housing of the mobile device, of between 1.00 - 2.00 joules per cubic centimeter.

In some examples, the computerized mobile device has an energy density, given by an energy of the electrotherapeutic pulse to be delivered to the patient relative to a volume defined by a housing of the mobile device, of between 2.00 - 3.00 joules per cubic centimeter. In some examples, the computerized mobile device has an energy density, given by an energy of the electrotherapeutic pulse to be delivered to the patient relative to a volume defined by a housing of the mobile device, of between 3.00 - 4.00 joules per cubic centimeter. In some examples, the computerized mobile device has a volume, defined by a housing of the mobile device, of between 400 - 1,200 cubic centimeters. In some examples, the computerized mobile device has a volume, defined by a housing of the mobile device, of between 200 - 800 cubic centimeters. In some examples, the computerized mobile device has a volume, defined by a housing of the mobile device, of between 100 - 400 cubic centimeters.

In some examples, the electrotherapeutic waveform is for defibrillation. In some examples, the electrotherapeutic waveform is for pacing. In some examples, the therapeutic current control network comprises a patient relay circuit configured for use in control of allowing current flow through a patient load. The patient relay circuit may comprise at least one patient relay switch comprising a wide bandgap material. In some examples, a peak energy efficiency of the system in delivering the electrotherapeutic pulse to the patient is at least 80 percent. In some examples, the electrotherapeutic pulse delivered to the patient delivers an amount of energy to the patient that is no more than 15 percent different than an amount of energy of the specified waveform. In some examples, the specified waveform is a biphasic rectilinear current waveform.

In some examples, the operation mode is selected based at least in part on maximizing a value of a Q factor. The value of the Q factor may represent a measure of peak energy relative to dissipated energy with regard to the resonant electrical circuit. The value of the Q factor may be affected by the operation mode and a patient impedance.

One example provides an exemplary method for generating an electrotherapeutic pulse for delivery to a patient, the method comprising: specifying a desired electrotherapeutic waveform for delivery to the patient. Energy for electrotherapy current may be provided by an energy storage capacitor; and operating a resonant therapeutic current control network, comprising at least one current control switch and a resonant tank, in controlling an electrotherapeutic waveform being delivered to the patient to correspond to the desired electrotherapeutic waveform, Energy provided by the energy storage capacitor may flow through the resonant tank, comprising operating the therapeutic current control network in an operation mode of two modes of operation, the two modes comprising: a first mode in which the resonant tank is connected with the patient in a parallel configuration, and a second mode in which the resonant tank is connected with the patient in a series configuration.

Some examples include operating of the resonant therapeutic current control network in controlling the electrotherapeutic waveform being delivered to the patient. The resonant tank may boost a voltage of the electrotherapeutic waveform being delivered to the patient. Some examples include operating the resonant therapeutic current control network in controlling the electrotherapeutic waveform being delivered to the patient without electrical isolation of the patient from the energy storage capacitor. Some examples include operating the resonant therapeutic current control network in controlling the electrotherapeutic waveform being delivered to the patient without requiring a transformer electrically connected between the energy storage capacitor and the patient.

Some examples include a controller selecting the operation mode as the first mode or the second mode. Some examples include a controller selecting the operation mode as the first mode or the second mode by controlling a configuration of at least one mode control switch. The configuration of the at least one mode control switch may determine the operation mode. Some examples include the controller selecting the operation mode as the first mode or the second mode by controlling a configuration of at least one mode control switch as open or closed. Some examples include the controller selecting the operation mode as the first mode or the second mode based at least in part on an impedance of the patient. Some examples include the controller selecting the operation mode as the first mode or the second mode based at least in part on an impedance of the patient. The controller may select the first mode based at least in part on the impedance of the patient being at or above a threshold impedance. The controller may select the second mode based at least in part on the impedance of the patient being below the threshold impedance. Some examples include the controller selecting the operation mode. The threshold impedance may be a value between 40 ohms and 60 ohms or 50 ohms.

Some examples include the controller selecting the operation mode as the first mode or the second mode based at least in part on a voltage of the electrotherapeutic waveform to be delivered to the patient. Some examples include the controller selecting the operation mode as the first mode or the second mode based at least in part on a voltage of the electrotherapeutic pulse to be delivered to the patient. The controller may select the first mode based at least in part on the voltage of the electrotherapeutic pulse to be delivered to the patient being at or above a threshold voltage. The controller may select the second mode based at least in part on the voltage of the electrotherapeutic pulse to be delivered to the patient being below the threshold voltage. Some examples include the controller selecting the operation mode as the first mode or the second mode such that, when the controller selects the operation mode to be the first mode, the configuration of the at least one mode control switch as open or closed causes the resonant tank to be connected with the patient in the parallel configuration, and, when the controller selects the operation mode to be the second mode, the configuration of the at least one mode control switch as open or closed causes the resonant tank to be connected with the patient in a series configuration.

Some examples include: determining a current at a point in the resonant therapeutic current control network from which a current flow to the patient can be determined or estimated; and comparing the current flow to the patient to the desired electrotherapeutic waveform. The operating of the resonant therapeutic current control network may comprise operating the at least one current control switch in adjusting the electrotherapeutic waveform being delivered to the patient to match the desired electrotherapeutic waveform.

In some examples, the method is performed by a defibrillator. In some examples, the operating of the therapeutic current control network comprises staggering a time of initiation of current switching relative to a time of initiation of voltage switching, the voltage switching being associated with the current switching, so as to reduce switching losses relative to substantially simultaneous initiation of the current switching and the associated voltage switching. In some examples, timing of voltage switching waveforms relative to associated current switching waveforms is controlled so as to minimize switching losses. Some examples include operating the therapeutic current control network to employ at least one of: substantially zero voltage switching (ZVS) and substantially zero current switching (ZCS). Some examples include operating the therapeutic current control network to employ closed-loop control of the electrotherapeutic waveform being delivered to the patient. Some examples include operating the therapeutic current control network to continuously adjust, over time, delivery of the electrotherapeutic waveform to the patient. In some examples, specifying the desired electrotherapeutic waveform for delivery to the patient comprises specifying a current waveform.

Some examples include charging the energy storage capacitor using a battery electrically coupled with the energy storage capacitor, and comprising returning at least a portion of energy stored by the energy storage capacitor to the battery for storage by the battery using a bidirectional charging control network, the bidirectional charging control network being electrically coupled to the battery and the energy storage capacitor. Some examples include using a bidirectional charging control network in transferring energy from the energy storage capacitor to a battery, and from the battery to the energy storage capacitor. The battery and the energy storage capacitor may be electrically coupled. The energy storage capacitor may be for providing energy for the electrotherapeutic waveform being delivered to the patient. In some examples, using the bidirectional charging control network comprises using the bidirectional charging control network comprising a first resonant tank. In some examples, generating the electrotherapeutic pulse for delivery to the patient is for defibrillation. In some examples, generating the electrotherapeutic pulse for delivery to the patient is for pacing.

In some examples, the at least one current control switch comprises a plurality of switches, and comprising controlling a configuration of each of the plurality of switches of the plurality of switches as open or closed in adjusting delivery of the electrotherapeutic waveform being delivered to the patient to match the desired electrotherapeutic waveform.

In some examples, the operation mode is selected based at least in part on maximizing a value of a Q factor. The value of the Q factor represents a measure of peak energy relative to dissipated energy with regard to the resonant tank. The value of the Q factor is affected by the operation mode and a patient impedance.

One aspect provides a system for generating an electrotherapeutic pulse to be delivered to a patient, the system comprising: an energy storage capacitor for providing electrotherapeutic current to the patient; a therapeutic current control network, electrically coupled to the energy storage capacitor, for controlling the electrotherapeutic current to be delivered to the patient, the therapeutic current control network comprising a resonant electrical circuit, the resonant electrical circuit being connected with the patient in a parallel configuration, and at least one current control switch, wherein energy provided by the energy storage capacitor flows through the resonant electrical circuit; and a controller electrically coupled to the energy storage capacitor and the therapeutic current control network, wherein the controller is configured to, in connection with the energy provided by the energy storage capacitor that flows through the resonant electrical circuit, control operation of the at least one current control switch of the therapeutic current control network in delivering an electrotherapeutic waveform to the patient to correspond with a specified waveform.

In some examples, the resonant electrical circuit boosts a voltage of the electrotherapeutic pulse to be delivered to the patient. In some examples, electrical isolation of the patient from the energy storage capacitor is not included. In some examples, a transformer electrically connected between the energy storage capacitor and the patient is not required.

In some examples, the energy storage capacitor and a first portion of the therapeutic current control network comprising the at least one current control switch and the resonant electrical circuit are connected via a first node and a second node; the first portion of the therapeutic current control network and a second portion of the therapeutic current control network comprising a rectifier are connected via a third node and a fourth node; the second portion of the therapeutic current control network and a third portion of the therapeutic current control network comprising a filter are connected via a fifth node and an sixth node; and the third portion of the therapeutic current control network and a fourth portion of the therapeutic current control network, comprising at least one polarity control switch, a patient load, and at least one sensor configured to sense at least one electrical parameter from which current flow to the patient can be determined or estimated, are connected via a seventh node and an eighth node.

In some examples, the at least one current control switch comprises two switches. In some examples, the at least one current control switch comprises four switches. In some examples, the first portion of the therapeutic current control network comprises at least one circuit component configured to reduce common-mode noise. In some examples, the at least one circuit component configured to reduce common-mode noise comprises at least one inductor and at least one capacitor. In some examples, the at least one sensor is configured to sense a current parameter. In some examples, at least one sensor is configured to sense a voltage parameter. In some examples, the controller comprises at least one processor.

In some examples, the resonant electrical circuit comprises a resonant tank. In some examples, the resonant tank is a two element resonant tank. In some examples, the two element resonant tank comprises an inductor and a capacitor. In some examples, the resonant tank is a three element resonant tank. In some examples, the resonant tank is an LCC resonant tank. In some examples, the resonant tank is a CLL resonant tank. In some examples, the resonant tank is an LLC resonant tank. In some examples, the resonant tank is a four element resonant tank. In some examples, the resonant electrical circuit is an LCLC resonant tank.

In some examples, the rectifier comprises a switching network configured to be controlled to perform synchronous rectification. In some examples, the rectifier comprises a diode network configured to be controlled to perform passive rectification. In some examples, the at least one polarity control switch is configured for use in controlling direction of the current flow. In some examples, the at least one polarity control switch comprises an H-bridge. In some examples, the sensed at least one electrical parameter comprises a current parameter. In some examples, the sensed at least one electrical parameter comprises a voltage parameter. In some examples, the rectifier is configured for use in conversion of alternating current to direct current. In some examples, the filter is configured for use in electrical signal frequency filtering.

Some examples include at least one sensor configured to sense at least one electrical parameter from which current flow to the patient can be determined or estimated. The controller may be configured to: process a signal associated with the sensed at least one electrical parameter; compare the processed signal with a second signal associated with the specified waveform; and control operation of the at least one current control switch of the therapeutic current control network in adjusting delivery of the electrotherapeutic waveform to the patient to correspond with the specified waveform. In some examples, the at least one current control switch comprises a plurality of switches. The control of the operation of the at least one current control switch of the therapeutic current control network in the adjusting of the delivery of the electrotherapeutic waveform to the patient to correspond with the specified waveform may comprise controlling a configuration of each of the plurality of switches as open or closed.

Some examples include a battery and a bidirectional charging control network. The battery and the bidirectional charging control network may be connected via a first node and a second node. The bidirectional charging control network and the energy storage capacitor may be connected via a third node and a fourth node. The bidirectional charging control network may be for controlling energy flow from the battery to the energy storage capacitor for storage by the energy storage capacitor, and from the energy storage capacitor to the battery for storage by the battery. In some examples, the bidirectional charging control network comprises a second resonant electrical circuit.

In some examples, a computerized mobile device comprises the system. The computerized mobile device may be configured to deliver the electrotherapeutic pulse to the patient. In some examples, the computerized mobile device is configured to deliver the electrotherapeutic pulse to the patient. The electrotherapeutic pulse may have an energy of between 1 - 400 joules. In some examples, the computerized mobile device is configured to deliver the electrotherapeutic pulse to the patient. The electrotherapeutic pulse may have an energy of between 50 - 200 joules. In some examples, the computerized mobile device has an energy density, given by an energy of the electrotherapeutic pulse to be delivered to the patient relative to a volume defined by a housing of the mobile device, of between 0.5 - 1.00 joules per cubic centimeter. In some examples, the computerized mobile device has an energy density, given by an energy of the electrotherapeutic pulse to be delivered to the patient relative to a volume defined by a housing of the mobile device, of between 1.00 - 2.00 joules per cubic centimeter. In some examples, the computerized mobile device has an energy density, given by an energy of the electrotherapeutic pulse to be delivered to the patient relative to a volume defined by a housing of the mobile device, of between 2.00 - 3.00 joules per cubic centimeter. In some examples, the computerized mobile device has an energy density, given by an energy of the electrotherapeutic pulse to be delivered to the patient relative to a volume defined by a housing of the mobile device, of between 3.00 - 4.00 joules per cubic centimeter. In some examples, the computerized mobile device has a volume, defined by a housing of the mobile device, of between 400 - 1,200 cubic centimeters. In some examples, the computerized mobile device has a volume, defined by a housing of the mobile device, of between 200 - 800 cubic centimeters. In some examples, the computerized mobile device has a volume, defined by a housing of the mobile device, of between 100 - 400 cubic centimeters.

In some examples, the electrotherapeutic waveform is for defibrillation. In some examples, the electrotherapeutic waveform is for pacing. In some examples, the therapeutic current control network comprises a patient relay circuit configured for use in control of allowing current flow through a patient load. The patient relay circuit may comprise at least one patient relay switch comprising a wide bandgap material.

In some examples, a peak energy efficiency of the system in delivering the electrotherapeutic pulse to the patient is at least 80 percent. In some examples, the electrotherapeutic pulse delivered to the patient delivers an amount of energy to the patient that is no more than 15 percent different than an amount of energy of the specified waveform. In some examples, the specified waveform is a biphasic rectilinear current waveform. One aspect provides a system for generating an electrotherapeutic pulse to be delivered to a patient, the system comprising: an energy storage capacitor for providing electrotherapeutic current to the patient; a therapeutic current control network, electrically coupled to the energy storage capacitor, for controlling the electrotherapeutic current to be delivered to the patient, the therapeutic current control network comprising a resonant electrical circuit and at least one current control switch. Energy provided by the energy storage capacitor flows through the resonant electrical circuit. The resonant electrical circuit may be configured to boost a voltage of the electrotherapeutic pulse to be delivered to the patient; and a controller electrically coupled to the energy storage capacitor and the therapeutic current control network. The controller may be configured to, in connection with the energy provided by the energy storage capacitor that flows through the resonant electrical circuit, control operation of the at least one current control switch of the therapeutic current control network in delivering an electrotherapeutic waveform to the patient to correspond with a specified waveform.

In some examples, the control of the operation of the at least one current control switch in delivering the electrotherapeutic waveform to the patient to correspond with the specified waveform comprises controlling a switching frequency of the at least one current control switch. In some examples, the at least one current control switch comprises at least one MOSFET. In some examples, the resonant electrical circuit is connected with the patient in a parallel configuration.

In some examples, the energy storage capacitor and a first portion of the therapeutic current control network comprising an inductor and are connected via a first node, the first portion of the therapeutic current control network and a second portion of the therapeutic current control network comprising the resonant electrical circuit and a rectifier are connected via a second node, and the second portion of the therapeutic current control network and a third portion of the therapeutic current control network comprising a capacitor and a patient load are connected via a third node. In some examples, the at least one current control switch is connected with at least a portion of the resonant electrical circuit in a series configuration.

Some examples include at least one sensor configured to sense at least one electrical parameter from which current flow to the patient can be determined or estimated. The controller may be configured to: process a signal associated with the sensed at least one electrical parameter; compare the processed signal with a second signal associated with the specified waveform; and control operation of the at least one current control switch of the therapeutic current control network in adjusting delivery of the electrotherapeutic waveform to the patient to correspond with the specified waveform.

Some examples include a battery and a bidirectional charging control network.

The battery and the bidirectional charging control network may be connected via a first node and a second node. The bidirectional charging control network and the energy storage capacitor may be connected via a third node and a fourth node. The bidirectional charging control network may be for controlling energy flow from the battery to the energy storage capacitor for storage by the energy storage capacitor, and from the energy storage capacitor to the battery for storage by the battery. In some examples, the rectifier is configured for use in conversion of alternating current to direct current. In some examples, the bidirectional charging control network comprises a second resonant electrical circuit.

In some examples, the resonant electrical circuit comprises a resonant tank. In some examples, the resonant tank is a two element resonant tank. In some examples, the two element resonant tank comprises an inductor and a capacitor. In some examples, the resonant tank is a three element resonant tank. In some examples, the resonant tank is an LCC resonant tank. In some examples, the resonant tank is a CLL resonant tank. In some examples, the resonant tank is an LLC resonant tank. In some examples, the resonant tank is a four element resonant tank. In some examples, the resonant electrical circuit is an LCLC resonant tank.

In some examples, a computerized mobile device comprises the system. The computerized mobile device may be configured to deliver the electrotherapeutic pulse to the patient. In some examples, the computerized mobile device is configured to deliver the electrotherapeutic pulse to the patient. The electrotherapeutic pulse may have an energy of between 1 - 400 joules. In some examples, the computerized mobile device is configured to deliver the electrotherapeutic pulse to the patient. The electrotherapeutic pulse may have an energy of between 50 - 200 joules. In some examples, the computerized mobile device has an energy density, given by an energy of the electrotherapeutic pulse to be delivered to the patient relative to a volume defined by a housing of the mobile device, of between 0.5 - 1.00 joules per cubic centimeter. In some examples, the computerized mobile device has an energy density, given by an energy of the electrotherapeutic pulse to be delivered to the patient relative to a volume defined by a housing of the mobile device, of between 1.00 - 2.00 joules per cubic centimeter. In some examples, the computerized mobile device has an energy density, given by an energy of the electrotherapeutic pulse to be delivered to the patient relative to a volume defined by a housing of the mobile device, of between 2.00 - 3.00 joules per cubic centimeter.

In some examples, the computerized mobile device has an energy density, given by an energy of the electrotherapeutic pulse to be delivered to the patient relative to a volume defined by a housing of the mobile device, of between 3.00 - 4.00 joules per cubic centimeter. In some examples, the computerized mobile device has a volume, defined by a housing of the mobile device, of between 400 - 1,200 cubic centimeters. In some examples, the computerized mobile device has a volume, defined by a housing of the mobile device, of between 200 - 800 cubic centimeters. In some examples, the computerized mobile device has a volume, defined by a housing of the mobile device, of between 100 - 400 cubic centimeters.

In some examples, the controller comprises at least one processor. In some examples, the electrotherapeutic waveform is for defibrillation. In some examples, the electrotherapeutic waveform is for pacing. In some examples, the therapeutic current control network comprises a patient relay circuit configured for use in control of allowing current flow through a patient load. The patient relay circuit may comprise at least one patient relay switch comprising a wide bandgap material.

In some examples, a peak energy efficiency of the system in delivering the electrotherapeutic pulse to the patient is at least 80 percent. In some examples, the electrotherapeutic pulse delivered to the patient delivers an amount of energy to the patient that is no more than 15 percent different than an amount of energy of the specified waveform. In some examples, the specified waveform is a biphasic rectilinear current waveform.

One aspect provides a system for generating an electrotherapeutic pulse to be delivered to a patient, the system comprising: an energy storage capacitor for providing electrotherapeutic current to the patient; a therapeutic current control network, electrically coupled to the energy storage capacitor, for controlling the electrotherapeutic current to be delivered to the patient, the therapeutic current control network comprising a resonant electrical circuit and at least one current control switch, wherein energy provided by the energy storage capacitor flows through the resonant electrical circuit, wherein: the energy storage capacitor and a first portion of the therapeutic current control network comprising an inductor are connected via a first node, the first portion of the therapeutic current control network and a second portion of the therapeutic current control network comprising the resonant electrical circuit and a rectifier are connected via a second node, and the second portion of the therapeutic current control network and a third portion of the therapeutic current control network comprising a capacitor and a patient load are connected via a third node; and a controller electrically coupled to the energy storage capacitor and the therapeutic current control network, wherein the controller is configured to, in connection with the energy provided by the energy storage capacitor that flows through the resonant electrical circuit, control operation of the at least one current control switch of the therapeutic current control network in delivering an electrotherapeutic waveform to the patient to correspond with a specified waveform.

In some examples, the at least one current control switch is connected with at least a portion of the resonant electrical circuit in a series configuration. In some examples, the resonant electrical circuit is configured to boost a voltage of the electrotherapeutic pulse to be delivered to the patient. In some examples, the control of the operation of the at least one current control switch in delivering the electrotherapeutic waveform to the patient to correspond with the specified waveform comprises controlling a switching frequency of the at least one current control switch. In some examples, the at least one current control switch comprises at least one MOSFET. In some examples, the resonant electrical circuit is connected with the patient in a parallel configuration.

Some examples include a battery and a bidirectional charging control network. The battery and the bidirectional charging control network may be connected via a first node and a second node. The bidirectional charging control network and the energy storage capacitor may be connected via a third node and a fourth node. The bidirectional charging control network may be for controlling energy flow from the battery to the energy storage capacitor for storage by the energy storage capacitor, and from the energy storage capacitor to the battery for storage by the battery. In some examples, the rectifier is configured for use in conversion of alternating current to direct current. In some examples, the bidirectional charging control network comprises a second resonant electrical circuit.

In some examples, the resonant electrical circuit comprises a resonant tank. In some examples, the resonant tank is a two element resonant tank. In some examples, the two element resonant tank comprises an inductor and a capacitor. In some examples, the resonant tank is a three element resonant tank. In some examples, the resonant tank is an LCC resonant tank. In some examples, the resonant tank is a CLL resonant tank. In some examples, the resonant tank is an LLC resonant tank. In some examples, the resonant tank is a four element resonant tank. In some examples, the resonant electrical circuit is an LCLC resonant tank.

In some examples, a computerized mobile device comprises the system. The computerized mobile device may be configured to deliver the electrotherapeutic pulse to the patient. In some examples, the computerized mobile device is configured to deliver the electrotherapeutic pulse to the patient. The electrotherapeutic pulse may have an energy of between 1 - 400 joules. In some examples, the computerized mobile device is configured to deliver the electrotherapeutic pulse to the patient. The electrotherapeutic pulse may have an energy of between 50 - 200 joules. In some examples, the computerized mobile device has an energy density, given by an energy of the electrotherapeutic pulse to be delivered to the patient relative to a volume defined by a housing of the mobile device, of between 0.5 - 1.00 joules per cubic centimeter.

In some examples, the computerized mobile device has an energy density, given by an energy of the electrotherapeutic pulse to be delivered to the patient relative to a volume defined by a housing of the mobile device, of between 1.00 - 2.00 joules per cubic centimeter. In some examples, the computerized mobile device has an energy density, given by an energy of the electrotherapeutic pulse to be delivered to the patient relative to a volume defined by a housing of the mobile device, of between 2.00 - 3.00 joules per cubic centimeter. In some examples, the computerized mobile device has an energy density, given by an energy of the electrotherapeutic pulse to be delivered to the patient relative to a volume defined by a housing of the mobile device, of between 3.00 - 4.00 joules per cubic centimeter. In some examples, the computerized mobile device has a volume, defined by a housing of the mobile device, of between 400 - 1,200 cubic centimeters. In some examples, the computerized mobile device has a volume, defined by a housing of the mobile device, of between 200 - 800 cubic centimeters. In some examples, the computerized mobile device has a volume, defined by a housing of the mobile device, of between 100 - 400 cubic centimeters.

In some examples, the controller comprises at least one processor. In some examples, the electrotherapeutic waveform is for defibrillation. In some examples, the electrotherapeutic waveform is for pacing. In some examples, the therapeutic current control network comprises a patient relay circuit configured for use in control of allowing current flow through the patient load. The patient relay circuit may comprise at least one patient relay switch comprising a wide bandgap material. In some examples, a peak energy efficiency of the system in delivering the electrotherapeutic pulse to the patient is at least 80 percent. In some examples, the electrotherapeutic pulse delivered to the patient delivers an amount of energy to the patient that is no more than 15 percent different than an amount of energy of the specified waveform. In some examples, the specified waveform is a biphasic rectilinear current waveform.

One example provides an exemplary for generating an electrotherapeutic pulse for delivery to a patient, the method comprising: specifying a desired electrotherapeutic waveform for delivery to the patient, wherein energy for electrotherapy current is provided by an energy storage capacitor; and operating a resonant therapeutic current control network, comprising at least one current control switch and a resonant tank, in controlling an electrotherapeutic waveform being delivered to the patient to correspond to the desired electrotherapeutic waveform, wherein energy provided by the energy storage capacitor flows through the resonant tank, wherein the resonant tank is configured to boost a voltage of the electrotherapeutic pulse for delivery to the patient.

In some examples, the method is performed by a defibrillator. In some examples, operating of the therapeutic current control network comprises staggering a time of initiation of current switching relative to a time of initiation of voltage switching, the voltage switching being associated with the current switching, so as to reduce switching losses relative to substantially simultaneous initiation of the current switching and the associated voltage switching. In some examples, timing of voltage switching waveforms relative to associated current switching waveforms is controlled so as to minimize switching losses. Some examples include operating the therapeutic current control network to employ at least one of: substantially zero voltage switching (ZVS) and substantially zero current switching (ZCS). Some examples include operating the therapeutic current control network to employ closed-loop control of the electrotherapeutic waveform being delivered to the patient. Some examples include operating the therapeutic current control network to continuously adjust, over time, delivery of the electrotherapeutic waveform to the patient. In some examples, specifying the desired electrotherapeutic waveform for delivery to the patient comprises specifying a current waveform.

Some examples include charging the energy storage capacitor using a battery electrically coupled with the energy storage capacitor, and comprising returning at least a portion of energy stored by the energy storage capacitor to the battery for storage by the battery using a bidirectional charging control network, the bidirectional charging control network being electrically coupled to the battery and the energy storage capacitor. Some examples include using a bidirectional charging control network in transferring energy from the energy storage capacitor to a battery, and from the battery to the energy storage capacitor. The battery and the energy storage capacitor may be electrically coupled. The energy storage capacitor may be for providing energy for the electrotherapeutic waveform being delivered to the patient. In some examples, using the bidirectional charging control network comprises using the bidirectional charging control network comprising a first resonant tank. In some examples, generating the electrotherapeutic waveform is for defibrillation. In some examples, generating the electrotherapeutic waveform is for pacing.

In some examples, the at least one current control switch comprises a plurality of switches, and the method comprises controlling a configuration of each of the plurality of switches of the plurality of switches as open or closed in adjusting delivery of the electrotherapeutic waveform being delivered to the patient to match the desired electrotherapeutic waveform. Some examples include controlling a switching frequency of the at least one current control switch in adjusting delivery of the electrotherapeutic waveform being delivered to the patient to match the desired electrotherapeutic waveform. One example provides an exemplary method for generating an electrotherapeutic pulse for delivery to a patient, the method comprising: specifying a desired electrotherapeutic waveform for delivery to the patient, wherein energy for electrotherapy current is provided by an energy storage capacitor; and operating a resonant therapeutic current control network, comprising at least one current control switch and a resonant tank, in controlling an electrotherapeutic waveform being delivered to the patient to correspond to the desired electrotherapeutic waveform, wherein energy provided by the energy storage capacitor flows through the resonant tank, wherein the resonant tank is configured to boost a voltage of the electrotherapeutic pulse for delivery to the patient.

In some examples, the method is performed by a defibrillator. In some examples, operating of the therapeutic current control network comprises staggering a time of initiation of current switching relative to a time of initiation of voltage switching, the voltage switching being associated with the current switching, so as to reduce switching losses relative to substantially simultaneous initiation of the current switching and the associated voltage switching. In some examples, timing of voltage switching waveforms relative to associated current switching waveforms is controlled so as to minimize switching losses. Some examples include operating the therapeutic current control network to employ at least one of: substantially zero voltage switching (ZVS) and substantially zero current switching (ZCS). Some examples include operating the therapeutic current control network to employ closed-loop control of the electrotherapeutic waveform being delivered to the patient. Some examples, include operating the therapeutic current control network to continuously adjust, over time, delivery of the electrotherapeutic waveform to the patient. In some examples, specifying the desired electrotherapeutic waveform for delivery to the patient comprises specifying a current waveform.

Some examples include charging the energy storage capacitor using a battery electrically coupled with the energy storage capacitor, and comprising returning at least a portion of energy stored by the energy storage capacitor to the battery for storage by the battery using a bidirectional charging control network, the bidirectional charging control network being electrically coupled to the battery and the energy storage capacitor. Some examples include using a bidirectional charging control network in transferring energy from the energy storage capacitor to a battery, and from the battery to the energy storage capacitor. The battery and the energy storage capacitor may be electrically coupled. The energy storage capacitor may be for providing energy for the electrotherapeutic waveform being delivered to the patient. In some examples, using the bidirectional charging control network comprises using the bidirectional charging control network comprising a first resonant tank.

In some examples, generating the electrotherapeutic waveform is for defibrillation. In some examples, generating the electrotherapeutic waveform is for pacing. In some examples, the at least one current control switch comprises a plurality of switches, and comprising controlling a configuration of each of the plurality of switches of the plurality of switches as open or closed in adjusting delivery of the electrotherapeutic waveform being delivered to the patient to match the desired electrotherapeutic waveform. Some examples include controlling a switching frequency of the at least one current control switch in adjusting delivery of the electrotherapeutic waveform being delivered to the patient to match the desired electrotherapeutic waveform.

One aspect provides a system for generating an electrotherapeutic pulse to be delivered to a patient, the system comprising: an energy storage capacitor for providing electrotherapeutic current to the patient; a therapeutic current control network, electrically coupled to the energy storage capacitor, for controlling the electrotherapeutic current to be delivered to the patient, the therapeutic current control network comprising a resonant electrical circuit and at least one current control switch, wherein energy provided by the energy storage capacitor flows through the resonant electrical circuit, the therapeutic current control network being configured to be operable in an operation mode of at least two modes of operation, wherein, in each of the at least two modes, the therapeutic current control network is configured to be in a different electrical circuit topology, the at least two modes comprising: a first mode in which the therapeutic current control network is configured to be in a first electrical circuit topology, and a second mode in which the therapeutic current control network is configured to be in a second electrical circuit topology, and a controller electrically coupled to the energy storage capacitor and the therapeutic current control network, wherein the controller is configured to, in connection with the energy provided by the energy storage capacitor that flows through the resonant electrical circuit, control operation of the at least one current control switch of the therapeutic current control network in delivering an electrotherapeutic waveform to the patient to correspond with a specified waveform.

In some examples, the at least two modes of operation comprise at least three, five or eight modes of operation. In some examples, the operation mode is selected at least in part based on maximizing a value of a Q factor. In some examples, the value of the Q factor represents a measure of peak energy relative to dissipated energy with regard to the resonant electrical circuit. In some examples, the value of the Q factor represents a measure of peak energy stored in the resonant electrical circuit divided by an average energy dissipated in the resonant electrical circuit per radian at resonance. In some examples, the value of the Q factor is affected by the operation mode and a patient impedance.

In some examples, the resonant electrical circuit is connected with the patient in a parallel configuration. In some examples, the resonant electrical circuit boosts a voltage of the electrotherapeutic pulse to be delivered to the patient. Some examples are without electrical isolation of the patient from the energy storage capacitor. In some examples, the therapeutic current control network is configured such that the operation mode can be changed between the at least two modes of operation. In some examples, the controller is configured to be capable of changing of the operation mode between the at least two modes of operation. In some examples, the controller is configured to select the operation mode to be the first mode or the second mode. In some examples, the therapeutic current control network comprises at least one mode control switch. A configuration of the at least one mode control switch may determine the operation mode.

In some examples, the controller is configured to select the operation mode to be the first mode or the second mode by controlling the configuration of the at least one mode control switch as open or closed. In some examples, the at least one mode control switch comprises two mode control switches. A configuration of each of the two mode control switches as open or closed may determine the operation mode. In some examples, the controller is configured to select the operation mode to be the first mode or the second mode by controlling the configuration of each of the at least two mode control switches as open or closed. In some examples, the controller is configured to select the operation mode to be the first mode or the second mode based at least in part on an impedance of the patient. In some examples, the controller is configured to select the operation mode, as being the first mode or the second mode, based at least in part on the impedance of the patient. The first mode may be selected based at least in part on the impedance of the patient being at or above a threshold impedance. The second mode may be selected based at least in part on the impedance of the patient being below the threshold impedance.

In some examples, the controller is configured to select the operation mode. The threshold impedance may be a value between 40 ohms and 60 ohms. In some examples, the controller is configured to select the operation mode. The threshold impedance may be 50 ohms. In some examples, the controller is configured to select the operation mode to be the first mode or the second mode based at least in part on a voltage of the electrotherapeutic pulse to be delivered to the patient. In some examples, the controller is configured to select the operation mode to be the first mode or the second mode based at least in part on a voltage of the electrotherapeutic pulse to be delivered to the patient. The first mode may be selected based at least in part on the voltage of the electrotherapeutic pulse to be delivered to the patient being at or above a threshold voltage. The second mode may be selected based at least in part on the voltage of the electrotherapeutic pulse to be delivered to the patient being below the threshold voltage. In some examples, the system is configured such that, when the controller selects the operation mode to be the first mode, the configuration of the at least one mode control switch as open or closed causes the resonant electrical circuit to be connected with the patient in the parallel configuration, and, when the controller selects the operation mode to be the second mode, the configuration of the at least one mode control switch as open or closed causes the resonant electrical circuit to be connected with the patient in the series configuration.

In some examples, the energy storage capacitor and a first portion of the therapeutic current control network comprising the at least one current control switch and the resonant electrical circuit are connected via a first node and a second node; the first portion of the therapeutic current control network and a second portion of the therapeutic current control network comprising at least one mode control switch are connected via a third node and a fourth node. A configuration of the at least one mode control switch may determine the operation mode; the second portion of the therapeutic current control network and a third portion of the therapeutic current control network comprising a rectifier may be connected via a fifth node and a sixth node; the third portion of the therapeutic current control network and a fourth portion of the therapeutic current control network comprising a filter may be connected via a seventh node and an eighth node; and the fourth portion of the therapeutic current control network and a fifth portion of the therapeutic current control network, comprising at least one polarity control switch, a patient load, and at least one sensor configured to sense at least one electrical parameter from which current flow to the patient can be determined or estimated, may be connected via a ninth node and a tenth node.

In some examples, the at least one current control switch comprises two switches. In some examples, the at least one current control switch comprises four switches. In some examples, the first portion of the therapeutic current control network comprises at least one circuit component configured to reduce common-mode noise. In some examples, the at least one circuit component configured to reduce common-mode noise comprises at least one inductor and at least one capacitor. In some examples, the at least one sensor is configured to sense a current parameter. In some examples, the at least one sensor is configured to sense a voltage parameter. In some examples, the controller comprises at least one processor.

In some examples, the resonant electrical circuit comprises a resonant tank. In some examples, the resonant tank is a two element resonant tank. In some examples, the two element resonant tank comprises an inductor and a capacitor. In some examples, the resonant tank is a three element resonant tank. In some examples, the resonant tank is an LCC resonant tank. In some examples, the resonant tank is a CLL resonant tank. In some examples, the resonant tank is an LLC resonant tank. In some examples, the resonant tank is a four element resonant tank. In some examples, the resonant electrical circuit is an LCLC resonant tank.

In some examples, the rectifier comprises a switching network configured to be controlled to perform synchronous rectification. In some examples, the rectifier comprises a diode network configured to be controlled to perform passive rectification. In some examples, the at least one polarity control switch is configured for use in controlling direction of current flow. In some examples, the at least one polarity control switch comprises an H-bridge. In some examples, the sensed at least one electrical parameter comprises a current parameter. In some examples, the sensed at least one electrical parameter comprises a voltage parameter. In some examples, the rectifier is configured for use in conversion of alternating current to direct current. In some examples, the filter is configured for use in electrical signal frequency filtering. In some examples, the system comprises at least one sensor configured to sense at least one electrical parameter from which current flow to the patient can be determined or estimated. The controller may be configured to: process a signal associated with the sensed at least one electrical parameter; compare the processed signal with a second signal associated with the specified waveform; and control operation of the at least one current control switch of the therapeutic current control network in adjusting delivery of the electrotherapeutic waveform to the patient to correspond with the specified waveform.

In some examples, the at least one current control switch comprises a plurality of switches. The control of the operation of the at least one current control switch of the therapeutic current control network in the adjusting of the delivery of the electrotherapeutic waveform to the patient to correspond with the specified waveform may comprise controlling a configuration of each of the plurality of switches as open or closed.

In some examples, the system comprises a battery and a bidirectional charging control network. The battery and the bidirectional charging control network may be connected via a first node and a second node, and the bidirectional charging control network and the energy storage capacitor may be connected via a third node and a fourth node. The bidirectional charging control network may be for controlling energy flow from the battery to the energy storage capacitor for storage by the energy storage capacitor, and from the energy storage capacitor to the battery for storage by the battery. In some examples, the bidirectional charging control network comprises a second resonant electrical circuit.

In some examples, a computerized mobile device comprises the system. The computerized mobile device may be configured to deliver the electrotherapeutic pulse to the patient. In some examples, the computerized mobile device is configured to deliver the electrotherapeutic pulse to the patient. The electrotherapeutic pulse may have an energy of between 1 - 400 joules. In some examples, the computerized mobile device is configured to deliver the electrotherapeutic pulse to the patient. The electrotherapeutic pulse may have an energy of between 50 - 200 joules. In some examples, the computerized mobile device has an energy density, given by an energy of the electrotherapeutic pulse to be delivered to the patient relative to a volume defined by a housing of the mobile device, of between 0.5 - 1.00 joules per cubic centimeter. In some examples, the computerized mobile device has an energy density, given by an energy of the electrotherapeutic pulse to be delivered to the patient relative to a volume defined by a housing of the mobile device, of between 1.00 - 2.00 joules per cubic centimeter.

In some examples, the computerized mobile device has an energy density, given by an energy of the electrotherapeutic pulse to be delivered to the patient relative to a volume defined by a housing of the mobile device, of between 2.00 - 3.00 joules per cubic centimeter, m In some examples, the computerized mobile device has an energy density, given by an energy of the electrotherapeutic pulse to be delivered to the patient relative to a volume defined by a housing of the mobile device, of between 3.00 - 4.00 joules per cubic centimeter. In some examples, the computerized mobile device has a volume, defined by a housing of the mobile device, of between 400 - 1,200 cubic centimeters. In some examples, the computerized mobile device has a volume, defined by a housing of the mobile device, of between 200 - 800 cubic centimeters. In some examples, the computerized mobile device has a volume, defined by a housing of the mobile device, of between 100 - 400 cubic centimeters.

In some examples, the electrotherapeutic waveform is for defibrillation. In some examples, the electrotherapeutic waveform is for pacing. In some examples, the therapeutic current control network comprises a patient relay circuit configured for use in control of allowing current flow through a patient load. The patient relay circuit may comprise at least one patient relay switch comprising a wide bandgap material. In some examples, a peak energy efficiency of the system in delivering the electrotherapeutic pulse to the patient is at least 80 percent. In some examples, the electrotherapeutic pulse delivered to the patient delivers an amount of energy to the patient that is no more than 15 percent different than an amount of energy of the specified waveform. In some examples, the specified waveform is a biphasic rectilinear current waveform.

One aspect of the disclosure provides a system for generating an electrotherapeutic pulse to be delivered to a patient comprising: an energy storage capacitor for providing electrotherapeutic current to the patient; a therapeutic current control network, electrically coupled to the energy storage capacitor, for controlling the electrotherapeutic current to be delivered to the patient, the therapeutic current control network comprising a resonant electrical circuit and at least one current control switch, wherein energy provided by the energy storage capacitor flows through the resonant electrical circuit; and a controller electrically coupled to the energy storage capacitor and the therapeutic current control network, wherein the controller is configured to, in connection with the energy provided by the energy storage capacitor that flows through the resonant electrical circuit, control operation of the at least one current control switch of the therapeutic current control network in delivering an electrotherapeutic waveform to the patient to correspond with a specified waveform.

One aspect of the disclosure provides a system comprising: energy storage for providing electrotherapeutic current to a patient; a control network electrically coupled to the energy storage and comprising a resonant electrical circuit and at least one control switch configured to control the electrotherapeutic current to be delivered to the patient; and a controller electrically coupled to the control network, wherein the controller is configured to control operation of the at least one control switch of the control network in delivering the electrotherapeutic current to the patient.

One example provides an exemplary method for generating an electrotherapeutic pulse for delivery to a patient, the method comprising: specifying a desired electrotherapeutic waveform for delivery to the patient, wherein energy for electrotherapy current is provided by an energy storage capacitor; and operating a resonant therapeutic current control network, comprising at least one current control switch and a resonant tank, in controlling an electrotherapeutic waveform being delivered to the patient to correspond to the desired electrotherapeutic waveform, wherein energy provided by the energy storage capacitor flows through the resonant tank.

One example provides an exemplary method comprising: specifying a desired electrotherapeutic waveform for delivery to the patient; and operating a control network, comprising at least one current control switch and a resonant tank, to control delivery, from an energy store, of an electrotherapeutic waveform to the patient to correspond to the desired electrotherapeutic waveform.

In general, an example described with reference to one aspect may be implemented in combination with another aspect.

## BRIEF DESCRIPTION OF THE DRAWINGS

Various aspects of embodiments of the present disclosure are discussed below with reference to the accompanying figures, which are not intended to be drawn to scale. The figures are included for illustrative purposes and a further understanding of the various aspects and examples, and are incorporated in and constitute a part of this specification, but are not intended to limit the scope of the disclosure. The drawings, together with the remainder of the specification, serve to explain principles and operations of the described and claimed aspects and examples. In the figures, identical or nearly identical components that are illustrated in various figures may be represented by like numerals. For purposes of clarity, not every component may be labeled in every figure.

FIG. 1A illustrates an example of use of a defibrillator incorporating a therapeutic current control system, in accordance with embodiments of the present disclosure.

FIG. 1B illustrates an example of use of a handheld defibrillator incorporating a therapeutic current control system, in accordance with embodiments of the present disclosure.

FIG. 2A is a block diagram of an illustrative example of a therapeutic current control system, in accordance with embodiments of the present disclosure.

FIG. 2B is a block diagram of an illustrative example of a therapeutic current control system, including a battery and a bidirectional charging control network, in accordance with embodiments of the present disclosure.

FIG. 2C is a block diagram of an illustrative example of a therapeutic current control system, including a battery and a bidirectional charging control network, in accordance with embodiments of the present disclosure.

FIG. 3 is a flow diagram of an illustrative example of a method for therapeutic current control, in accordance with embodiments of the present disclosure.

FIG. 4A is a block diagram of an illustrative example of a therapeutic current control system, in accordance with embodiments of the present disclosure.

FIG. 4B is an exemplary schematic diagram of a therapeutic current control system with parallel resonance mode and series resonance mode capabilities, shown in parallel resonance mode, in accordance with embodiments of the present disclosure.

FIG. 4C is an exemplary schematic diagram of a therapeutic current control system with parallel resonance mode and series resonance mode capabilities, shown in series resonance mode, in accordance with embodiments of the present disclosure.

FIG. 4D is an exemplary schematic diagram of a therapeutic current control system implemented using a full bridge configuration in association with current control switches, shown in parallel resonance mode, in accordance with embodiments of the present disclosure.

FIG. 5A is a block diagram of an illustrative example of a therapeutic current control system, in accordance with embodiments of the present disclosure.

FIG. 5B is an exemplary schematic diagram a therapeutic current control system with parallel resonance, in accordance with embodiments of the present disclosure.

FIG. 6 is a three-axis graphical illustration of example simulation results relating to use of a therapeutic current control network with parallel resonance, showing switching frequencies corresponding with a range of capacitor voltages and delivered patient voltages, in accordance with embodiments of the present disclosure.

FIG. 7 is a graphical illustration of example simulation results relating to use of a therapeutic current control network with parallel resonance, showing biphasic rectilinear voltage waveforms over a range of patient load impedance values, in accordance with embodiments of the present disclosure.

FIG. 8 is a graphical illustration of example simulation results relating to use of a therapeutic current control network with parallel resonance, showing biphasic rectilinear current waveforms over a range of patient load impedance values, in accordance with embodiments of the present disclosure.

FIG. 8A is a graphical illustration of example simulation results relating to use of a therapeutic current control network with parallel resonance, showing a single phase of a current waveform with a patient load impedance of 50 ohms and a delivered energy of 200 joules.

FIG. 9A is a block diagram of an illustrative example of a therapeutic current control system, in accordance with embodiments of the present disclosure.

FIG. 9B is an exemplary schematic diagram of a quasi-resonant therapeutic current control system, in accordance with embodiments of the present disclosure.

FIG. 10 is a flow diagram of a method implemented using a quasi-resonant therapeutic current control system, in accordance with embodiments of the present disclosure.

FIG. 11 is a graphical illustration showing balances between various system features in overall system optimization, in accordance with embodiments of the present disclosure.

FIGS. 12A - 12Q are exemplary schematic diagrams of three-element resonant tank configurations that can be used in a therapeutic current control network, in accordance with embodiments of the present disclosure.

FIGS. 12R - 12U are exemplary schematic diagrams of two-element resonant tank configurations that can be used in a therapeutic current control network, in accordance with embodiments of the present disclosure.

FIG. 12V is a schematic exemplary diagram of a four-element resonant tank configuration that can be used in a therapeutic current control network, in accordance with embodiments of the present disclosure.

FIG. 13 includes flow diagrams illustrating two different charging related methods, in accordance with embodiments of the present disclosure.

FIG. 14 is a schematic diagram illustrating components of a system of various devices that may incorporate aspects of a therapeutic current control system, in accordance with embodiments of the present disclosure.

FIG. 15 is a schematic diagram illustrating components of a system, including a patient monitor and a therapeutic medical device, which may incorporate aspects of a therapeutic current control system, in accordance with embodiments of the present disclosure.

## DETAILED DESCRIPTION

Systems are presented herein for generating and delivering an electrotherapeutic pulse according to a specified current waveform to a patient, such as for defibrillation or pacing.

It is to be understood that, with reference to diagrams herein that depict circuit components, for instance, additional circuit components beyond those that are depicted may be included. An "electrical circuit" includes one or more electrical components. A "resonant electrical circuit" is a type of electrical circuit that includes inductance and capacitance. A "resonant tank" is a type of resonant electrical circuit.

Embodiments described herein provide systems for delivering an electrotherapeutic pulse to a patient, such as a defibrillation shock, with advantages over previously existing systems. Some embodiments provide a therapeutic current control network, which may include an electrical circuit, for generating and delivering the pulse to the patient. A controller may also be provided, which may be electrically coupled with the therapeutic current control network, for controlling aspects of operation of the therapeutic current control network in delivering the pulse to the patient. Some embodiments include systems that are capable of delivering the pulse to the patient more efficiently, more accurately, or with smaller and lighter devices as compared with previously existing systems and devices. In some embodiments, the therapeutic current control network may include a resonant electrical circuit, in which energy provided by an energy storage capacitor, which may be used in the delivery of the electrotherapeutic waveform to the patient, flows through the resonant electrical circuit. Embodiments are provided that include use of non-closed loop control or closed loop control in connection with the delivery of an electrotherapeutic waveform to the patient, where closed loop control may include, for example, continuous monitoring and adjusting of the delivery, examples of which are described in further detail herein.

In some embodiments, the therapeutic current control network may include at least two modes of operation (such as, e.g., two, three, four, five, six, seven, eight, nine, ten, eleven, twelve or more), and the therapeutic current control network may be in a different electrical circuit topology in each of the at least two modes of operation. In some embodiments, the controller may determine or select an operation mode. In some embodiments, the operation mode may be determined or selected based at least in part on an impedance of the patient. For example, in some embodiments, as described further herein, a parallel mode (as described herein) may be selected for, and may be more optimal for, higher patient impedances, and a series mode (as described herein) may be selected for, and may be more optimal for, lower patient impedances. In other examples, however, modes of operation (such as two or more) may include other different electrical circuit topologies, and an operation mode may be determined or selected in different ways, which may include being based at least in part on patient impedance.

In some embodiments, one or more aspects, features or components of the system or its operation, such as the selected operation mode, may be based at least in part on the Q factor associated with the resonant electrical circuit, or resonant tank, of the electrical circuit topology associated with the selected operation mode. In electrical circuits, such as RLC circuits (which include one or more resistors (R), inductors (L) and capacitors (C)), the Q factor is a measure that may be used to characterize resonators, such as a resonant electrical circuit. The Q factor may represent a measure of peak energy relative to dissipated energy with regard to the resonant electrical circuit. The Q factor may be defined as the peak energy stored in the resonant electrical circuit divided by the average energy dissipated in the resonant electrical circuit per radian (relative to an angular frequency associated with a resonance frequency) at resonance. Generally and conceptually, a higher Q factor may be associated with a lower rate of energy loss, such that oscillations reduce in magnitude more slowly, or oscillate longer. Also, generally, a higher Q factor may be associated with oscillation in a smaller range of frequencies and with greater stability.

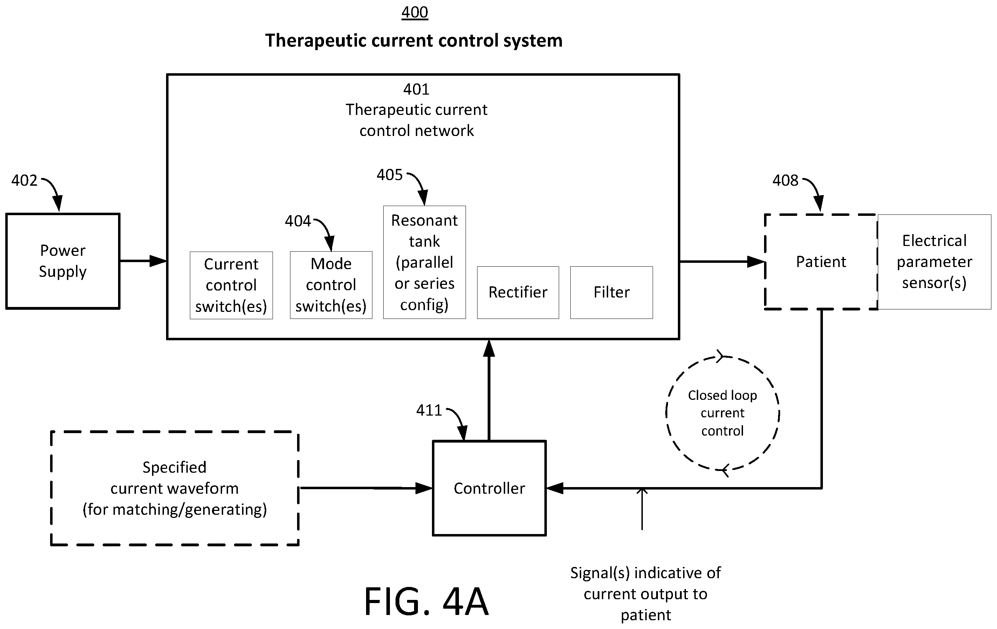

In some embodiments in which a parallel mode or a series mode may be selected, as described, for example, with reference to FIGs. 4A-4D herein, the operation mode may be selected based at least in part to maximize the Q factor.

For an (ideal) series circuit (with a series circuit being associated with series mode), the Q factor is given by:

$$\text{(Equation 1)} \quad Q = (1/R) \text{ (square root of } (L/C))$$

However, for an (ideal) parallel circuit (with a parallel circuit being associated with parallel mode), the Q factor is given by:

$$\text{(Equation 2)} \quad Q = R \text{ (square root of } (C/L))$$

In equations 1 and 2, above, as applied to series mode and parallel mode, respectively, R is associated with the patient impedance. As described herein, in some embodiments, series mode may generally be selected for, and more optimal for, lower patient impedances, while parallel mode may generally be selected for, and more optimal for, higher patient impedances. This is logical in view of equations 1 and 2, above. Particularly, in series mode, the lower the patient impedance (which is associated with R), the higher the value of the Q factor (which is generally desirable), since, in this case, the value of the Q factor generally varies inversely proportionately with R. However, in parallel mode, the higher the patient impedance (which is associated with R), the higher the value of the Q factor (which is, again, generally desirable), since, in this case, the value of the Q factor generally varies proportionally with R.

Some embodiments include a first mode in which the resonant electrical circuit is connected with the patient in a parallel configuration and a second mode in which the resonant electrical circuit is connected with the patient in a series configuration. In some embodiments, the controller may select the first mode or the second mode for operation. In some embodiments, operation in the first mode may include boosting of a voltage of the pulse to be delivered to the patient. In some embodiments, the selection may be made, for example, to increase or optimize efficiency or accuracy with regard to the delivery of an electrotherapeutic waveform to the patient. In some embodiments, however, the therapeutic current control network may always operate with the resonant electrical circuit connected with the patient in a parallel configuration. Some embodiments do not require electrical isolation of the patient from the energy storage capacitor, and some embodiments do not require a transformer electrically connected between the energy storage capacitor and the patient.

In some embodiments, the therapeutic current control network may include at least one current control switch.

The at least one current control switch may include, for example, two current control switches or four current control switches. The controller may control a configuration of the at least one current control switch as open or closed in adjusting the delivery of the electrotherapeutic waveform to the patient to correspond to a specified waveform.

In some embodiments, the controller selecting the first mode or the second mode may be based at least in part on the impedance of the patient. In some embodiments, based at least in part on the impedance of the patient (or a parameter incorporating the impedance of the patient) being above, or at or above, a threshold, the first mode may be determined to be more optimal and selected, whereas, based at least in part on the impedance of the patient (or the parameter incorporating the impedance of the patient) being below, or at or below, the threshold, the second mode may be determined to be more optimal and selected. In some embodiments, the parameter may be or incorporate a ratio of the patient impedance to the resonant electrical circuit impedance, for example.

In some embodiments, a therapeutic current control network as described above may have a configuration that includes the following. The energy storage capacitor and a first portion of the therapeutic current control network including the at least one current control switch and the resonant electrical circuit are connected via a first node and a second node. The first portion of the therapeutic current control network and a second portion of the therapeutic current control network including at least one mode control switch are connected via a third node and a fourth node, in which a configuration of the at least one mode control switch determines the operation mode. The second portion of the therapeutic current control network and a third portion of the therapeutic current control network including a rectifier are connected via a fifth node and a sixth node. The third portion of the therapeutic current control network and a fourth portion of the therapeutic current control network including a filter are connected via a seventh node and an eighth node. The fourth portion of the therapeutic current control network and a fifth portion of the therapeutic current control network, including at least one polarity control switch, a patient load, and at least one sensor configured to sense at least one electrical parameter from which current flow to the patient can be determined or estimated, are connected via a ninth node and a tenth node.

In some embodiments, the therapeutic current control network may be configured to boost a voltage of the electrotherapeutic pulse to be delivered to the patient. In some embodiments, the therapeutic current control network may include at least one current control switch. The at least one current control switch may include, for example, two current control switches. The controller may control a switching frequency of the at least one current control switch in controlling or adjusting delivery of the electrotherapeutic waveform to the patient to correspond to a specified waveform. In some embodiments, the resonant electrical circuit is connected with the patient in a parallel configuration.

In some embodiments, a therapeutic current control network as described above may have a configuration that includes the following. The energy storage capacitor and a first portion of the therapeutic current control network including an inductor are connected via a first node. The first portion of the therapeutic current control network and a second portion of the therapeutic current control network including the resonant electrical circuit and a rectifier are connected via a second node. The second portion of the therapeutic current control network and a third portion of the therapeutic current control network including a capacitor and a patient load are connected via a third node.

Embodiments described herein provide systems for generating electrotherapeutic pulses and waveforms for delivery to patients with high safety, reliability, accuracy and precision. Moreover, embodiments of the present disclosure provide for electrical circuit topologies that are built to generate and deliver a wide variety of desired electrotherapy waveforms to the patient, allowing great flexibility in the therapy that may be delivered. This can also provide an ability to explore use of a wide range of different electrotherapeutic waveforms for various potential uses. Still further, embodiments described herein provide or enable use of devices, such as defibrillators, that, in addition to being higher performing, may also be more efficient, more powerful, lighter, smaller, more portable and less expensive than previously existing systems and devices. In some embodiments, with higher performance and efficiency at less size, weight or cost relative to previously existing systems, various previously unachievable combinations and balances of features can be struck as needed for particular uses or implementations. Such balances can include, for example, a balance between minimum necessary particular performance or power parameters for a certain use, and maximum size or weight that is practical for the use. In some embodiments, such uses may include use of portable, wearable or handheld defibrillators or other devices.

Many advantages of embodiments described herein can be further appreciated in part by comparison with previously existing systems and techniques. Previously existing defibrillators, for example, may operate as follows. In an electrical circuit, a power converter such as a flyback converter is used in charging a high voltage capacitor using a battery. Since capacitor current leakage generally must be kept low in such a system to assure that the intended energy reaches the patient, the capacitor must be large and heavy enough to sufficiently minimize or account for such leakage. Some embodiments allow for a significant reduction in the maximum voltages and capacitance imposed on the high voltage capacitor thus allowing for a significant reduction in the size and weight of the high voltage capacitor. Since the high voltage capacitor occupies a significant percentage of the overall volume of a typical defibrillator as well as imposing minimum cross-sectional dimensions on the overall defibrillator package due to the capacitor bulk, the size reductions in the high voltage capacitor made possible by the embodiments herein can result in size reductions exceeding a 50% volumetric decrease.

One example of previously existing defibrillators includes those including a circuit including a number of resistors, a set or subset of which can be engaged and used according to a particular selected resistor schedule. A resistance is engaged and initial patient impedance is determined. Based on parameters including the initially determined patient impedance and a desired amount of energy to be delivered to the patient over a time period of milliseconds, an appropriate series of resistors is selected and utilized to deliver a defibrillation pulse to the patient. Energy is provided by discharge of the high voltage capacitor. The generated waveform of the defibrillation pulse is largely governed by the available and selected series of resistors. The circuit requires a substantial amount of electronic components, such as silicon controlled rectifiers (SCRs), gate drivers and resistors. Excess energy may be offloaded or "dumped" into high voltage resistors, producing heat.

Embodiments of the present disclosure provide various advantages. For example, some embodiments allow for generation and delivery of a wide range and variety of waveforms with high accuracy and precision. Some embodiments optionally utilize closed loop control of a therapeutic waveform, which can include continuous, repeated or substantially real-time monitoring and adjustment of a waveform, for example, throughout delivery to a patient. Since the waveform can be monitored during delivery, such as based on a specified waveform of any of a wide variety of shapes and forms, for example, and also tracked or matched very accurately, a great variety and range of waveforms can be generated and delivered. Some embodiments optionally utilize non-closed or open loop control of a therapeutic waveform, which can include a preset schedule or configuration of electrical execution that is predetermined through extensive testing, verification and validation procedures, so as to result in delivery of the desired therapeutic waveform to the patient without requiring real-time continuous monitoring used in closed loop control. In some embodiments, a resonant electrical circuit based system or circuit topology allows for a high degree of control and accuracy. In some embodiments, wide bandgap materials, such as silicon carbide (SiC) or gallium nitride (GaN), further enhance performance, including enhancing the accuracy and precision of the provided waveform. Since a selected particular waveform to be provided to a patient can be critical in optimizing both therapeutic effect and reduction of risk to the patient, being able to provide a selected waveform with exactness can likewise be critical.

Some embodiments further provide greater overall performance and efficiency compared to many previously existing systems. For example, in aspects of closed or non-closed loop control, for example, use of a resonant tank or other resonant electrical circuit, use of soft switching, and use of wide bandgap materials may contribute to system performance and energy efficiency. Moreover, embodiments described herein may provide greater performance and efficiency at a smaller size or weight, or greater performance and efficiency for a given size, weight or cost. These attributes, in turn, can provide great practical advantages in terms of aspects such as portability, availability or fast availability to a potential user, often in contexts where rapid availability, even down to seconds, can be critical or even life-saving.

In some embodiments, when closed loop operation of a system is employed, such operation may enhance reducing patient risk by allowing monitoring and control during generation and delivery of a waveform. For example, if some unexpected condition causes an unplanned and undesired alteration in the waveform during delivery, then monitoring, control and adjustment can effectively allow for detection and prevention, mitigation or fast/immediate correction of any such deviation. Moreover, use of resonant electrical circuit based systems and circuit topologies, in closed loop current control embodiments, can allow unexpected developments to be detected and addressed, or as well addressed, during delivery of a waveform. However, with sufficient verification and validation design controls in place, embodiments described herein also allow for non-closed or open loop operation of the system to be implemented in accordance with aspects of the present disclosure.

Also, some embodiments as disclosed herein are inherently stable. Still further, some embodiments provide higher bandwidth and higher dynamic range for electrotherapeutic delivery to the patient than previous implementations. Various high performance attributes, such as those mentioned above, can contribute to these aspects, including the use of a resonant electrical circuit based electrical circuit topology, use of soft switching, and use of wide bandgap materials.

Some embodiments provide a system that provides a peak efficiency of 80, 85 or 90 percent or more in delivering an electrotherapeutic waveform to a patient, which may be measured based on energy used by the system relative to energy delivered to a patient. Furthermore, some embodiments deliver an amount of energy to a patient that is no more than 15, 10, 5 or 3 percent different, or from 0.1% to no more than any of 15, 10, 5 or 3 percent different, than an amount of energy of the specified waveform, or, for example, no more than 3 joules more or less than a specified amount of energy. Furthermore, some embodiments provide devices that, for a given required level of performance or efficiency, can be implemented in a device that is smaller, lighter, with generally fewer and smaller electrical components, and less expensive than would be possible with previously existing systems. Still further, less precise or "strict" system calibration may not be necessary, or as necessary, in embodiments of the present disclosure, relative to open loop systems, for instance, due to the system's ability to correct deviations by monitoring and adjusting a waveform during generation and delivery.

In some embodiments, previously known electrotherapy circuits can be improved, thereby significantly reducing printed board (PCB) area and thus reducing the overall volume and weight of the defibrillator.

Some embodiments of the present disclosure utilize a circuit topology and technique for optional "closed loop" control of an electrotherapeutic waveform being delivered to a patient. For example, in some embodiments, the waveform being delivered can be controlled or adjusted during its delivery to match a specified waveform with high accuracy and precision. Hence, the therapeutic current control network may actively sense or otherwise measure the current as it is being applied to the patient, and subsequently control (e.g., increase or decrease) the level of current so as to substantially track with the specified waveform. The closed loop topology may include a controller, such as a field programmable gate array (FPGA) or a digital signal processor (DSP) based controller, which controls operation of at least a portion of a therapeutic current control network, potentially among other things. A therapeutic current control network according to some embodiments includes a resonant tank, or other resonant electrical circuit, as described further herein. The controller may control, potentially among other things, operation of at least a portion of at least one switch of the therapeutic current control network to adjust the waveform over the time of delivery to the patient, which time is typically on the order of milliseconds, to match a specified waveform. The controller may also control other components of the therapeutic current control network, such as a rectifier, or other components of the overall system, in adjusting the waveform or otherwise. In some embodiments, use of a resonant tank or other resonant electrical circuit, in the therapeutic current control network, as described further herein, allows for high performance and efficiency, including by allowing faster switching for a given amount of switching loss. Closed loop topologies of embodiments described herein may offer many advantages over previously existing topologies, such as higher overall performance and efficiency, greater flexibility in terms of waveforms that can be produced or reproduced, less produced heat and thus a smaller required cooling system, fewer and smaller electrical components (which can increase system reliability), smaller footprint, greater portability, longer battery life and reduced cost. Though, as discussed above, embodiments of the present disclosure may implement non-closed loop control topologies as well, which may be sufficiently effective and safe provided that the appropriate verification and validation design controls are put in place for the therapeutic waveform to be predetermined.

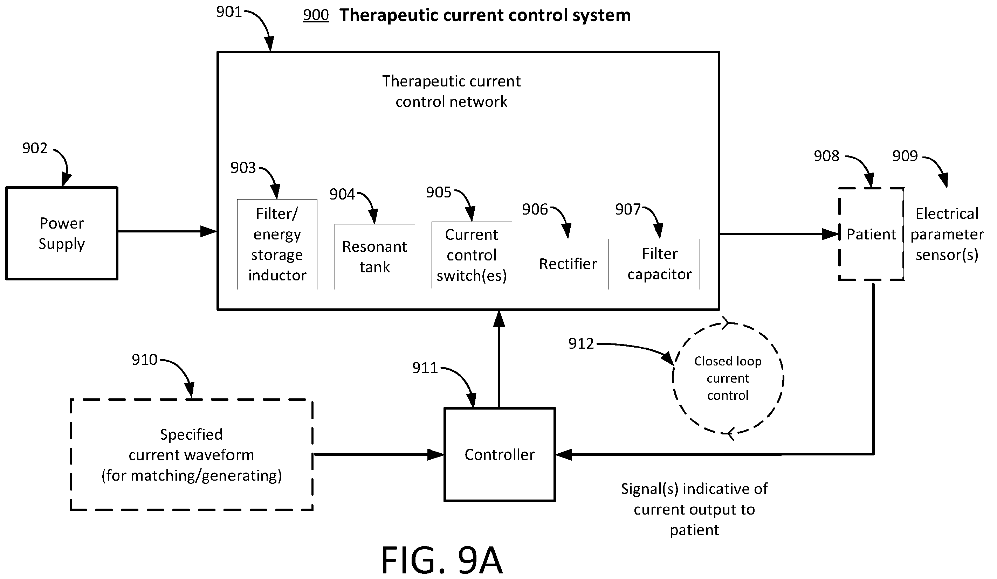

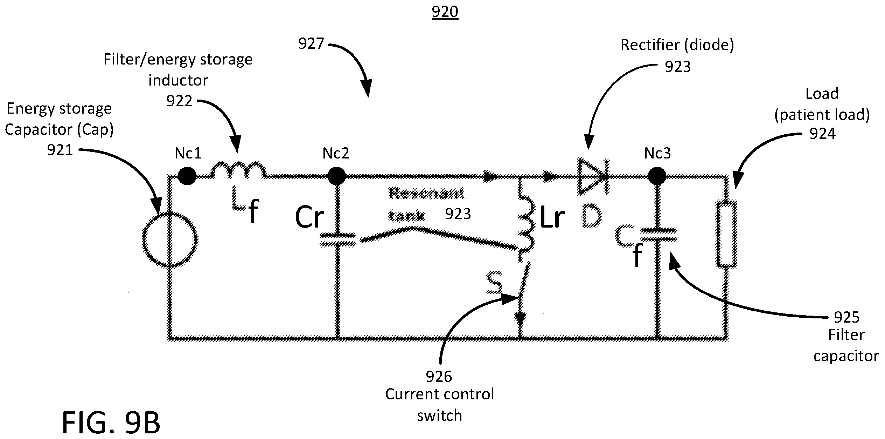

Additionally, some embodiments utilize soft switching, substantially zero voltage switching (ZVS) or substantially zero current switching (ZCS), as further described herein, such as with reference to Fig. 9, further reducing switching losses and increasing efficiency. In general, soft switching can include controlling timing of device turn-on and turn off at near zero current or near zero voltage, thus minimizing intersection of the current and voltage waveforms, which in turn minimizes energy losses associated with the switching. Additionally, in some embodiments, utilization of soft switching allows for reduced or low electromagnetic interference (low EMI) for higher system performance.

Furthermore, some embodiments use wide bandgap switch component materials such as SiC or GaN, or others, increasing performance and efficiency by enabling higher bandwidth and faster switching, resulting in minimized switching loss,, and enabling use of higher voltage devices.

The following table, Table 1, provides a general, non-limiting, example comparison of examples of some existing defibrillator aspects relative to aspects of examples of defibrillators according to some embodiments of the present disclosure.

Table 1 - comparison of examples of types of defibrillators/systems

| Row | Aspect | (1) Defibrillator using some previously existing therapeutic control systems | (2) Defibrillator using therapeutic control system according to some embodiments herein, using regular bandgap materials | (3) Miniaturized defibrillator using therapeutic control system according to some embodiments herein, using wide bandgap materials |
|---|---|---|---|---|
| 1 | Switches | SCRs | IGBT | SiC and/or GaN |
| 2 | Number of H bridge switches | 12 (3 per arm x 4) | 4 | 4 |
| 3 | High voltage component device availability | No | Yes | Yes |
| 4 | Usage of dump resistors | Yes | No | No |
| 6 | Energy efficiency | Lowest of the three | Middle of the three | Highest of the three |
| 7 | Filter size | Largest of the three | Middle of the three | Smallest of the three |

(continued)

| Row | Aspect | (1) Defibrillator using some previously existing therapeutic control systems | (2) Defibrillator using therapeutic control system according to some embodiments herein, using regular bandgap materials | (3) Miniaturized defibrillator using therapeutic control system according to some embodiments herein, using wide bandgap materials |
|---|---|---|---|---|
| 8 | Total defibrillator size | Largest of the three | Middle of the three | Smallest of the three |
| 9 | Energy storage capacitor or high voltage capacitor | Largest of the three | Smaller than (1) | Smaller than (1) |
| | size | | | |

It is noted that Table 1 is not intended to be limiting, but rather provides a comparison of example defibrillators and systems. System (1) is an example of a previously existing therapeutic control system. System (2) is an example therapeutic control system according to some embodiments described herein, in which regular (not wide bandgap) materials are used. System (3) is an example therapeutic control system according to some embodiments described herein, in which wide bandgap materials are used.

Table 1 provides a general comparison of various aspects of the example systems, relative to each other. Specifically, Row 1 compares the types of switches used. Row 2 compares the number of H bridge switches utilized. Row 3 indicates whether high voltage component devices may be utilized. Row 4 indicates whether resistors are used to dump at least a portion of excess or unused energy from an energy storage capacitor or a high voltage capacitor. Row 5 indicates relative estimated price. Row 6 compares relative energy efficiency. Row 7 compares relative filter size. Row 8 compares the relative size of a defibrillator utilizing each system. Row 9 compares the relative size of an energy storage capacitor or a high voltage capacitor used in each system.

Additionally, some embodiments may use one or more planar transformers. By using flat windings, which may, in some embodiments, be printed on a printed circuit board (PCB), instead of copper wire coils, the height of the associated device may be reduced, and the size or footprint of the overall device may therefore also potentially be reduced. In some embodiments, planar transformers may also offer advantages, for example, in terms of providing precise electrical characteristics such as capacitance, output and aspect ratio.

Still further, some embodiments utilize a bidirectional power supply. A battery may be used to charge an energy storage device, such as an energy storage capacitor, that is then used to provide current for an electrotherapeutic waveform. However, instead of dumping excess energy into one or more resistors, the bidirectional power supply may enable return of at least a portion of the stored capacitor energy back to the battery for storage in the battery. Relative to energy dumping at one or more resistors, this can provide for less wasted energy, less produced heat, and greater battery life, or more available defibrillation cycles or other uses before recharging of the battery is required. In some embodiments, a network including a resonant tank, or other resonant electrical circuit, may be utilized for efficient bidirectional energy transfer between the battery and the energy storage device, as further described herein. For example, some embodiments described herein may utilize a bidirectional charging control network.

In some embodiments described herein, many SCRs may be replaced by four insulated gate bipolar transistors (IGBTs), allowing for significantly reduced printed circuit board (PCB) layout area, thereby reducing overall volume of the defibrillator. Furthermore, some embodiments described herein generally may allow use of high voltage devices, increasing the overall level of system performance. Some embodiments of systems and devices described herein may be produced at a lower cost than previously existing systems and devices. Utilization of wide bandgap materials may increase cost, but still allow a cost that is lower than the cost of previously existing systems. Some embodiments include advantages over previously existing systems including higher energy efficiency (which may be highest with use of wide bandgap materials), and smaller total size, filter size and energy storage capacitor size (all of which may be smallest with use of wide bandgap materials). Furthermore, in some embodiments, signal tracking is most accurate and precise with use of fast switching facilitated by use of wide bandgap materials

In some embodiments, monitoring and control of a waveform during delivery, continuously and substantially in real time, provides safety advantages over previously existing therapeutic control systems. For example, when closed loop control is employed according to some embodiments described herein, more accurate and precise matching of a delivered waveform to a desired waveform may occur. Since a desired waveform may be selected or determined for optimal positive effect and safety, better matching can mean greater positive effect and greater safety. Additionally, particular safety hazards can sometimes arise in previously existing therapeutic control systems that can be avoided in embodiments of therapeutic control systems described herein. Herein, the term "patient" is broadly intended to include any individual, whether a person or an animal, or simulated patient in the form of a defibrillation test equipment, to whom an electrotherapeutic pulse or waveform may be delivered according to embodiments described herein, whether or not such individual is within a medical or healthcare context or environment. It is noted that embodiments are included in which a waveform may be generated to match a specified waveform, but not delivered to a patient, or may be used for test or simulation purposes, such as in experimental/laboratory, training, and/or manufacturing settings. It is further noted that, herein, the term "pulse" is intended to be broadly construed, and can include, for example, among other things, any of various types of bursts, as well as any type or shape of, or particular, waveform.

According to embodiments disclosed herein, devices, or components of devices, may be provided with higher energy densities than may be achievable using previously existing systems. Herein, the term "energy density" refers to the amount of energy delivered or deliverable to a patient, such as via an electrotherapeutic pulse or waveform, by a device, relative to the size of the device. The device may be, for example, a defibrillator or mobile defibrillator. Various aspects and features of embodiments disclosed herein may contribute to allowing high energy densities. These aspects may include, for example, as described herein, device or component specifics and configurations, use of planar transformers, use of high voltage devices, use of methods and systems for generation and delivery of electrotherapeutic pulses or waveforms, overall circuit topologies, optional use of closed loop therapeutic current control systems, use of controllers within therapeutic current control systems, use of therapeutic current control networks, use of resonant electrical circuits or resonant tanks within therapeutic current control networks, use of bidirectional power supplies, use of resonant electrical circuits within bidirectional power supplies, use of synchronous or active rectification, use of fast switching, use of soft switching, use of zero current switching (ZCS), use of zero voltage switching (ZVS), and use of wide bandgap switching materials such as silicon carbide (SiC) or gallium nitride (GaN).

Some embodiments provide a computerized mobile or pocket-sized device configured to provide an electrotherapeutic pulse to a patient may include one or more of the following features. A device may include a resonant electrical circuit that includes a resonant tank. A device may be configured to provide an electrotherapeutic waveform for defibrillation or pacing. A device may be configured to deliver an electrotherapeutic waveform to the patient, wherein the electrotherapeutic waveform has an energy of between, for example, 1 - 400 or 50 - 200 joules. A device may have an energy density, given by an energy of the electrotherapeutic waveform to be delivered to the patient relative to a volume defined by the housing, for example, of between 0.5 - 1.00, 1.00 - 2.00, 2.00 - 3.00 or 3.00 - 4.00 joules per cubic centimeter. A device may have an energy density of 4.00 - 6.00 joules per cubic centimeter. A device may have an energy density of over 4.00 joules per cubic centimeter. A device may have an energy density of over 6.00 joules per cubic centimeter. A device may have an energy density of less than 0.5 joules per cubic centimeter. A device may have a volume defined by the housing, for example, of between 400 - 1,200, 200 - 800 or 100 - 400 cubic centimeters. A device may have a volume, defined by the housing, of less than 100 cubic centimeters. A device may have a volume, defined by the housing, of greater than 1,200 cubic centimeters. A device may have at least one planar transformer of, for example, between 2,500 - 4,000, 2,750 - 3,500 or 3,000 - 3,300 volts. An electronic system may include at least one switching device including a wide bandgap material. A device may include at least one switching device that may include at least one of silicon carbide (SiC) or gallium nitride (GaN). At least one switch or a switch network may include a plurality of switches, and wherein at least a portion of the switches includes a semiconductor material with a band gap of, for example, between 2 and 6 ev. An electronic system may include at least one switching device with a switching speed, for example, of between 150 - 500, 200 - 600, 300 - 500 or 375 - 500 kilohertz. An electronic system may include at least one switching device with a switching speed of less than 150 kilohertz. An electronic system may include at least one switching device with a switching speed of greater than 500 kilohertz.

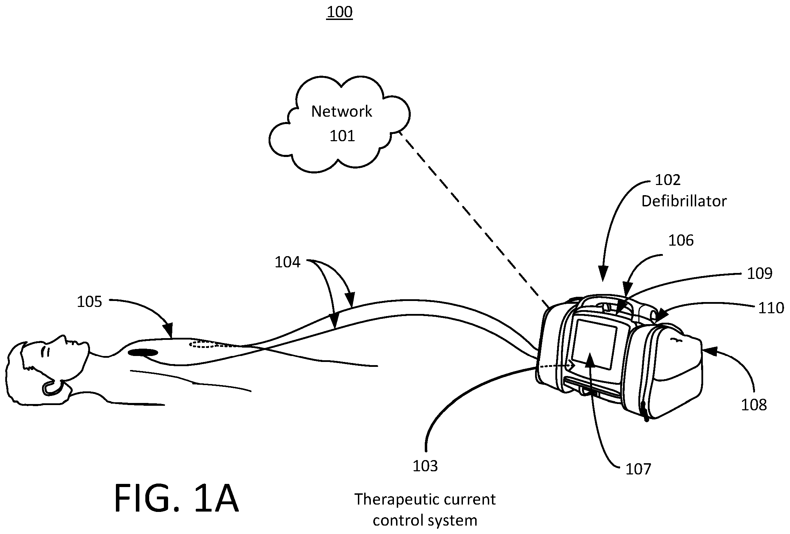

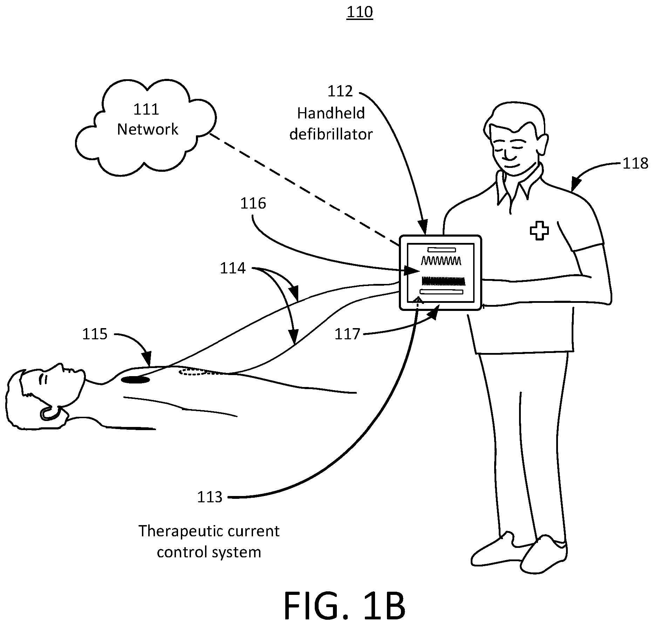

FIGS. 1A and 1B illustrate examples of uses 100, 110 of defibrillators 102, 112, employing therapeutic current control systems 103, 113 to provide electrotherapy defibrillation current to patients 105, 115. Use of a resonant electrical circuit and/or closed loop therapeutic current control may allow delivery of an electrotherapeutic waveform to a patient with greater safety, accuracy and precision than previously existing systems, while also permitting use of a smaller, lighter, more portable or less expensive defibrillator. Additionally, therapeutic current control systems may enable generation and delivery of a wide variety of waveforms, including waveforms that may be desired to be delivered to a patient. It is noted that, while not shown in FIGS. 1A and 1B, in some circumstances, electrotherapy may be provided in tandem or combination with other therapies, such as chest compressions, ventilation, or drug therapies. For example, during a cardiac arrest protocol, a rescuer may deliver cardiopulmonary resuscitation (CPR), such as in the form of chest compressions and, in some cases, ventilations, followed by a period of ECG analysis to determine whether the heart is in need of a defibrillation shock. Embodiments described herein provide for a compact, efficient electrical circuit topology for generating such a defibrillation shock, in accordance with a desired current waveform.

More particularly, FIG. 1A illustrates an example 100 of use of a defibrillator 102 incorporating a therapeutic current control system 103. While various methods of use are possible, in the method depicted, two electrodes 104 are placed and oriented on the torso of the patient 105 for delivery of defibrillation current through the heart of the patient 115. The defibrillator 102 includes a carrying case 110 with a handle 106 and side storage pockets 108 that may be used to store peripherals such as tablets, sensor accessories (e.g., blood pressure cuff, electrodes) and/or other electronic devices that may be adapted for use in coordination with the defibrillator 102. The defibrillator 102 also includes a monitor 107 for use by a health care provider or other user who is using the defibrillator to provide defibrillation electrotherapy to the patient 105. The defibrillator 102 and any peripheral electronic devices may be connected to one or more networks 101 via one or more wired or wireless connections.

The therapeutic current control system 103 may include electronic circuitry and components partially or fully contained within a housing 109 of the defibrillator 102. The defibrillator 102 and therapeutic current control system 103 may be used in delivering an electrotherapeutic defibrillation waveform to the patient 105.

FIG. 1B illustrates an example of use 110 of a handheld or pocket-sized defibrillator 112, with a monitor 116, incorporating a therapeutic current control system 113 (in various embodiments of the present disclosure, non-closed loop and closed loop therapeutic current control systems may be employed), being used by a health care provider or other user 118. As depicted, two electrodes 114 are placed on the torso of the patient 115 for delivery of defibrillation current through the heart of the patient 115. The defibrillator 102 and any associated peripheral electronic devices may be connected to one or more networks 111 via wired or wireless connections. As discussed herein, embodiments of circuit topologies of the present disclosure provide for the ability to integrate safe and effective defibrillation components into a mobile computing device, such as a tablet, phone, or other portable device. Such a system may allow for greater access of such devices to the general public, as well as emergency medical services (EMS). Further, during a cardiac medical event, it is of utmost importance for the rescuer to begin treatment on the patient immediately. Accordingly, embodiments described may help to save precious time for EMS personnel to reach the patient, not having to carry heavy and sometimes awkward equipment to the patient right away. In addition, as such embodiments may be integrated into a portable computing device as small as a tablet or phone, it is possible for lay users to be equipped with, or may otherwise have easy access to, a handheld defibrillator 112. This would allow people who are in close proximity to the medical emergency to begin treatment well before EMS or other trained personnel arrive at the scene.

The therapeutic current control system 113 may include electronic circuitry and components partially or fully contained within a housing 117 of the handheld defibrillator 112. The handheld defibrillator 112 and the therapeutic current control system 113 are used in delivering an electrotherapeutic defibrillation waveform to the patient 115. In some embodiments, use of a therapeutic current control system can allow for a smaller, lighter associated device, such as a defibrillator, for a given set of required performance or power parameters. For example, as described further herein, embodiments of the present disclosure would not require bulky components, such as large resistors or considerably sized high voltage capacitors, which are commonly associated with conventional defibrillators. In some cases, this can allow for a portable, wearable or handheld defibrillator or other electrotherapy device with sufficient performance or power parameters, such as might not be practical or possible with previously existing systems.

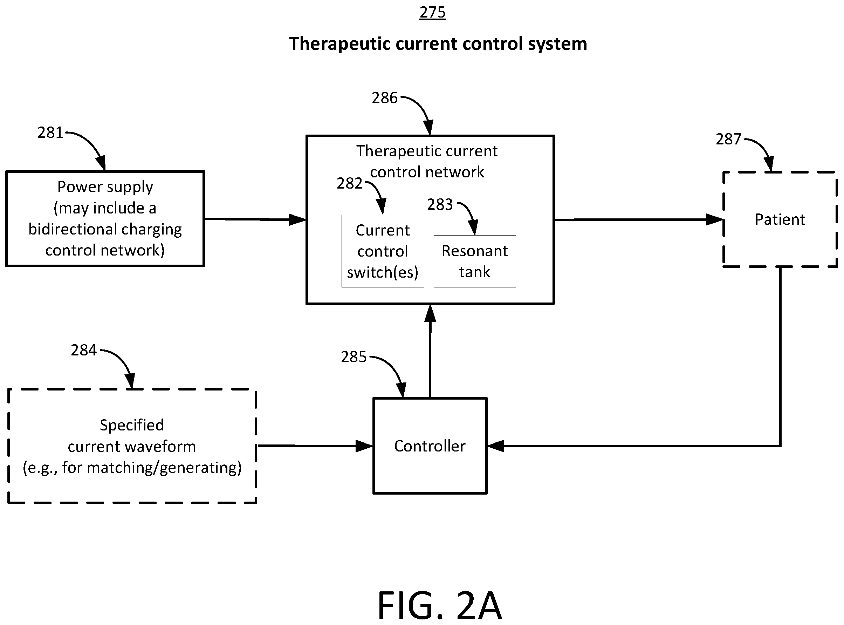

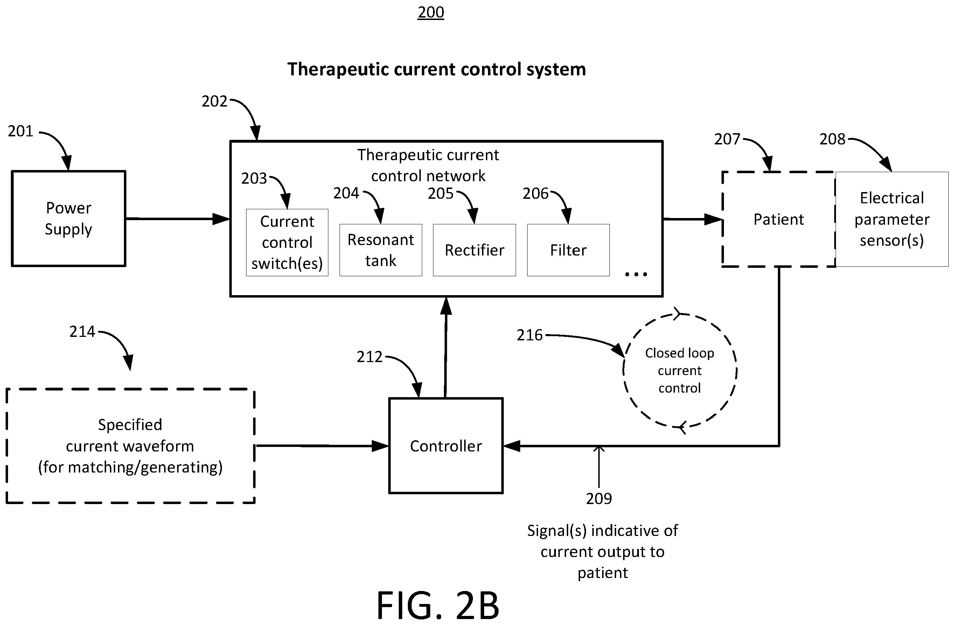

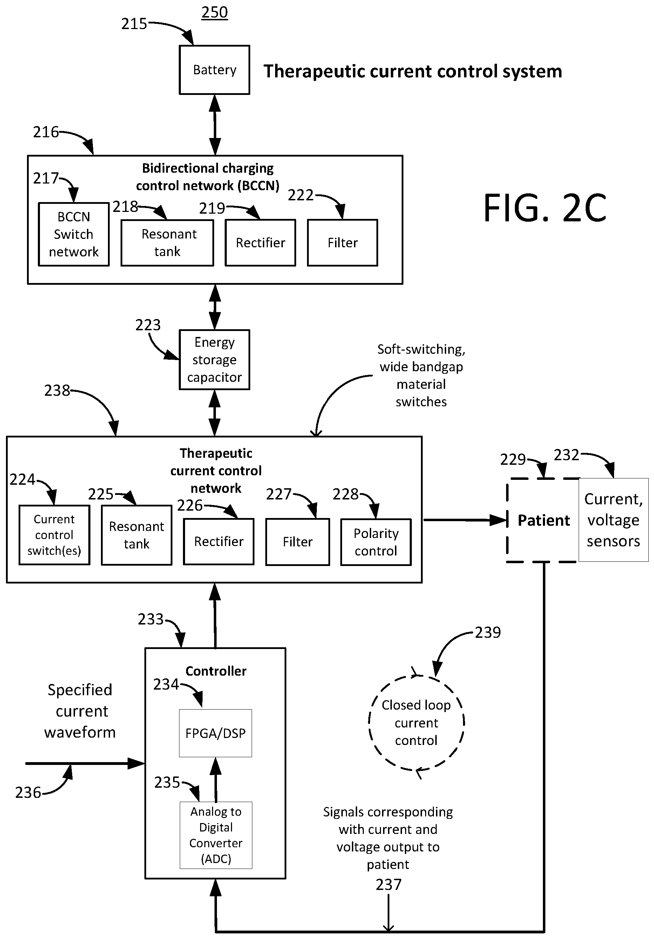

FIGS. 2A, 2B and 2C are block diagrams of example therapeutic current control systems 275, 200, 250 of the present disclosure that can be used, for example, in defibrillators, pacemakers or other electrotherapy devices, which may include portable devices.

In FIG. 2A, the therapeutic current control system 275, which may, in various embodiments, be closed loop or non-closed loop, includes a power supply 281 electrically coupled with a therapeutic current control network 286. The power supply 281 may be or include an energy storage device such as a high voltage capacitor, which may be charged, such as by a battery or other power source, prior to delivery of electrotherapy current to the patient 287. Various types of capacitors can be used in various embodiments, which may include, for example, film-foil capacitors or metallized film capacitors, as well as capacitors utilizing various materials, including, for example, aluminum electrolytic capacitors, ceramic capacitors, polycarbonate, polypropylene, polystyrene, tantalum or niobium, among others.

The therapeutic current control network 286 may include at least one current control switch 282 (e.g., one or more switches and/or a switch network for controlling the therapeutic current), a resonant tank or other resonant electrical circuit 283 and a controller 285. Input to the controller 285 may include a specified current waveform 284. In various embodiments, the therapeutic current control network 286 may include closed or non-closed loop current control, such as with regard to an electrotherapeutic waveform generated and delivered to a patient. Some non-closed loop systems may utilize a selected pre-set schedule (e.g., according to a predetermined switching schedule or look up table when switches are used to control therapeutic current delivery) and/or series of resistors to shape the delivered waveform to correspond with a specified waveform or type of waveform. For example, in a non-closed loop therapeutic current control network with a resonant electrical circuit or resonant tank, current provided by an energy storage capacitor, to be delivered to the patient via the therapeutic current control network, may flow through the resonant electrical circuit or resonant tank.

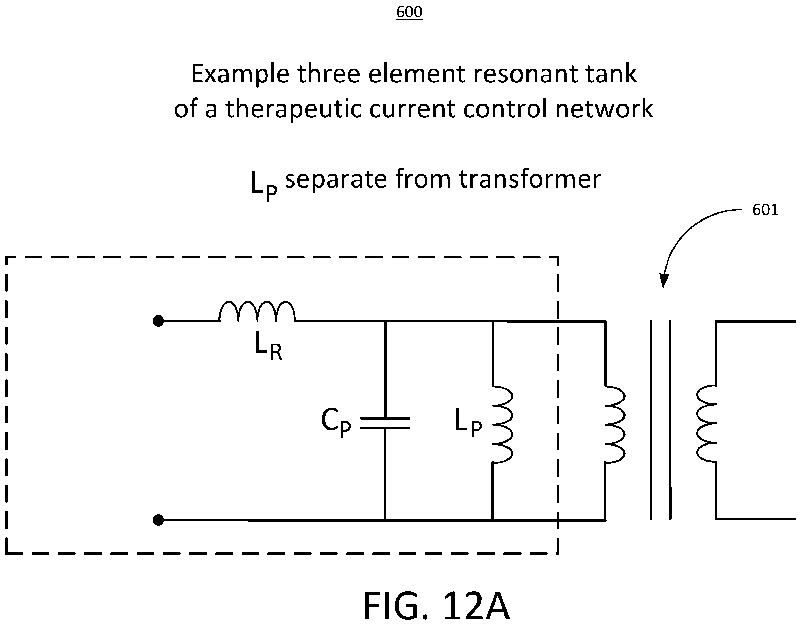

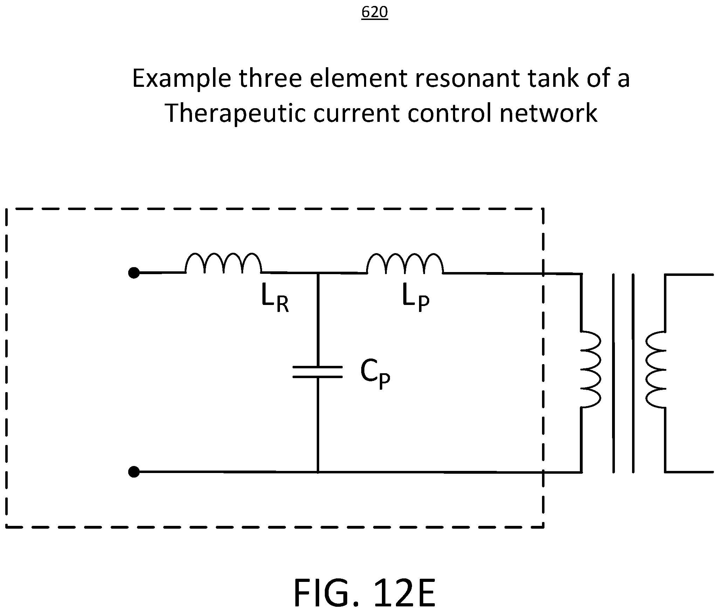

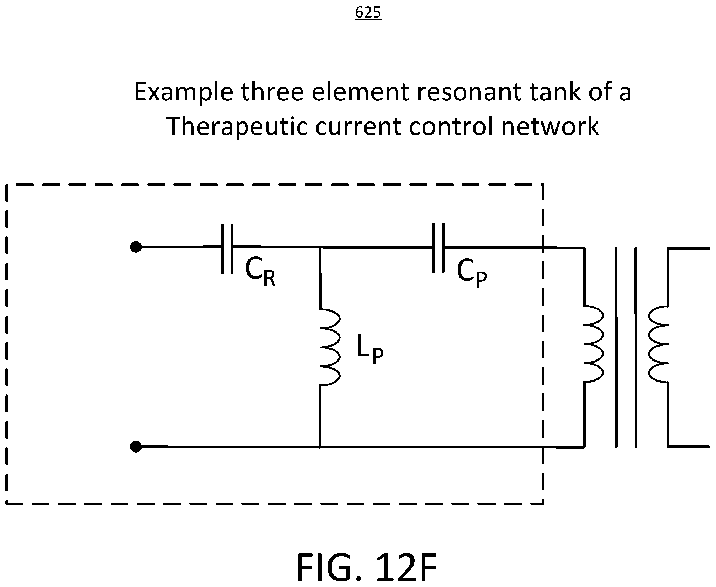

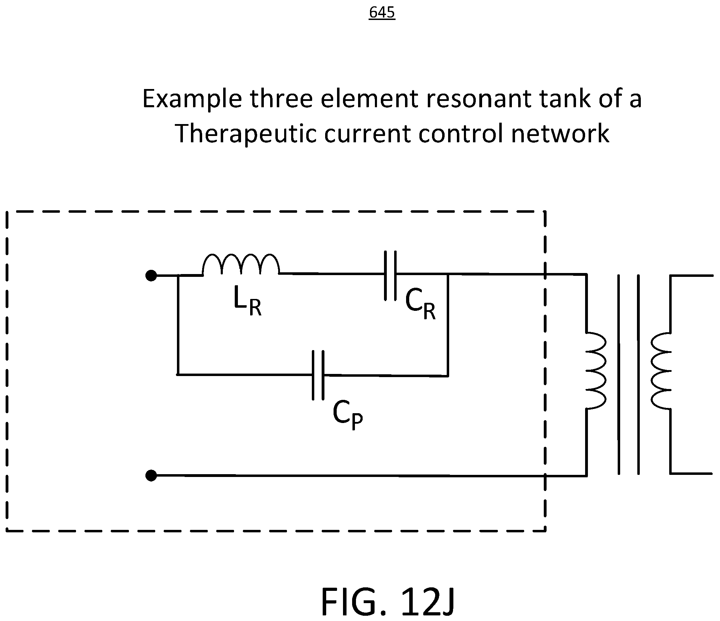

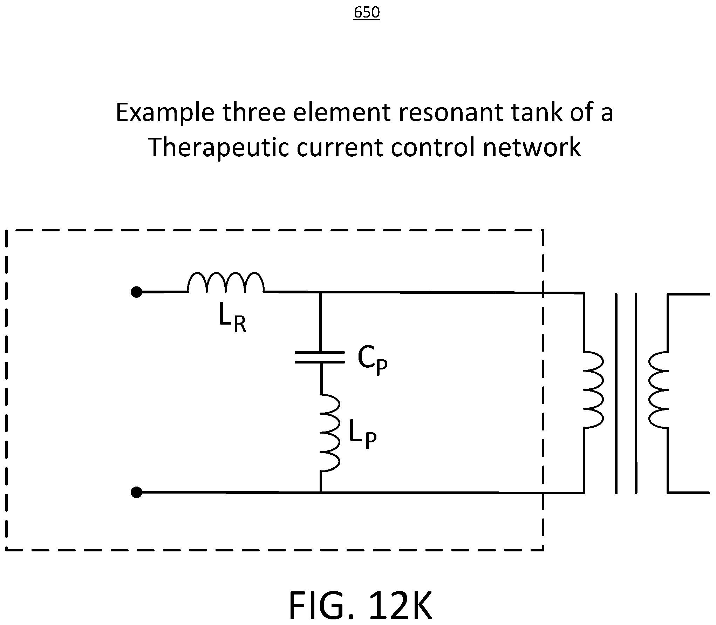

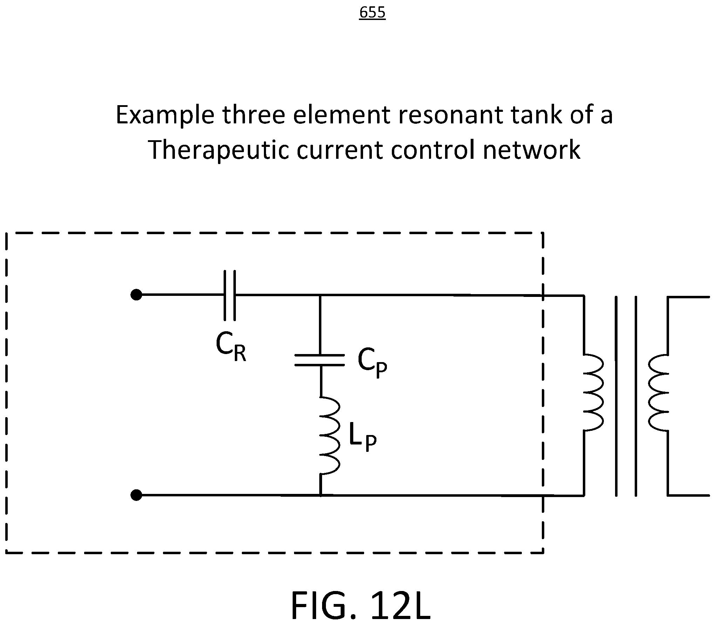

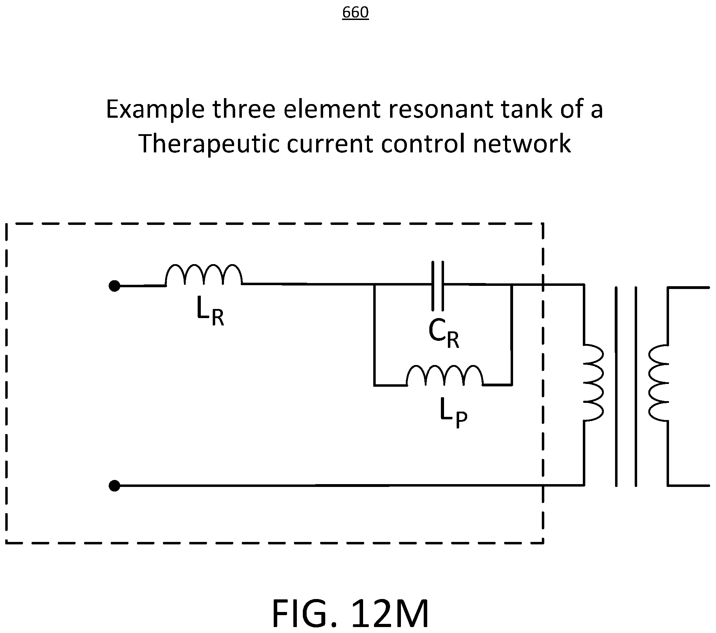

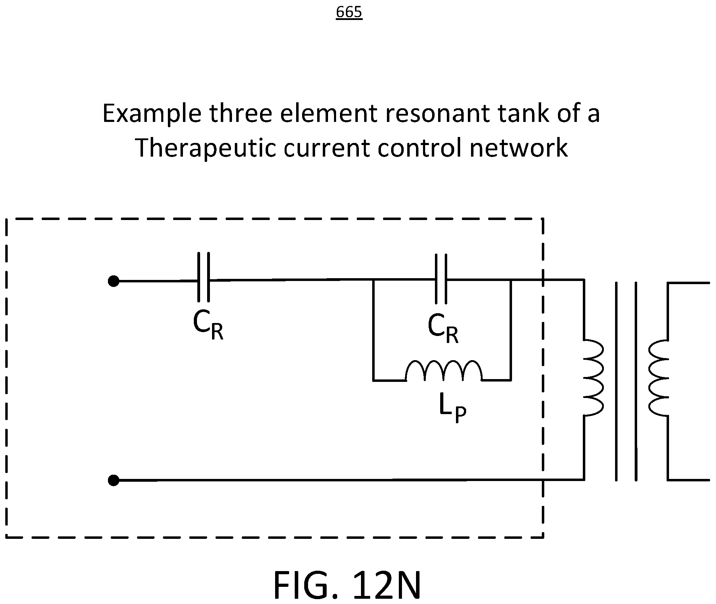

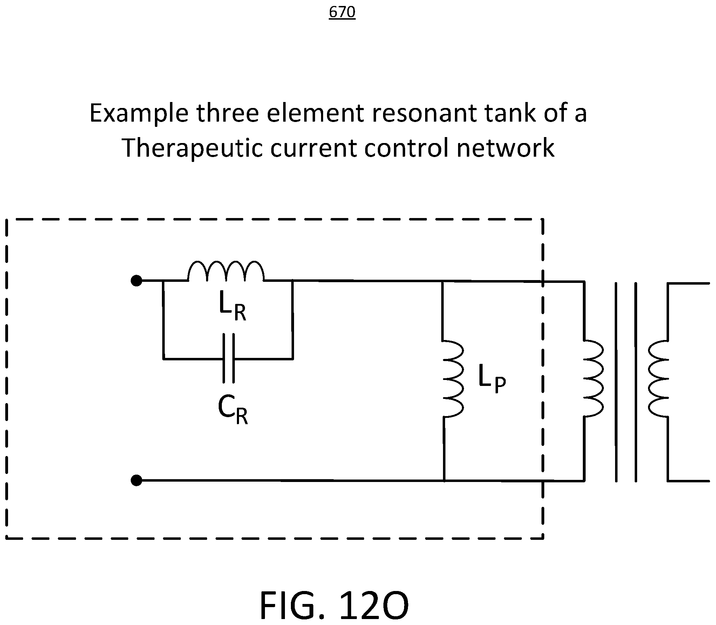

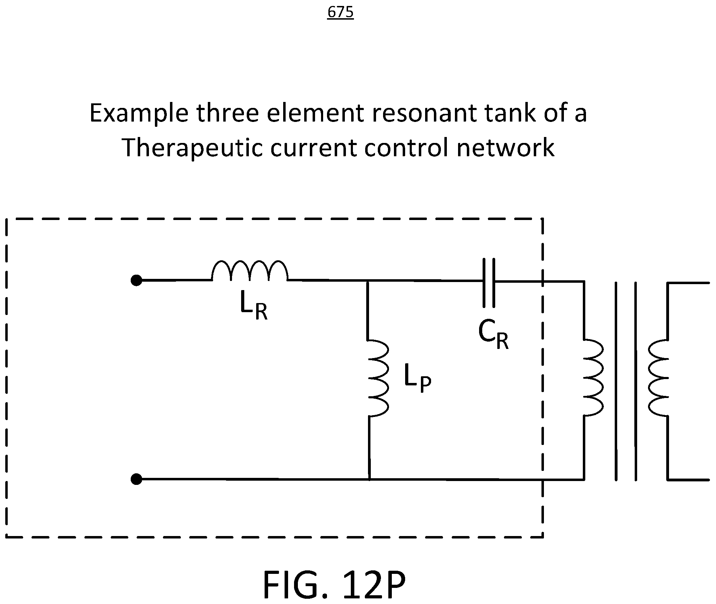

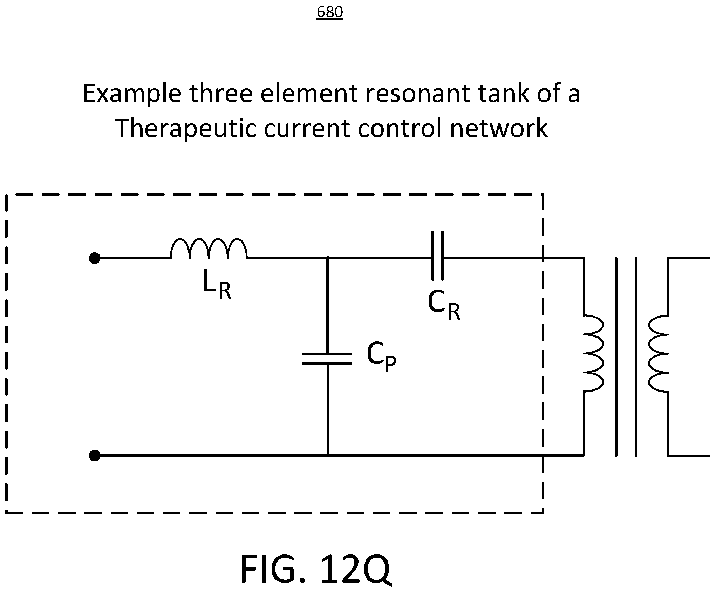

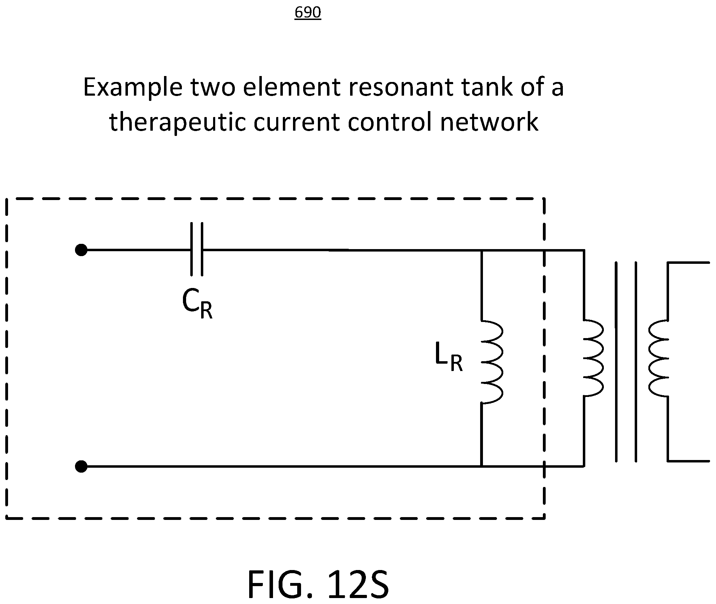

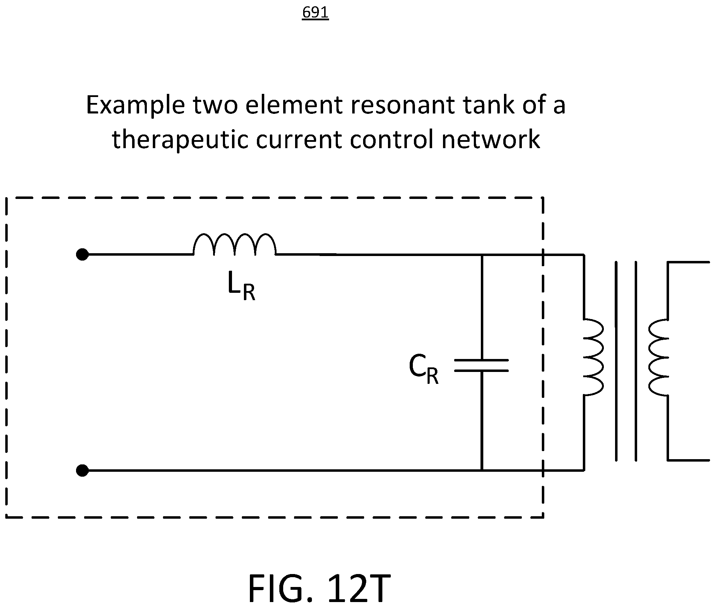

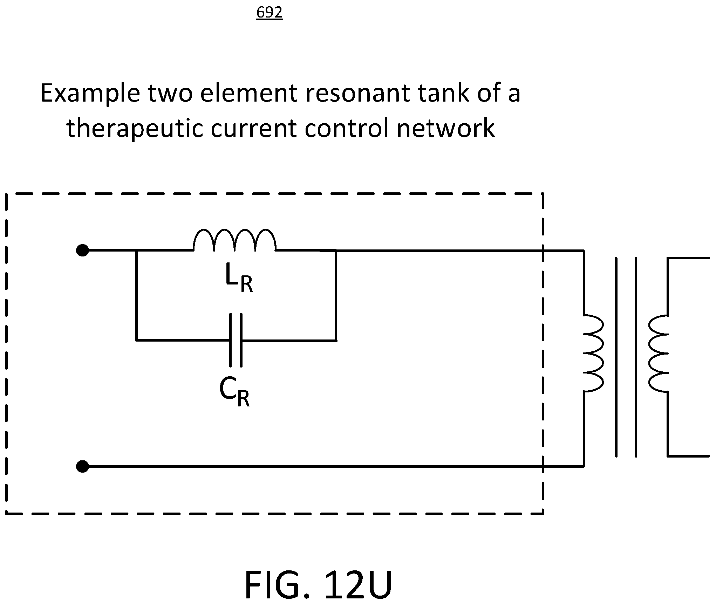

The resonant tank 283 may, in some embodiments, include one or more inductors and one or more capacitors that act as an electrical resonator, storing energy oscillating at the resonant tank's resonant frequency or frequency range. Non-limiting examples of resonant tank configurations that may be used in a therapeutic current control network are provided in FIGS. 12A - 12V. In various embodiments, a resonant tank may be beneficial to effectively store energy in a manner that minimizes or otherwise reduces the overall loss or dissipation of energy that may occur in other energy storage configurations. This may be advantageous in the context of defibrillation where a substantial amount of energy is stored and released over a short period of time. Other aspects and advantages of resonant tanks, as well as other components and aspects of therapeutic current control systems and controllers, including various electrical components and configurations, are described with reference to other figures.

In various embodiments, power supply 281 of the therapeutic current control system 275 may or may not include or make use of a bidirectional charging control network, such as may be used in connection with a battery and an energy storage capacitor of the power supply, for example.

FIG. 2B is a block diagram of an illustrative example of a therapeutic current control system 200. The therapeutic current control system 200 includes a power supply 201 electrically coupled with a therapeutic current control network 202. The power supply 201 may be or include an energy storage device such as a high voltage capacitor, which may be charged, such as by a battery or other power source, prior to delivery of electrotherapy current to the patient 207.

The therapeutic current control network 202 may include at least one current control switch 203, a resonant tank 204, a rectifier 205 and a filter 206.

Generally, by way of example and without limitation, components of the therapeutic current control network 202 may be configured or function as follows. The at least one current control switch 203 may include one or more switches that control the flow of current from the power supply 201 through sections of the therapeutic current control network 202, and ultimately to the patient 207. As described below, a controller 212 may control operation of at least a portion of the at least one current control switch 203 of the therapeutic current control network 202, and may control other components of the therapeutic current control network including the rectifier 205 or filter 206, or other components of the system 200, to control or adjust a level of current being delivered to the patient 207.

In some embodiments, the resonant tank 204 may include one or more inductors and one or more capacitors that act as an electrical resonator, storing energy oscillating at the resonant tank's resonant frequency or frequency range. Non-limiting examples of resonant tank configurations that may be used in a therapeutic current control network are provided in FIGS. 12A - 12V. In various embodiments, a resonant tank may be beneficial to effectively store energy in a manner that minimizes or otherwise reduces the overall loss or dissipation of energy that may occur in other energy storage configurations. This may be advantageous in the context of defibrillation where a substantial amount of energy is stored and released over a short period of time.

The rectifier 205 may be used in converting alternating current to direct current. The rectifier may be a passive rectifier, or it may be an active or synchronous rectifier for performing active or synchronous rectification, which may be more efficient than a passive rectifier. Diodes may be used in passive rectification, where current is effectively permitted to flow in a single direction, once a threshold potential has been reached. Alternatively, in active or synchronous rectification, diodes may be replaced by actively controlled switches, which permit current to flow upon actuation of the switch(es) according to controlled timing.

The filter 206 may be used in electrical signal frequency filtering, for example, by allowing certain frequencies to pass through the network, thereby altering the bandwidth characteristic of the current control loop.

In the illustrative embodiment of Fig. 2B, the therapeutic current control network 202 is electrically coupled with the patient 207 for delivery of current to the patient 207, and to an electrical parameter sensor 208 (or more than one) configured to sense one or more electrical parameters of the circuit that may be, alone or in combination with other available information, indicative of current flow to the patient. In some examples, the electrical parameter sensor(s) 208 is configured for sensing a current output to the patient 207 during and over a period of delivery of current to the patient. In other examples, the electrical parameter sensor 208 is configured to sense voltage.

A signal 209 corresponding with the sensed electrical parameter, which sensed signal may vary over the period of delivery of the current to the patient, is received by the controller 212, which is electrically coupled with the electrical parameter sensor 208. It can be appreciated that various other types of sensors for measuring or sensing a signal indicative of current delivered to the patient may be employed. The signal(s) 209 indicative of the sensed electrical parameter may take the form of sensing the current and/or voltage across the output circuit nodes to the patient. In some examples, the controller 212 may then determine current output based at least in part on the current and/or voltage measurement. In various embodiments, a current and/or a voltage sensor(s) may also be included and coupled with the patient, for sensing a current and/or a voltage associated with a current output to the patient 207, and one or more signals corresponding with the sensed current and/or voltage may be received an utilized by the controller 212.

In some embodiments, a controller may utilize and compare signals, and/or, in some embodiments, parameters associated with signals may be derived, calculated, generated or obtained, such as at least in part based on associated signals, and utilized or compared, or some combination of both signals and parameters may be utilized and compared. In some embodiments, a controller may derive, calculate or generate such parameters, whereas in some embodiments, such parameters may be derived, calculated or generated, at least in part, outside of the controller. Furthermore, in some embodiments, such parameters (or signals) may be obtained within the controller and/or may be obtained from outside the controller, such as by the controller requesting them and/or by the parameters (or signals) being input to the controller.

Furthermore, in some embodiments, during delivery of current to the patient 207, the impedance of the patient 207, which may vary slightly during delivery of the waveform, may be monitored and associated information may be utilized by the controller 212 to appropriately adjust, if and as necessary, the delivery of electrical therapy, to ensure that the desired waveform is as accurately as possible matched and provided to the patient. This can be an advantage of some embodiments of the present disclosure, in that many previously existing systems are not capable of monitoring and utilizing information regarding variations in patient impedance that may occur during delivery of a waveform to a patient, and therefore cannot adjust the waveform accordingly. Additionally, in some embodiments, other physiological patient parameters may be sensed and/or monitored, and obtained information may be utilized by a controller in determining or optimizing a waveform generated and delivered to the patient. It is noted that, in open loop systems, patient impedance is not actively monitored and utilized during delivery of a waveform on a continuous basis, but rather measured at a single instance to calibrate the waveform.

A specified waveform 214 is input to the controller 212, prior to or during delivery of the electrotherapy current to the patient 207. As conceptually indicated by broken circle 216 the controller 212 may be used in optionally providing closed loop control of an electrotherapeutic waveform as it is being delivered to the patient 207. When used in this context, the controller 212 may in effect monitor (by utilizing the received signal 209, for instance) and adjust the current waveform being delivered to the patient during and over the period of delivery. In a cyclical, repeating fashion over a time of delivery (typically on the order of milliseconds) of the electrotherapy current to the patient 207, based at least in part on the received signal 209, which may vary during delivery, the controller 212 may continually adjust the current being delivered to the patient to as closely as possible match the specified waveform 214. In particular, the controller 212 may control, potentially among other things, operation of at least a portion of the at least one current control switch 203 of the therapeutic current control network 202 to control or adjust a level of current or waveform being delivered to the patient 207. In various embodiments, the controller 212 operates in a non-closed loop control fashion, for example, where the electrotherapeutic waveform is predetermined according to a preset schedule such as in a look up table where switch control is prearranged so as to result in current delivery according to the desired electrotherapeutic waveform.

FIG. 2C is a block diagram of an example therapeutic current control system 250, including a battery 215 and bidirectional charging control network 216. In this illustrative embodiment, the bidirectional charging control network 216 includes a bidirectional charging control switch network 217, a resonant tank 218 or other resonant electrical circuit, a rectifier 219 and a filter 222. Although a bidirectional charging control network 216 including a resonant tank 218 is shown, in other embodiments, other types of power converters without resonant tanks can be used in the bidirectional charging, such as a buck-boost or flyback converter, for instance. However, a resonant tank based bidirectional charging control network 216 may provide more efficiency in the manner in which energy is transferred between the battery 215 and the energy storage capacitor 223.

The bidirectional charging control network 216 is used both in facilitating charging of the energy storage capacitor 223 by the battery 215 or potentially a different power source, as well as in returning energy from the energy storage capacitor 223 to the battery 215, providing charge to the battery 215 to prolong its storage life. In various embodiments, bidirectional charging control networks are provided that may be used with or in closed or non-closed loop current control systems. The energy storage capacitor 223 is charged prior to use in providing energy for generating and delivering an electrotherapeutic waveform to a patient. The bidirectional charging control network 216 may be used in returning at least a portion of such excess energy back from the energy storage capacitor 223 to the battery 215 to be stored at the battery 215. This can reduce waste and produced heat, as well as increase battery life, increase battery uses available before recharge of the battery, or allow for use of a smaller battery. As such, it can contribute to increased system efficiency and potentially smaller and lighter system size, a smaller cooling system, and smaller battery size, for example, as may be appropriate or needed for a particular use or a given set of performance requirements or minimum performance requirements. For example, systems where unused energy is dumped from the energy storage capacitor require circuit components (e.g., resistors, heat sinks) dedicated to address the effects of this action in a manner that does not detrimentally affect other parts of the system. Hence, a bidirectional charging control network may alleviate the necessity to build in such additional components, which could otherwise lead to inefficiencies and space constraints.

A therapeutic current control network 238 is electrically coupled with the energy storage capacitor 223 and includes at least one current control switch 224, a resonant tank 225, a rectifier 226, a filter 227 and a polarity control element 228, although, in some embodiments, one or more of the rectifier 226, the filter 227 and the polarity control element 228 may not be included. The polarity control element 228 may be for use in control of direction of current flow. It may be therapeutically beneficial for an electrical current to pass through a patient in two directions. For example, in the case of defibrillation, the polarity control element 228 may provide for a biphasic shock where the direction of current flow is reversed during electrical discharge. In some embodiments, the polarity control element 228 employs an H-bridge switch configuration. Details of example resonant tank configurations that can be used in various embodiments of a therapeutic current control network 238 are described herein with reference to FIGS. 12A-12V. It is noted that other types of therapeutic current control networks can be utilized, various embodiments of which are described herein

The therapeutic current control network 238 is electrically coupled with the patient 229 as well as current and/or voltage sensors 232 for sensing a current level and/or voltage level associated with or output to the patient 229. In various embodiments, various systems described herein may include an electrical parameter sensor configured to sense at least one electrical parameter from which a current flow to the patient / patient load can be determined or estimated. In some embodiments, the sensor 1216 may sense an electrical parameter to or through the patient. In some embodiments, however, a sensor may not directly sense an electrical parameter at or through the patient, but may sense an electrical parameter elsewhere, such as at another node within a circuit of a therapeutic current control network, and generate signal(s) based at least in part on which current to the patient can be determined or estimated. For example, in some embodiments, the average current through at least one current control switch may be measured and used, potentially among other data or measured parameters, in determining or estimating the current through the patient.

A controller 233 receives signals 237 corresponding with the sensed current and/or voltage. Various types of controllers are contemplated in different embodiments. In the embodiment depicted in FIG. 2C, the controller 233 may be field programmable gate array (FPGA) based or digital signal processor (DSP) based, as depicted by block 234. As depicted, the controller 233 also includes an analog to digital converter (ADC) 235 that receives the signals 237 corresponding with current and voltage output to the patient 229. The controller 233 may also include various other components that are not depicted.

The controller 233 controls at least a portion of the at least one current control switch 224 of the therapeutic current control network 238, and may control other aspects of operation of the therapeutic current control network 238 or of the system 250, to adjust current being delivered to a patient or for other purposes. The controller 233, when optionally used in a closed loop fashion as conceptually depicted by broken circle 239, adjusts a current level being delivered to the patient 229 to match a specified current waveform 236 input to the controller 233, and may perform other control operations within the system 250 as well. In various embodiments, the controller 233 is used in a non-closed loop control fashion, as discussed herein, for example, where the electrotherapeutic waveform is predetermined according to a preset schedule such as in a look up table where switch control is prearranged so as to result in current delivery according to the desired electrotherapeutic waveform.

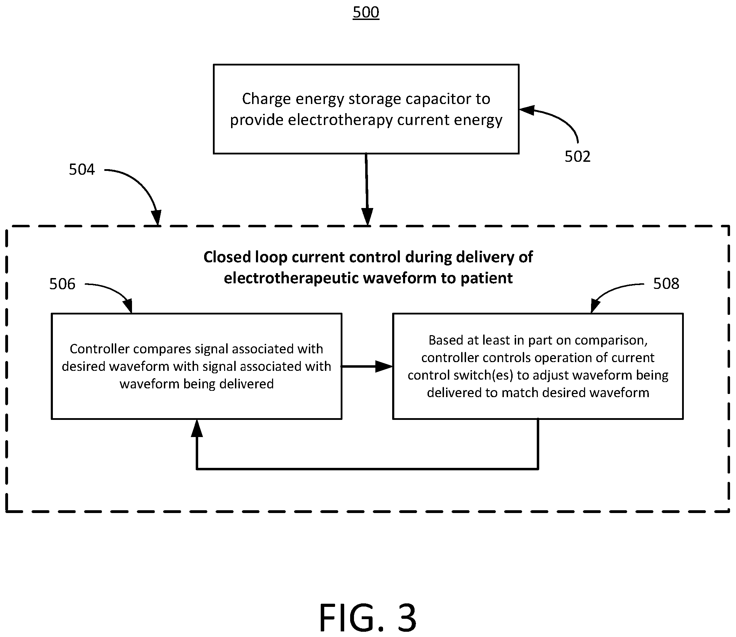

FIG. 3 is a flow diagram of an example method 500 for closed loop therapeutic current control, when optionally implemented. At step 502, an energy storage capacitor, such as a high voltage capacitor, is charged to provide energy for electrotherapy current, such as for defibrillation or pacing. Broken box 504 conceptually represents closed loop current control used during delivery of an electrotherapeutic waveform to a patient, using energy provided by the energy storage capacitor. At step 506, the controller compares one or more signals associated with a specified waveform with one or more signals associated with a waveform being delivered to the patient, such as, for example, a signal corresponding with a current output to a patient. At step 508, based at least in part on this comparison, the controller controls, at least, operation of at least one current control switch of a closed loop therapeutic current control system to adjust the electrotherapeutic waveform being delivered to the patient to match the specified waveform.

FIG. 4A is a block diagram of an illustrative example of a therapeutic current control system 400. The power supply, one or more current control switches (which may be or include high voltage MOSFET switches, which may be SiC based, for example), rectifier, filter, electrical parameter sensor(s), controller, signal(s) indicative of current output to patient, and specified current waveform, and potentially among other features and components, may be analogous or similar to those components as depicted in other figures and embodiments. However, various details of various components may differ, and the arrangement or placement, such as within a circuit, of various components may also differ, such as the components, and arrangement of components, of the therapeutic current control network 401. Although, in the embodiment depicted, the electrical parameter sensor(s), closed loop current control and signals indicative of current output to the patient 408 are included, in other embodiments, some or all of these components may not be included or used, and, in some embodiments, the system 400 may operate using non-closed loop current control, for example.

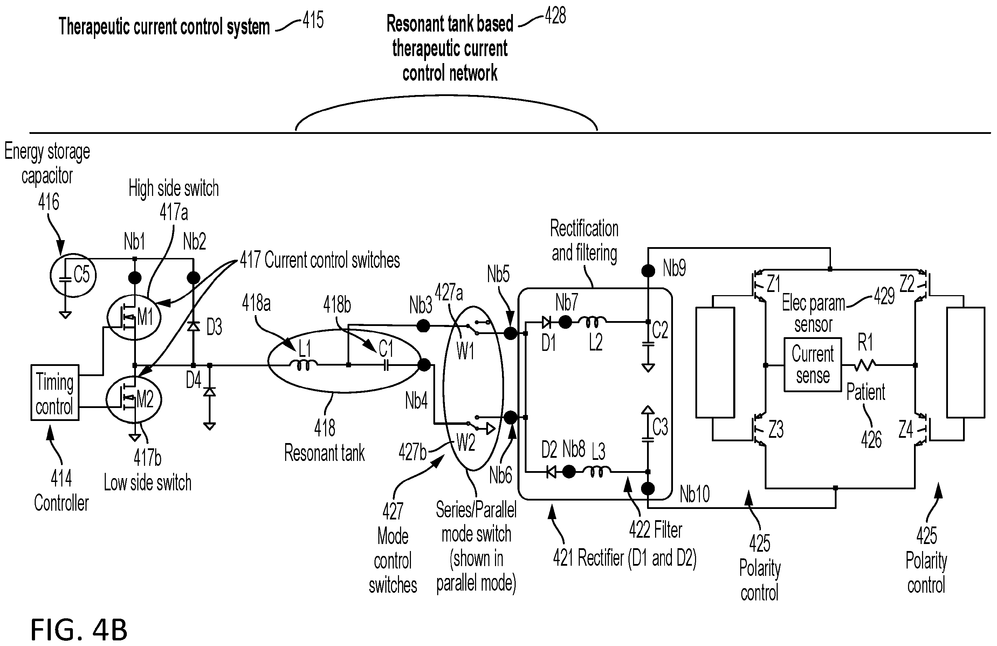

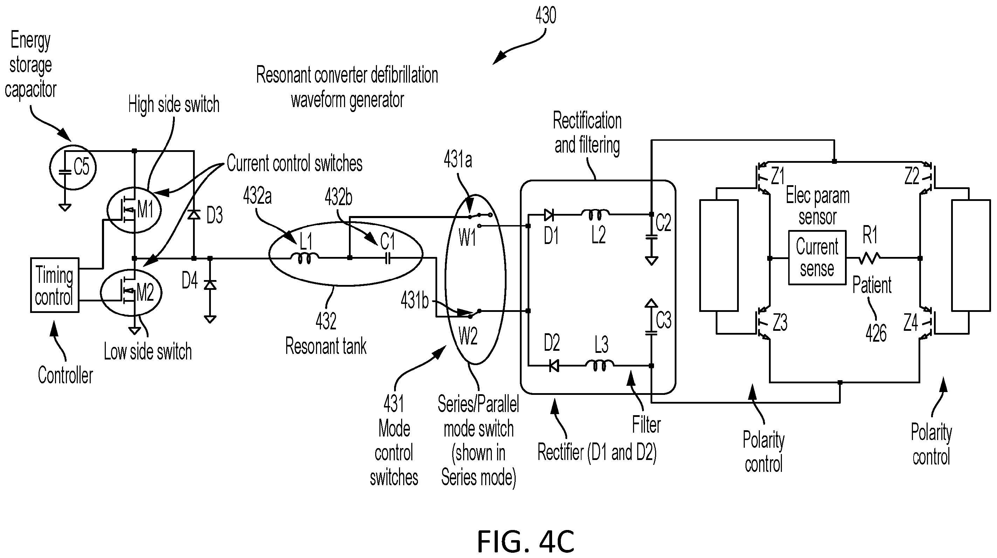

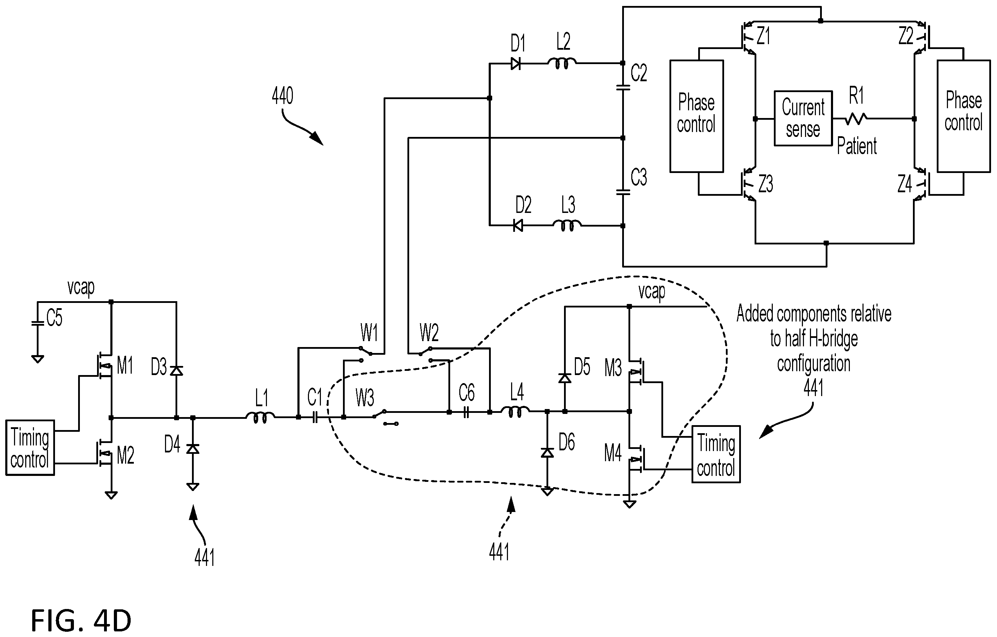

The embodiments described with reference to FIGs. 4-11 herein include LC resonant tanks (resonant tanks with one inductor and one capacitor), which may include parallel or series resonance (as described further below). However, in other embodiments, other types of resonant tanks may be used, including various resonant tanks shown in FIGs. 12A-12V herein, for example.

In FIG. 4A, the therapeutic current control network 401 includes one or more mode control switches 404. In some embodiments, the controller 411 controls a configuration of the one or more mode control switches 404 to control a mode of operation of the therapeutic current control network 401. In some embodiments, the network 401 may be configured to be operable in one of two modes, although some embodiments may include more than two modes. The two modes may include a first mode in which the resonant tank 405 is connected with the patient 408 in a parallel configuration and a second mode in which the resonant tank 405 is connected with the patient 408 in a series configuration. Energy provided by power supply 402 flows through the resonant tank 405. When the resonant tank 405 is connected with the patient 408 in a parallel configuration, one element of the resonant tank 405 (e.g., an inductor or a capacitor) is connected with the patient in a parallel configuration. When the resonant tank 405 is connected with the patient 408 in a series configuration, all elements of the resonant tank 405 (e.g., all inductors and all capacitors) are connected with the patient in a series configuration. In some embodiments, as described further herein, a resonant tank may be used, or may sometimes be used, to boost a voltage of an electrotherapeutic waveform delivered to a load, such as a patient load.

In some embodiments, parallel mode may be selected for, and may be more optimal for, higher impedance patient loads, whereas series mode may be selected for, and may be more optimal for, higher impedance patient loads. In some embodiments, as described previously, use of parallel mode may help maximize the Q factor for higher impedance loads, whereas use of series mode may help maximize the Q factor for higher impedance loads

FIG. 4B is an exemplary schematic diagram of a therapeutic current control system 415 with parallel resonance mode and series resonance mode capabilities, shown in parallel resonance mode. The system 415 may be an embodiment of the system 400 of FIG. 4A. As depicted, the system 415 includes an energy storage capacitor 416 (C5) connected with a resonant tank based therapeutic current control network 428. The network 428 includes current control switches 417 (which may be or include high voltage MOSFET switches, which may be SiC based, for example), a resonant tank 418, mode control switches 427, a rectifier 421, a filter 422 and a polarity control 425. The network 428 is connected with a patient 426, and an electrical parameter sensor 423 is connected with the patient 426. The current control switches 417 comprise two switches, specifically, a high side switch 417a (M1) (power supply side) and a low side switch 417b (M2) (ground side). The resonant tank 418 includes a resonant inductor 418a (L1) and a resonant capacitor 418b (C1). The mode control switches 427 include mode control switch 427a (W1) and mode control switch 427b (W2). The rectifier 421 includes diodes D1 and D2. The filter 422 includes filter inductors L2 and L3 and filter capacitors C2 and C3. The polarity control 425 includes switches Z1, Z2, Z3 and Z4. Various other components may be included that are not depicted. In some embodiments, the system may utilize zero voltage switching and/or zero current switching, such as at switch turn on and switch turn off. In some embodiments, as described further herein, a resonant tank may be used, or may sometimes be used, to boost a voltage of an electrotherapeutic waveform delivered to a load, such as a patient load.

In the configuration of the system 415 of FIG. 4B, mode control switch 427a (W1) is in a configuration connecting node Nb3 to node Nb5, while mode control switch 427b (W2) is in a configuration connecting node Nb4 to ground. As such, the resonant tank capacitor 418b (C1) is connected with the patient 426 in a parallel configuration. Therefore, the resonant tank 418 is connected with the patient 426 in a parallel configuration and, as such, the system 415 is in parallel resonance mode.

While various nodal configurations may be used, in some embodiments, including in the configuration of the system 415 of FIG. 4B, the following nodal connections are used. The energy storage capacitor 416 and a first portion of the network 428 including the current control switches 417 and the resonant tank 418 are connected via nodes Nb1 and Nb2. The first portion of the network 428 and a second portion of the network 428 including the mode control switches 427 are connected via nodes Nb3 and Nb4. The second portion of the network 428 and a third portion of the network 428 including the rectifier 421 are connected via nodes Nb5 and Nb6. The third portion of the network 428 and a fourth portion of the network 428 including the filter 422 are connected via nodes Nb7 and Nb8. The fourth portion of the network 428 and a fifth portion of the network 428 including the polarity control 425, the patient 426 (the patient load), and electrical parameter sensor 429, are connected via nodes Nb9 and Nb10.

In some embodiments, operation of the system 415 of FIG. 4B may include the following. Energy stored in the energy storage capacitor 416 may be applied to the resonant tank 418 via the current control switches 417. This generates a circulating current in the resonant tank 418, the amplitude of which depends on the frequency of operation of the current control switches 417. Diodes D3 and D4 are used in providing a current path for the circulating current, based on polarity.

The controller 414 controls the switching frequency of the current control switches 417 to adjust the current applied to the patient 426, such as to make adjustments to deliver a particular waveform or to match a specified waveform. Increasing the switching frequency of the current control switches 417 decreases the current and voltage delivered to the patient 426, while decreasing the switching frequency (timing) of each of the current control switches 417 increases the current and voltage delivered to the patient 426. Operation of the current control switches 417 above a resonant frequency of the resonant tank 418 results in reducing the voltage and current being delivered to the patient 426.

By the current control switches 417 operating above the resonant frequency of the resonant frequency of the resonant tank 418, high side current control switch 417a (M1) only switches on when current is flowing through diode D3 and low side current control switch 417b (M2) only switches on when current is flowing through diode D4. In some embodiments, the two current control switches 417 are used in allowing the resonant tank 418 to be energized by an alternating current. This provides substantial advantage in terms of reduced switching losses, allowing high frequency operation of the current control switches 417, which in turn allows the resonant tank inductor 418a and capacitor 418b to be relatively physically small.

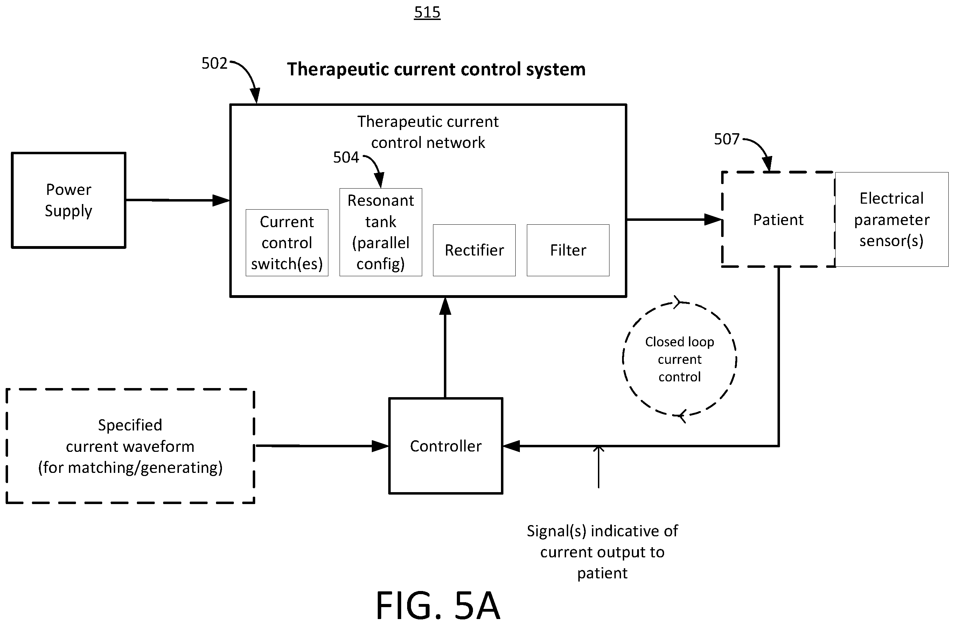

Additionally, operating in parallel mode, as depicted in FIG. 4B allows the voltage delivered to the patient 426 to be higher than the voltage of the energy storage capacitor 416. This, among other things, allows for more complete depletion (and less residual charge) of the energy of the energy storage capacitor 416, relative to a various other systems. As such, for example, for a particular required electrotherapeutic or defibrillation energy level, a lower capacitor voltage may be used, allowing use of a lower voltage, smaller, lighter capacitor, and thereby reducing overall system 415 size (and/or footprint), weight and cost. Furthermore, embodiments described herein, including those depicted in FIGs. 4A-D and 5A-B, can allow for greater simplicity and fewer components overall, as well as greater energy efficiency, relative to other systems.

The current (or voltage) delivered to the patient 426 is monitored (whether, for example, directly measured using a sensor in series with the patient 426 or determined based at least in part on a potentially different level of current or voltage measured in a different portion of the network 428, from which the level of current or voltage to the patient can be determined, calculated or estimated. Signal(s) indicative of the current are provided as input to the controller 414. The controller 414 may use the input signals in regulating, such as by adjusting by increasing or decreasing, the level of current being delivered to the patient 426, such as to match a specified waveform also input (and/or stored in) the controller 414.

Based on the configuration of the mode control switches 427, with mode control switch 427a (W1) connecting nodes Nb3 to node Nb5 and mode control switch 427b (W2) connected node Nb4 to ground, the resonant tank 418 is connected with the patient 426 in a parallel configuration. Alternatively, as shown in FIG. 4C, based on the opposite configuration of the mode control switches 431, with mode control switch 431a (W1) open and mode control switch 431b (W2) connecting capacitor 432b (C1) to diodes D1 and D2, the resonant tank 432 is connected with the patient 426 in a series configuration.

The alternating current from the resonant tank 418 flows to the rectifier 421 (D1 and D2), then to filter 422 including filter inductors L2 and L3 and filter capacitors C2 and C3, leading to the current delivered to the patient 426. The polarity control 425, including switches Z1, Z2, Z3 and Z4, is used to control the polarity of the current applied to the patient 426.

FIG. 4C is an exemplary schematic diagram of a therapeutic current control system 430 with parallel resonance mode and series resonance mode capabilities, shown in series resonance mode. More specifically, the system 430 of FIG. 4C is identical in terms of components to the system 415 of FIG. 4B. However, in the configuration of the system 430 of FIG. 4C, mode control switch 431a (W1) (which is the same component as mode control switch 427a (W1) of FIG. 4B but in a different configuration) is in an open configuration, while mode control switch 431b (W2) (which is the same component as switch 427b (W2) of FIG. 4B but in a different configuration) is in a configuration connecting capacitor C1 to diodes D1 and D2. As such, regarding the resonant tank 432 of FIG. 4C (which includes the same components as resonant tank 418 of FIG. 4B), both the resonant tank inductor 432a (L1) (which is the same component as the resonant tank inductor 418a (L1) of FIG. 4B) and the resonant tank capacitor 418b (C1) (which is the same component as the resonant tank capacitor 418b (C1) of FIG. 4B) are connected with the patient 426 in a series configuration. Therefore, the resonant tank 432 of FIG. 4C is connected with the patient in a series configuration and, as such, the system 415 is in series resonance mode.

In some embodiments, as described previously, use of parallel mode, such as shown in FIG. 4B, may help maximize the Q factor for higher impedance loads, whereas use of series mode, such as shown in FIG. 4C, may help maximize the Q factor for higher impedance loads.

FIG. 4D is an exemplary schematic diagram of a therapeutic current control system 440 implemented using a full bridge configuration 441 in association with current control switches, shown in parallel resonance mode. Relative to the systems 400, 415 of FIGs. 4B and 4C, the system 440 of FIG. 4D has additional components including mode control switch W3, inductor L4 and capacitor C6 (which, in this embodiment, form part of the resonant tank, in addition to inductor L1 and capacitor C1), diodes D5 and D6, and current control switches M3 and M4 (which may be or include high voltage MOSFET switches). The addition of capacitor C6 and inductor L4 minimizes common mode noise output, which otherwise could be much higher. The addition of current control switches M3 and M4, as well as the associated diodes D5 and D6, are added for the full bridge configuration 441.

In the system 440 of FIG. 4D, additional mode control switch W3 is used for transitioning between parallel mode (as shown in FIG. 4D) and series mode (in which each of the current control switches W1, W2 and W3 are in the opposite configurations. In some embodiments, while adding some components, relative to, for example, the embodiments depicted in FIGs. 4B and 4C, an advantage of a full bridge embodiment, such as the embodiment depicted in FIG. 4D, is that gain can be greater, allowing for a lower capacitor voltage as well as a lower voltage rating required for current control switches M1-M4.

FIG. 5A is a block diagram of an illustrative example of a therapeutic current control system 515, in accordance with embodiments of the present disclosure. The therapeutic current control network 502 of FIG. 5A includes a resonant tank 504 that is connected with the patient 507 in a parallel configuration, and, as such, the system 515 has parallel resonance.

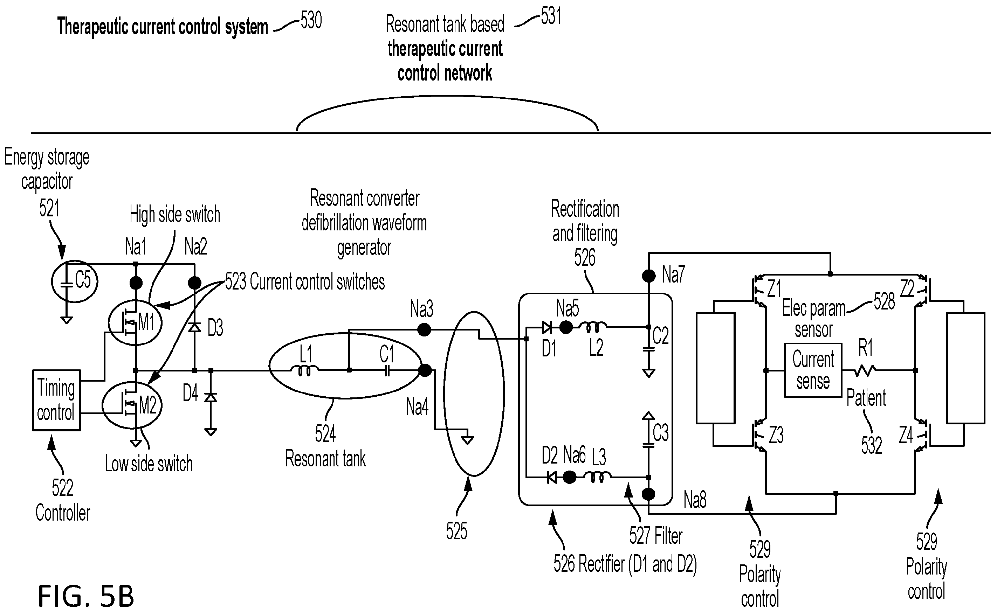

FIG. 5B is an exemplary schematic diagram a therapeutic current control system 520 with parallel resonance, which is one example embodiment corresponding with the system 515 of FIG. 5A. While various nodal configurations may be used, in some embodiments, including in the configuration of the system 530 of FIG. 5B, the following nodal connections are used. The energy storage capacitor 521 and a first portion of the network 531 including the current control switches 523 (which may be or include high voltage MOSFET switches, which may be SiC based, for example) and the resonant tank 524 are connected via nodes Na1 and Na2. The first portion of the network 531 and a second portion of the network 531 including the rectifier 526 are connected via nodes Na3 and Na4. The second portion of the network 531 and a third portion of the network 531 including the filter 527 are connected via nodes Na5 and Na6. The third portion of the network 531 and a fourth portion of the network 531 including the polarity control 529 and electrical parameter sensor 528 connected with the patient 532, are connected via nodes Na7 and Na8.

In various embodiments, therapeutic current control systems are provided that include resonance - that is, a resonant electrical circuit or resonant tank, in which energy provided by an energy storage capacitor flows through the resonant electrical circuit, which can impart many advantages that have been described herein, including advantages relating to reduced system complexity, fewer components, lighter weight, smaller size, greater portability, greater efficiency, higher energy densities, greater accuracy and precision, closed loop current control capability, and other advantages.

Furthermore, in some embodiments, such as some embodiments of the systems described with reference to FIGs. 4A-4D and 5A-5B herein, among others, parallel resonance may be used, or may sometimes be used, and may provide advantages or increased advantages. For example, parallel resonance may be used in generating or boosting a voltage delivered to the patient that is greater than a voltage of the energy storage capacitor - that is, in this regard, producing a gain of greater than 1, or greater than unity. This, in turn, can lead to various advantages. Generally, for example, it can allow for portions of a circuit to not be exposed to the very high voltage that may be delivered to the load. This, in turn, can lead to the ability to use smaller or lighter circuit components. For example, it can allow use of a lower voltage capacitor, which can be a smaller and lighter capacitor, thereby helping to reduce system size, footprint and weight. Furthermore, use of a lower voltage capacitor can provide increased energy efficiency and reduced residual (unused) charge on the capacitor. Moreover, use of a lower voltage capacitor can allow use of smaller, simpler or lighter components such as switches with lower voltage ratings, further adding to the provided advantages. Additionally, parallel resonance can be particularly efficient when used high impedance loads, including high impedance patient loads (e.g., loads of over 30, 35, 40, 45, 50, 55, 60, 65 or 70 ohms, and up to, e.g., 200, 250, 300 or 350 ohms). With use of parallel resonance, generally, a high impedance load may lead to a voltage drive to the load, which may be desirable in various applications. Furthermore, as described previously, for a resonant electrical circuit, with parallel resonance, a value of the Q factor varies proportionately with R, which is associated with the patient impedance load. As such, use of parallel resonance, may help maximize the value of the Q factor, particular for higher impedance loads.

Furthermore, in some embodiments, such as some embodiments of the systems described with reference to FIGs. 4A-4D and 5A-5B herein, among others, electrical isolation of the patient from the energy storage capacitor may not be required, needed or used, and no transformer may be required or used that is electrically connected between the energy storage capacitor and the patient. Since such a transformer may add complexity, size and weight to a system, embodiments that do not include such a transformer may accordingly provide advantages of reduced system size and weight relative to inclusion of such a transformer. Some embodiments that do not include such a transformer include parallel resonance, or can include parallel resonance, to generate gain of greater than unity as described above.

In some embodiments, such as the embodiments described with reference to FIGs. 4A-4D and 5A-5B, parallel or series resonance modes may be used. In some embodiments, for example, a mode may be determined by the controller, such as may include use of one or more algorithms. A mode may be selected, for example, by appropriate control by the controller of one or more mode control switches, such as based on which mode may be more appropriate, efficient, or effective under particular, anticipated or current circumstances or conditions. With regard specifically to series and parallel modes, for a resonant electrical circuit, with parallel resonance, a value of the Q factor varies proportionately with R, where R is associated with the patient impedance load. As such, in some embodiments, use of parallel resonance for higher patient impedance loads may help maximize the value of the Q factor. By contrast, with series resonance, a value of the Q factor varies inversely proportionately with R. As such, in some embodiments, use of series resonance for lower patient impedance loads may help maximize the value of the Q factor.

For example, in some embodiments, parallel mode may be optimal and selected for relatively high impedance loads, such as patient loads above (or at or above) a certain threshold (e.g., loads of over 10, 20, 30, 35, 40, 45, 50, 55, 60, 65 or 70 ohms, and up to, e.g., 100, 150, 200, 250, 300, 350 ohms or over 350 ohms), while series resonance mode may be optimal and selected for relatively low impedance loads, such as patient loads below (or at or below) a certain threshold (e.g., loads of under 10, 20, 30, 35, 40, 45, 50, 55, 60 or 70 ohms). In some embodiments and instances, series resonance may be more efficient or otherwise preferred over parallel resonance for lower impedance loads. The ability to switch between and select an optimal mode can be very valuable, for example, in defibrillation applications, where patient load impedance values can vary substantially, such as, for example, between 10 ohms (or more or less) and 300 ohms (or more or less). As such, in some embodiments, patient load impedance may be sensed/detected and input to the controller, or the controller may determine it, and the controller may utilize it in determining and selecting an optimal mode.

Reasons for the foregoing may include the following. With parallel resonance, all of the voltage on the resonant tank is applied to the load. At higher load impedances, this leads to higher current through the resonant tank, which leads to more optimal system operation. However, at lower impedances, it leads to a low current through the resonant tank, which can lead to less optimal system operation. By contrast, with series resonance, all of the current through the resonant tank is applied to the load. At lower load impedances, this leads to higher voltages on the resonant tank, which leads to more optimal system operation. However, at higher load impedances, it leads to lower voltage on the resonant tank, which can lead to less optimal system performance. For these reasons, potentially among others, it may be optimal to select parallel resonance for higher load impedances, potentially among other factors, whereas it may be optimal to select series resonance for lower load impedance, potentially among other factors. As such, in some embodiments, a controller controls selection of parallel resonance or series resonance according to which may be determined or assessed to be optimal for a particular set of circumstances, which circumstances may include the load impedance level.

In some embodiments, in determining and selecting a mode, factors other than, or in addition to, patient load impedance may be, or may also be, taken into account, such as, for example, factors relating to system or network parameters, or others. For example, in some embodiments, a mode may be selected based at least in part on factors including both the load impedance and the resonant tank impedance. More particularly, in some embodiments, mode selection may be based at least on part on a ratio of the load impedance to the resonant tank impedance. In some embodiments, if the ratio is above (or at or above) a particular threshold value, then parallel mode is selected, whereas, if the ratio is below (or at or above) a particular threshold value, then series mode is selected.

In some embodiments, one or more algorithms may be used and executed by the controller in determining and selecting an optimal mode, which algorithm may incorporate a number of parameters, which may include patient load impedance as one parameter. In some embodiments, such an algorithm may, for example, utilize a calculated mode selection parameter, where parallel mode is selected for mode selection parameter values of a certain range (e.g., at or above a threshold value) and series mode is selected for mode selection parameter values of a different range (e.g., below a threshold value). The value of the mode selection parameter (or the threshold value, or both) may be calculated, for example, based on a mathematical formula including values for multiple other parameter values, or appropriately weighted other parameter values, one of which other parameters may be patient load impedance.

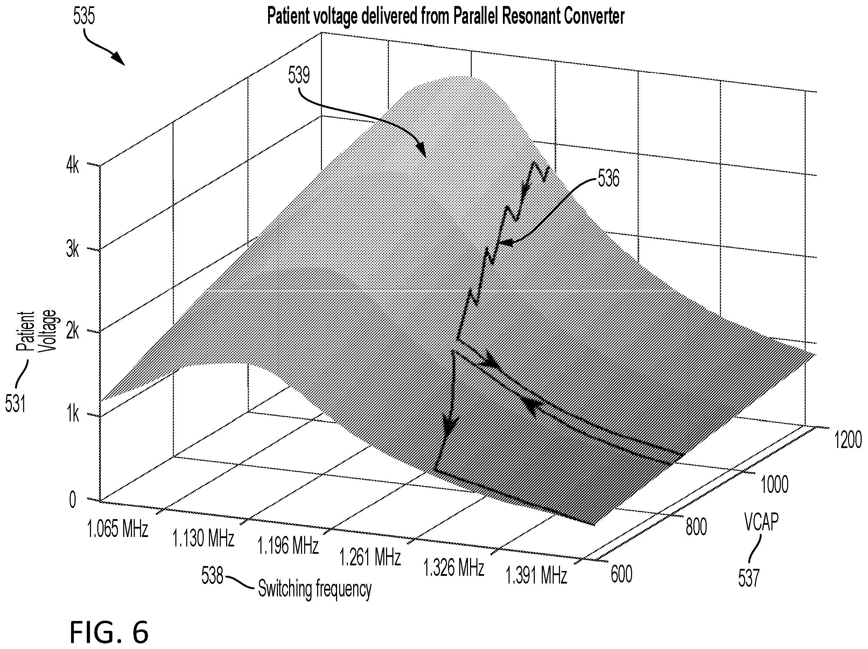

FIG. 6 is a three-axis graphical illustration of example simulation results 535 relating to use of a therapeutic current control network with parallel resonance, such as may be generated by embodiments depicted herein, including, for example the embodiments depicted in FIGs. 4A-B and 4D. While not depicted, the patient load impedance is 200 ohms. The depicted axes include a vertical patient voltage axis 531 (voltage delivered to the patient), a horizontal energy storage capacitor voltage axis 537 and a horizontal current control switch(es) switching frequency axis 538. A capacitor voltage range of 1200V to 600V is shown, which can correspond to a decreasing capacitor voltage from an initial charge of 1200V, where the capacitor voltage decreases as it discharges during delivery of a waveform to the patient, for example. Among other things, the results 535 show that therapeutic current control networks with parallel resonance, according to embodiments described herein, can be used to generate and deliver various waveforms over a large range of capacitor voltages.

The depicted curve 536 identifies a set of points on the surface 539 that correspond with a set of patient voltages - that is, desired voltages to be delivered to the patient over a very short period of time, though time is not a dimension specifically included in FIG. 6. Each point, of the identified set of points, also corresponds with a particular capacitor voltage, from 1200V to 600V, which could represent decreasing capacitor voltage over time. Finally, each point corresponds with a particular current control switch(es) switching frequency that would be used to generate the desired patient voltage at each of the associated capacitor voltage. As such, the set of points (represented by the curve 536) essentially provides an indication of a set of current control switch switching frequencies that could be used to generate a particular patient voltage waveform corresponding to the associated patient voltages, for a capacitor with a particular pattern of varying/decreasing voltage. Curve 536 shows one curve along the surface 539. However, of course, many other curves along the surface 539 are also possible, which could show, for example, different corresponding sets of anticipated capacitor voltages, desired patient voltages, and required current control switch(es) frequencies needed to generate the desired patient voltages.

As shown by the surface 539, generally, for a given capacitor voltage (on the simplifying assumption that capacitor voltage stayed constant, while actually it may be decreasing), as a higher patient voltage is needed, the current control switch switching frequency would need to be decreased (e.g., by a controller), and vice versa. Furthermore, generally, as capacitor voltage decreases, for a given current control switch(es) switching frequency, the patient voltage would decrease. As such, as capacitor voltage decreases, maintaining a constant patient voltage could require decreasing the current control switch switching frequency.

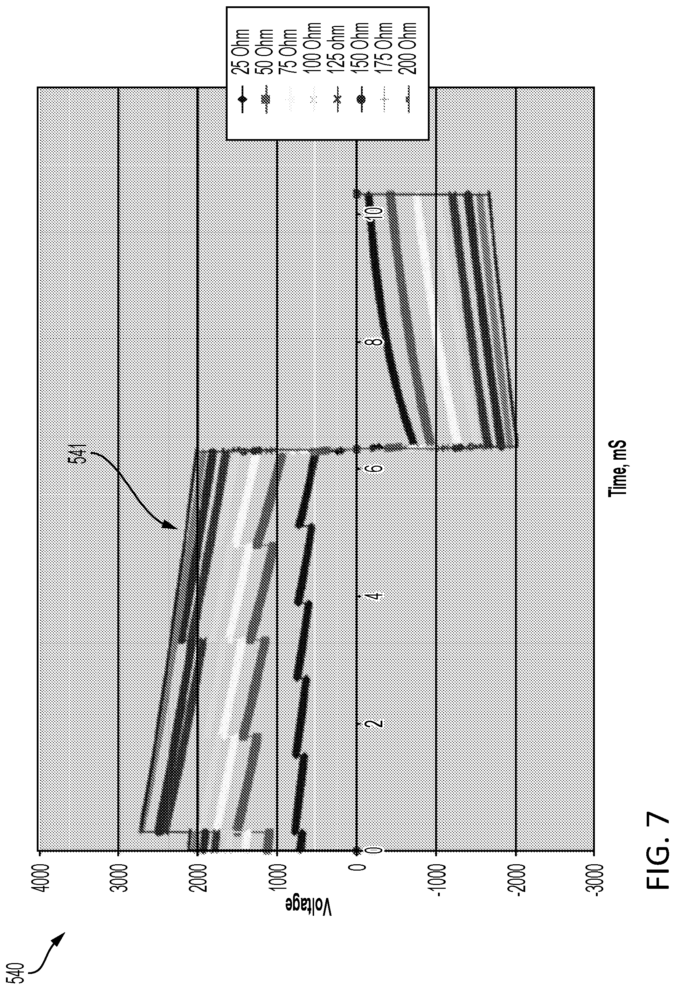

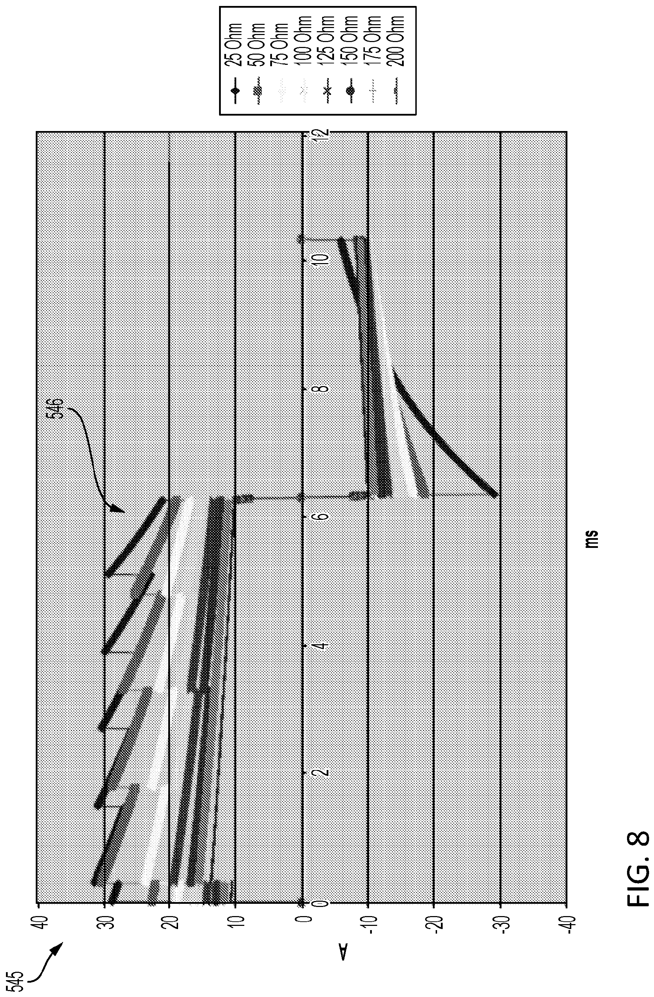

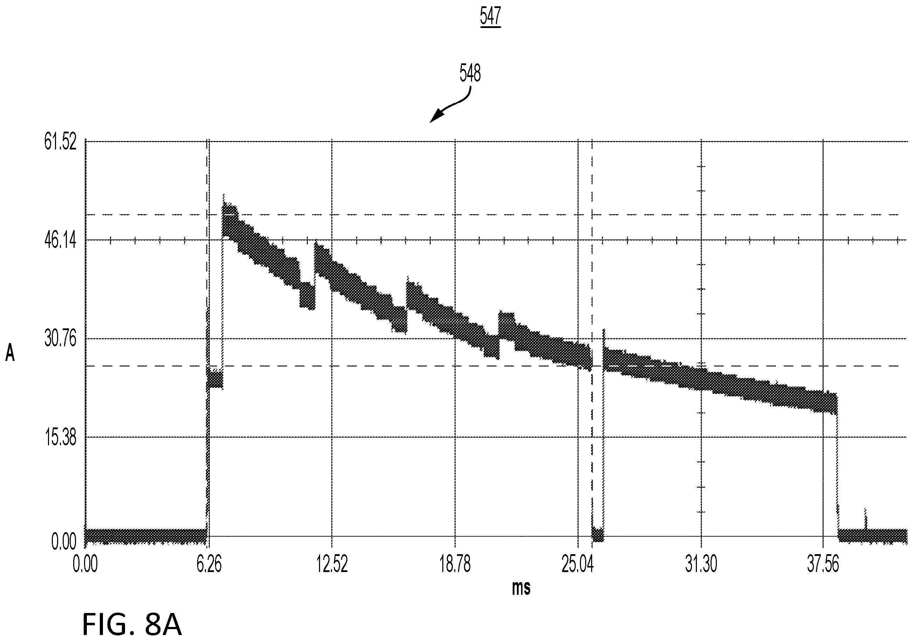

FIGs. 7 and 8 are graphical illustrations 540, 545 of example simulation results relating to use of a therapeutic current control network with parallel resonance, showing biphasic rectilinear waveforms, including voltage waveforms 541 and current waveforms 546, respectively, over a range of patient load impedance values of 25 to 200 ohms, for an energy delivered to the patient of 200 joules. FIG. 8A is a graphical illustration 547 of example simulation results 548 relating to use of a therapeutic current control network with parallel resonance, showing a single phase of a current waveform with a patient impedance load of 50 ohms and a delivered energy of 200 joules, generated using a function generator, and using an oscilloscope with a current probe to measure current of the waveform across the patient load. As evidenced in the illustrations 540, 545 and 547, systems with parallel resonance, according to various embodiments, such as those depicted in FIGs. 4A-B, 4C and 5A-B, can be used to generate and deliver to a patient, for example, biphasic rectilinear voltage and current waveforms for a wide range of patient impedances.

FIG. 9A is a block diagram of an illustrative example of a therapeutic current control system 900. The system 900 includes a power supply 902, an electrical parameter sensor 909 connected with a patient 908, a controller 911, a specified current waveform 910 input to the controller 911, and may use closed loop current control 912 (or, in other embodiments, may use non-closed loop current control). These, among other components, may be similar or analogous to components depicted in other figures and embodiments. However, various details of various components may differ, and the arrangement or placement, such as within a circuit, of various components may also differ, such as the components, and arrangement of components, of the therapeutic current control network 901. Although, in the embodiment depicted, the electrical parameter sensor(s) 909, closed loop current control 912 and signals 913 indicative of current output to the patient 908 are included, in other embodiments, some or all of these components may not be included or used, and, in some embodiments, the system 900 may operate using non-closed loop current control, for example. The therapeutic current control network 901 includes a filter/energy storage inductor 903, a resonant tank 904, one or more current control switches 905 (which may be or include high voltage MOSFET switches), a rectifier 906 and a filter capacitor 907.

FIG. 9B is an exemplary schematic diagram of a quasi-resonant therapeutic current control system 920, which may represent an embodiment of the system 900 as depicted in FIG. 9A. As depicted, the system 920 is implemented as a circuit 927 that includes an energy storage capacitor (Cap) 921, a filter/energy storage inductor (Lf) 922, a rectifier 923 implemented as a diode, a filter capacitor (Cf) 925, a current control switch (S) 926 (which may be or include a high voltage MOSFET switch, which may be SiC based, for example), and a resonant tank 923 including a resonant capacitor Cr and a resonant inductor Lr. Energy provided by the energy storage capacitor 921 flows through the resonant tank 405. A patent load 924 is also shown.

Some embodiments of quasi-resonant systems as described herein, including the embodiment of FIGs. 9A and 9B, for example, can provide various advantages over other systems. These advantages may include, among others, more efficient use of an filter/energy storage inductor due to a higher voltage applied to it by the resonant tank. The advantages may also include reduction in switching losses and increased energy efficiency, such as by use of zero voltage switching (ZVS) at switch turn off. The advantages may further include the ability to use a lower voltage (and potentially less expensive, smaller and lighter) energy storage capacitor, such as for particular applications and requirements, such as may include particular defibrillation applications and requirements, and lower energy storage requirements. Furthermore, use of a lower voltage capacitor can lead to other advantages such as smaller required electrical isolation barriers within the circuit and greater flexibility and options with regard to switching devices, for example. In some embodiments, as described further herein, a resonant tank may be used, or may sometimes be used, to boost a voltage of an electrotherapeutic waveform delivered to a load, such as a patient load.

In some embodiments, the circuit 927 of FIG. 9B may have certain component and configuration aspects that are similar to certain component and configuration aspects of a non-resonant, or conventional, boost converter circuit. However, potentially among other things, the circuit 927 of FIG. 9B is quasi-resonant and includes a resonant tank 923.

While various nodal configurations may be used, in some embodiments, including in the configuration of the circuit 920 of FIG. 9B, the following nodal connections are used. The energy storage capacitor 921 and a first portion of the circuit 927 including the filter/energy storage inductor 922 are connected via node Nc1. The first portion of the network 927 and a second portion of the network 927 including the resonant tank 923 are connected via node Nc2. The second portion of the network 927 and a third portion of the network 927 including the filter capacitor 925 and the patient/patient load 924 are connected via node Nc3.

Quasi-resonant circuits (and associated or corresponding networks and systems) according to some embodiments, such as circuit 927, may provide advantages relative to other circuits, including, for example, conventional boost converter circuits. These may include, for example, among other things, more efficient use of the filter/energy storage inductor 922, as well as incorporating, for example, zero voltage switching at switch turn off, which reduces switching losses and increases system efficiency. The resonant tank 923 boosts a voltage delivered to the patient/patient load 924, such as, for example, the voltage of an electrotherapeutic pulse delivered to the patient 924. Availability of high voltage MOSFETs, including SiC based MOSFET switches allow use of higher switching speeds and a smaller filter/energy storage inductor, for example. Further advantages may include use of a lower voltage capacitor, such for a particular patient load output requirement, and associated advantages, such as, for example, smaller isolation barriers.

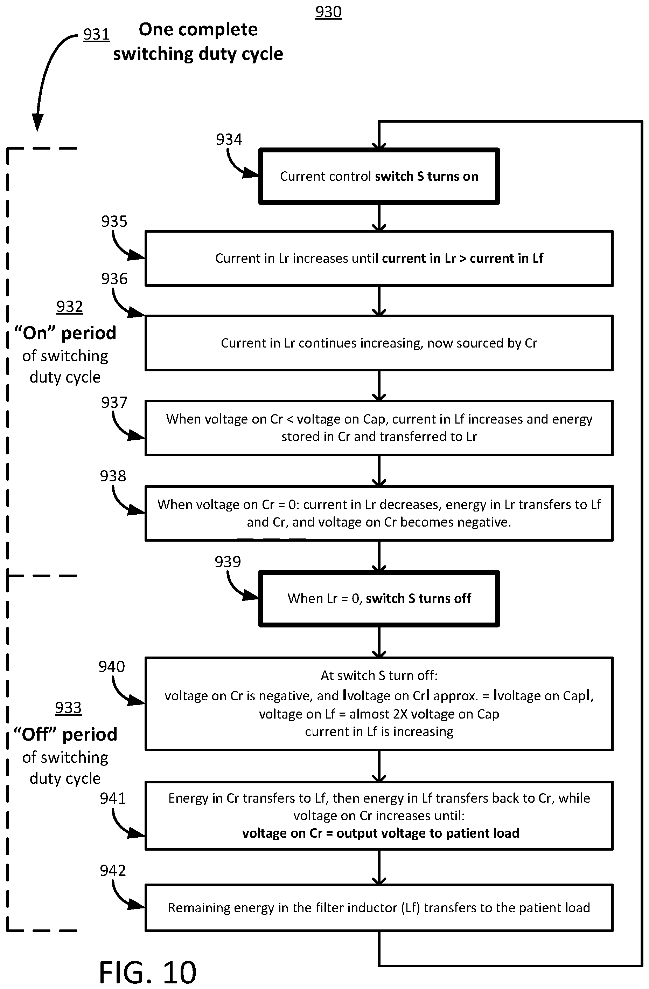

FIG. 10 is a flow diagram of a method 930 implemented using an example quasi-resonant therapeutic current control system. The method 930 can be implemented, for example, by embodiments of the systems 900, 920 of FIGs. 9A and 9B. More particularly, the method 930 represents operation of the system during one full switching duty cycle 931 of the one or more current control switches of the system, such as the current control switch(es) 905 of the system 900 of FIG. 9A or the current control switch (S) 926 of the system 920 of FIG. 9B, for example. As depicted in FIG. 10, the method 930 is described with regard to the system 900 of FIG. 9B. The switching duty cycle 931 includes an "on" period, during which the current control switch (or switches) is turned on (in a closed configuration so that current can pass), and an "off" period during which the current control switch is turned off (in an open configuration so that current cannot pass), then the cycle continuously repeats, for example, during she short but critical period of delivery of an electrotherapeutic waveform to a patient/patient load.

At step 934, the current control switch (S) turns on. This begins the "on" period of the switching duty cycle 931, which is represented by steps 934-938. Following switch turn on, at step 935, the current in the resonant inductor (Lr) begins increasing until the current in the resonant inductor (Lr) exceeds the current in the filter inductor (Lf). Next, at step 936, once the current in the resonant inductor (Lr) exceeds the current in the filter inductor (Lf), the current in the resonant inductor (Lr) continues to increase, now being sourced by energy stored in the resonant capacitor (Cr). Next, at step 937, once the voltage on the resonant capacitor (Cr) decreases to below the voltage on the energy storage capacitor (Cap), the current in the filter inductor (Lf) increases, and energy stored in the resonant capacitor (Cr) is transferred to the resonant inductor (Lr). Next, at step 938, Once voltage on the resonant capacitor (Cr) decreases to zero, current in the resonant inductor (Lr) begins to decrease, energy stored in the resonant inductor (Lr) transfers to the filter inductor (Lf) and to the resonant capacitor (Cr), and voltage on the resonant capacitor (Cr) becomes negative (polarity reverses).

Next, at step 939, the current control switch (S) turns off. This begins the "off" period of the switching duty cycle 931, which is represented by steps 939-942. As depicted at step 940, at switch turn off, the voltage on the resonant capacitor (Cr) has become negative and approximately equal in magnitude to the voltage on the energy storage capacitor (Cap), the voltage on the filter inductor (Lf) is almost twice the voltage on the energy storage capacitor (Cap), and the current in filter inductor (Lf) is increasing. Next, at step 941, energy in the resonant capacitor (Cr) transfers to the filter inductor (Lf), then energy in the filter inductor (Lf) transfers back to the resonant capacitor (Cr), while the voltage on the resonant capacitor (Cr) increases to the output voltage to the patient load. Next, at step 942, remaining energy in the filter inductor (Lf) transfers to the patient load. This completes the "off" period of the switching cycle 931. Following step 942, the method 930 returns to step 934, at which the next switching duty cycle begins with current control switch "on" at step 934.

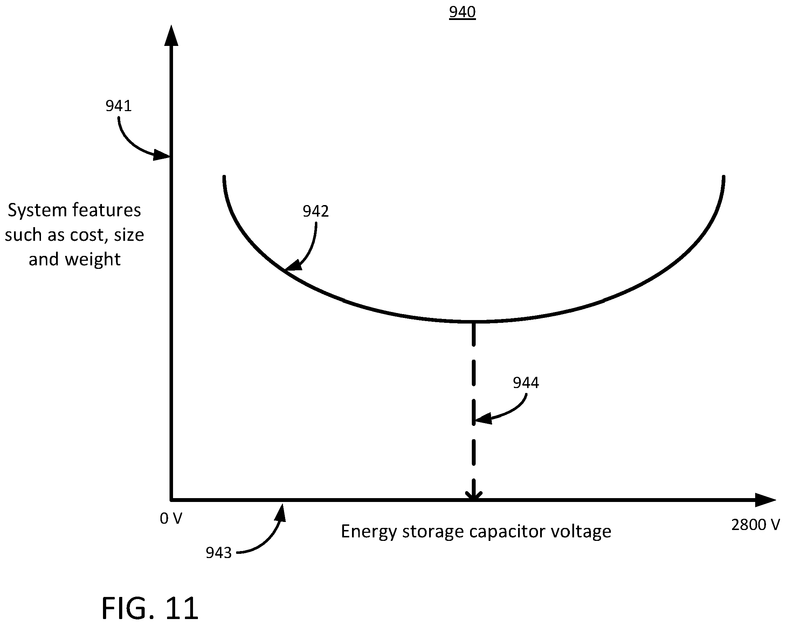

FIG. 11 is a graphical illustration 940 showing potential balancing or trade-off, in some embodiments, between various system features, such as, for example, overall system cost, size and weight (potentially among other factors), as represented, conceptually and qualitatively, by axis 941, and one or more system component features, such as, for example, energy storage capacitor voltage, or energy storage capacitor voltage rating (potentially among other factors), as represented by axis 943. The portion of axis 943 shown in FIG. 11 runs between 0 V and 2800V, but energy storage capacitor voltages may run higher than 2800V.

In some embodiments, the voltage of the energy storage capacitor drives or limits defibrillation shock energy. As such, in some embodiments, the voltage on the energy storage capacitor, and the voltage rating of the energy storage capacitor, must be sufficient to produce the needed defibrillation energy for the system and its anticipated applications. However, generally, higher voltage capacitors may be considerably more expensive, larger and heavier than lower voltage capacitors. Furthermore, the cost, size and weight of the energy storage capacitor may be a considerable portion or fraction of the overall system cost, size and weight. As such, there may be a tension between maximizing energy storage capacitor voltage/voltage rating, on the one hand, and minimizing system features such as overall system cost, size and weight, on the other hand.

Given the foregoing, a balance or optimization may need to be drawn between the required minimum energy storage capacitor voltage/voltage rating, on the one hand, and system requirements such as maximum cost, maximum size and maximum weight (and various combinations thereof and balances there between), on the other hand. This balance or optimization may lead to a selected energy storage capacitor voltage, or voltage rating, that is high enough to be sufficient for the anticipated applications of the system and yet low enough to allow the system to meet requirements that may relate, for example, to maximum cost, size and/or weight. Such a balanced or optimized selection of energy storage capacitor voltage/voltage rating is conceptually represented by broken line 944. Furthermore, in some embodiments, various system aspects and components are selected in view of such optimization considerations. These aspects and components may include, for example, selection of current control switch switching speed, and particular resonant tank and filter inductors and capacitors.

It is noted that various embodiments of systems described herein may provide substantial advantages in terms of operational optimization and efficiency than various other systems. Furthermore, embodiments of systems described herein may allow use of a lower voltage capacitor than other systems, for a given set of requirements or applications. As such, embodiments described herein may allow for lower cost, smaller size, and lower weight than other systems, such as for particular required electrotherapy or defibrillation shock energies to be delivered to patients, for example. As such, embodiments of systems described herein may provide for, or provide for a higher degree of, practicality, portability, transportability, speed of set-up, or hand-held use, for example relative to other systems. This, in turn, can provide great practical advantages including in, for example, non-hospital or field use advantages, among other settings and applications.

Various types and embodiments of controllers may be used in various embodiments described herein. For example, a controller may be digital or analog, including fully analog, and various embodiments may or may not use components such as a CPU, a processor or a microprocessor. It is noted that, in some embodiments, including some embodiments using non-closed loop control, a controller may not need to, for example, process a signal associated with, for example, at least one sensed electrical parameter, or compare such a processed signal with a second signal, for example, associated with a specified waveform.

In some embodiments, a controller may effectively or actually implement or execute a control algorithm or control loop. For example, a controller may be or include a proportional-integral-derivative (PID) controller, a proportional-integral (PI) controller, or be or include an error amplifier (implemented as digital or analog), or another form of controller, digital or analog. Generally, a controller, based on input including signals corresponding with the sensed current and voltage and the specified current waveform, outputs one or more signals to control operation of at least a portion of at least one current control switch of a therapeutic current control network, as well as potentially other components, such as a rectifier in the case of synchronous rectification, or active rectification, where active switch components rather than passive diodes are used, for example. In some embodiments, the controller processes the signals at least in the sense of receiving them and utilizing them as input.

In some embodiments, a controller processes a signal relating to at least one sensed electrical parameter indicative of current flow to a patient, such as a current parameter and/or a voltage parameter. The controller may then compare the signal, and/or an obtained parameter associated with the signal, with a second signal or parameter associated with a specified waveform (or may generate, utilize or compare parameters associated with signals). Based at least in part on the comparison, the controller may control, at least, operation of at least a portion of at least one current control switch of a therapeutic current control network to adjust control of an electrotherapeutic waveform being delivered to the patient to correspond with the specified waveform.

It is noted that various embodiments of controllers are contemplated, which may be configured differently and operate in different ways. For example, in some embodiments, data, parameters or signaling providing a specification of a waveform may be input to a memory of the controller or elsewhere and provided to the controller, such as prior to delivery of the electrotherapeutic waveform. As another example, in other embodiments, a specification of a waveform may effectively be input or fed over time, such as substantially in real time during delivery of the waveform. Furthermore, data, parameters or signaling associated with a current provided to a patient may also be provided or facilitated in different ways. For example, in some embodiments, a controller may receive a varying signal corresponding with sensed current being provided to the patient. However, in other embodiments, sensed current may not be utilized, and, instead, one or more other varying signals may be utilized in specifying, implicitly or explicitly, a waveform being delivered to a patient, for instance, measuring the sensed voltage across the output circuit nodes to the patient.

Continuously or repeatedly, for example, the controller may effectively compare the specified waveform with the waveform being delivered. Continuously or repeatedly, based at least in part on the comparison, the controller adjusts operation of, at least, one or more switches of a therapeutic current control network as and when needed to adjust the waveform being delivered to the patient to match the specified waveform.

In particular, for example, in some embodiments, the controller may utilize a control loop or otherwise operate to create, calculate or utilize a signal or parameter indicative of a quantitative difference between a current provided to a patient and a desired current to be provided to the patient, such as may be associated with a specified waveform. The controller may then operate to minimize this difference, thus minimizing, over time of delivery, any difference between a current provided to the patient and a current desired to be provided to the patient, such as by control, at least, of one or more current control switches of the system. More particularly, in some embodiments, the controller may create, calculate or obtain (or repeatedly or continuously create, calculate or obtain) an e(t) (error signal as a function of time), or e(t) based, signal or parameter, where:

$$\text{(Equation 3):} \quad e(t) = C_M - C_R$$

Where, in equation 3:

$C_M$ is measured (or determined or estimated) current to the patient (or an electrical parameter indicative of, or associated with, current to the patient); and
$C_R$ is current associated with a specified current or waveform.

E(t) may therefore quantitatively indicate a (or any) difference, for example, at a given time/time period or for a particular individual comparison, between a measured (or determined or estimated) level of current to a patient and a level of current associated with a specified current or waveform. This may include indicating whether a level of current to the patient is higher or lower (such as may be given by the sign of e(t) as positive or negative, for example) and the amount by which it is higher or lower (such as may be given by the magnitude or absolute value of e(t), for example). The controller may then operate to minimize e(t) to appropriately affect a current level provided to the patient, which results in the current level provided to the patient matching the specified current or waveform as closely as possible.

The controller may receive input including a signal relating to a specified waveform, such as a signal corresponding with current level, and one or more other signals, which may include one or more signals corresponding with current level relating to the actual electrotherapeutic waveform being delivered to the patient.

In some embodiments, signal output from a PID portion of a controller is used in generation of a phase shift PWM signal that is used in assuring that a carrier signal is kept within certain limits. In some embodiments, however, a frequency changing signal could be utilized. An output signal from the controller controls operation of one or more current control switches to make any necessary adjustments, such as increasing or decreasing current, to cause the waveform being delivered to the patient to match the specified waveform.

In some embodiments, clock related components of a controller, including a dead time element, are used in controlling timing relating to switch operation. The clock related components and switch turn-on and turn-off timing may be used in implementing soft-switching, substantially zero voltage switching (ZVS) or substantially zero current switching (ZCS).

It is also noted that one or more switches that are operatively controlled by the controller may include switch components including wide bandgap, such as silicon carbide (SiC) or gallium nitride (GaN), further reducing any switching losses and increasing overall system efficiency.

In some embodiments, a controller may use a servo loop control including an operational amplifier (op amp), though in other embodiments, implementations and applications, another form or forms of control may be used an may be more optimal and/or flexible, such as, for example, an FPGA based controller or a DSP microcontroller. Furthermore, some embodiments may use an application-specific integrated circuit (ASIC) based controller. In some embodiments, a servo loop control op amp may be used in controlling a voltage-controlled oscillator (VCO). Output from the servo loop control may be input to the VCO, and the VCO may generate fixed duration pulses used in control of the switching frequency of one or more current control MOSFET switches, which may be used is used in adjusting current level through the patient, for example. In some embodiments, high speed comparators may be used, such as in a VCO.

In various embodiments, the reference waveform generator may generate a reference waveform using various techniques and components, such as may include use of a digital to analog converter (DAC), for example.

In some embodiments, components such as Zener diodes may be used in protecting circuits against an overvoltage condition. Furthermore, in some embodiments, some components may be customized for high voltage operation, or to perform better or be more reliable under high voltage conditions. For example, in some embodiments, some components, such as a filter inductor and a resonant tank inductor, are be required to withstand high voltage and each may require a substantial layer of insulation on the conductors. This may, in some embodiments, include customization of these components, such as to add such insulation accordingly.

In some embodiments, circuits include, for example, soft switching including a zero voltage switching (ZVS) converter that may be set at a high switching speed, which may be selected considering optimization of features including size and weight, such as, for example, 1 MHz (or, e.g., 0.7-1.3MHz), which may, given the high frequency and voltage, make use of high bandgap materials, such as silicon carbide (SiC) or Gallium nitride (GaN) for components associated with components such as, for example, switches, diodes and others. Furthermore, a zero current detector may be used in detecting, detecting more accurately or precisely, or confirming a zero current condition in order to more facilitate more accurately or precisely accomplishing soft switching such as ZVS.

In some embodiments, one or more current control high voltage MOSFET switches may be or include, for example, one or more Silicon (Si) MOSFETs or Silicon Carbide (SiC) MOSFETs, such as, for example, a 3.3kV MOSFET driven in a cascode configuration.

In some embodiments, switches of a polarity control may be implemented, for example, as a monolithic IGBT bridge, or in other ways, although as depicted includes full bridge (e.g., H-bridge) using four MOSFETs and four bipolar PNP transistors to switch between positive and negative phases of the resolution bandwidth filter (RBF). The associated gate drive could be or include, for example, independent voltage sources or isolated gate drivers, such as used for motor control. In some embodiments, various components such as diodes may be implemented using wide bandgap materials, such as silicon carbide (SiC) or Gallium nitride (GaN), including, for example, one or more 3.3kV rated SiC or GaN diodes.

In some embodiments, a specified waveform generator may be used that may include, for example, a voltage source programmed to generate a specified waveform for a servo loop control that controls the mean source current in one or more current control MOSFET switches.

In some embodiments, a current sense resistor may be used in obtaining a signal or signals that can be used by the controller (potentially among other information or measured parameters) in determining any necessary adjustment to one or more current control switches to cause the electrotherapeutic waveform being delivered to the patient to correspond, or to more accurately or precisely correspond, to a specified waveform. The current sense resister may be a component of a current sensing portion of the circuit that outputs the average current being drawn from the power supply. Switching frequency of one or more current control MOSFET switches may be adjusted in order to adjust the waveform being delivered to the patient, such as by causing an incremental increase or decrease in the amount of current delivered to the patient relative to amount that would be delivered with no switching frequency adjustment.

In embodiments in which current to the patient is not directly measured, operation of the controller, in determining a necessary adjustment of the switching frequency of one or more current control MOSFET switches, may need to account for the difference between the measured current and the current to the patient. This may be necessary since it is ultimately the current through the patient that must be determined or estimated and adjusted as necessary. There are a number of different ways in which this can be accomplished.

In some embodiments, current to the patient / patient load is determined or estimated based at least in part on the measured current, potentially also using other data, such as measured electrical parameters within a circuit, data about circuit configuration or components, data about actual or anticipated conditions present during delivery of the electrotherapeutic waveform to the patient, or other information. For example, in some embodiments, current to the patient may be determined or estimated based on measured current through one or more current control MOSFET switches in combination with other measured or estimated parameters such as one or more capacitor voltages, one or more measured or estimated switching losses, or other data. In some embodiments, one or more algorithms may be used in this regard. As such, a correspondence or mapping may be made between the combination of parameters including the measured current and the determined or estimated current through the patient load. This mapping may be utilized by the controller in determining necessary adjustments to the one or more current control MOSFET switches such that the current waveform being delivered to the patient matches the specified waveform.

In various embodiments, this mapping may be done prior to delivery of the electrotherapeutic waveform to the patient, potentially by using one or more algorithms, and for a range of parameters and current levels, or may be done by the controller during delivery, such as in real time, potentially using one or more algorithms stored in a memory of the controller. Furthermore, the mapping may be done analytically, such as by using circuit analysis, or may be done empirically, such as by actual testing using operation of the circuit, or may done by some combination of the two. In some embodiments, mapping data may be stored in the memory of the controller, or may be input to the controller.

In various embodiments, the controller may operate in various ways in incorporating the mapping. For example, the controller may compare a signal representative of the measured current with a signal representative of the specified waveform current, generating an error signal relating to the difference between the measured current and the specified waveform current. However, in some embodiments, the controller may determine the necessary adjustment based on minimizing an error function e(t), which represents, over time, the difference between the current to the patient and the reference waveform current. As such, in some embodiments, the mapping may be used to determine a derived error signal representing the difference between the determined or estimated current to the patient and reference waveform current, and the error function e(t) may then be used. Of course, there are many other ways in which the controller could operate. In some embodiments, however, these may have in common that the current to the patient must be determined or estimated using one or more parameters including the measured current (not to the patient), and the adjustment to the one or more current control MOSFET switches must be based on adjusting the current waveform being delivered to the patient to correspond to the specified waveform.

In some embodiments, typically commercially available electrical components may not be designed, or ideally designed, to handle the high voltages and associated stresses associated with circuits according to embodiments of the disclosure, such as in defibrillation applications, for example. As such, in some embodiments, adjustment, modification, or additions are made to some components, or multiple components may be used, for example, in series to achieve, or better achieve, an additive effect using available components. For example, some commercially available components, such as inductors, may not be rated for or capable of withstanding very high voltages, such as, for example, 2.8 kV, 5.6kV, or more, which may not be sufficient or ideal for some applications according to the present disclosure. In some embodiments, for example, measures are taken to improve such specifications or tolerances, such as by applying some form of encapsulation or conformal coat immersion to commercially available components, which can effectively lead to creation of custom components better suited to the very high voltage applications. Extensive experimentation and testing may be performed to ensure the effectiveness, reliability and safety of such customizations. As such, in some embodiments, custom components with sufficiently high voltage ratings may be created and used, which may be created by applying measures such as those decribed, or other measures, to otherwise commercially available or standard components or parts, for example.

As noted above, some embodiments utilize wide bandgap materials in circuit components, such as may include switch components. Generally, a wide bandgap material can include a semiconductor material with a relatively large band gap compared to conventional semiconductors, where a band gap is essentially an energy range in a solid in which no electronic states exist. Conventional semiconductor materials typically have a band gap in the range of 1 - 1.5 ev, whereas wide bandgap materials may have bandgaps at or above 2 ev, such as between 2 - 4 ev or between 2 - 6 ev. Generally, use of wide bandgap materials can permit electronic devices and components to operate at much higher voltages, frequencies and temperatures, as well as higher power conversion efficiencies, which allows for enhanced overall component or device performance or efficiency, as well as permits use of higher power density devices. Wide bandgap, such as may include ultra-wide bandgap, materials that may be used in various embodiments can include materials such as SiC and GaN, as well as other materials such as diamond, gallium oxide ($Ga_2O_3$), aluminum gallium nitride (AlGaN) or aluminum nitride (AlN). For example, some embodiments use the wide bandgap material SiC, which has useful properties including high thermal conductivity, high electric field breakdown strength and high maximum current density.

FIGS. 12A - 12V are schematic diagrams of example resonant tank configurations that can be used in embodiments of a therapeutic current control network or as a bidirectional charging control network or an element thereof. Resonant tanks may also be used elsewhere in a closed loop or non-closed loop therapeutic current control system according to embodiments described herein.

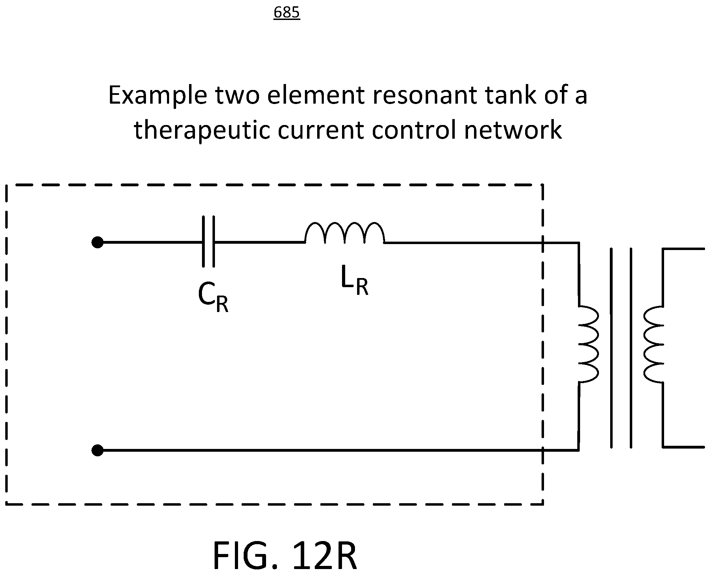

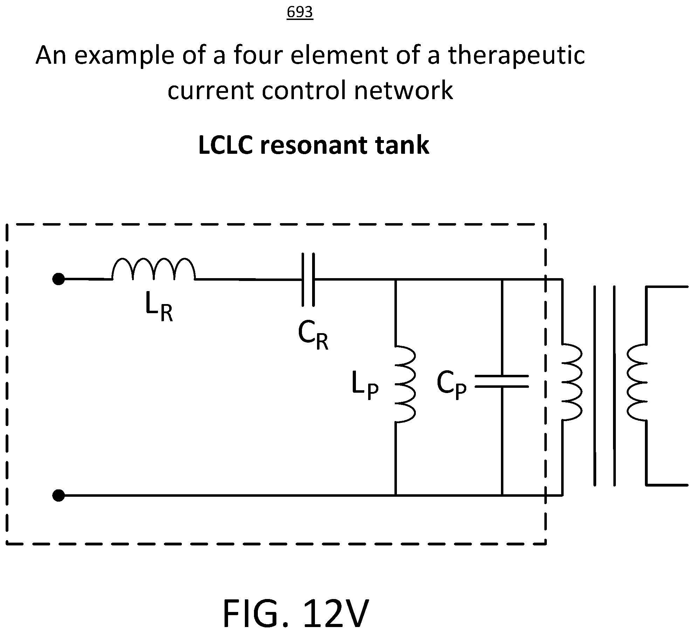

More particularly, FIGS. 12A - 12Q depict examples of three element resonant tank configurations, FIGS. 12R - 12U depict examples of two element resonant tank configurations, and FIG. 12V depicts an example of a four element resonant tank configuration, various of which can be used in various embodiments described herein. Each element is either an inductor (L) or a capacitor (C). In FIGS. 12A - 12V, components connected in series in the resonant tank are designated with the subscript "R" while components in parallel in the resonant tank are designated with the subscript "P". In general, in an electrical circuit, a resonant tank includes a configuration of one or more inductors and one or more capacitors, each of which is coupled either in series or in parallel in the resonant tank. The resonant tanks shown in FIGS. 12A-12V are not intended to be limiting, and other types of resonant tanks, or other resonant electrical circuits, may be used in various embodiments of closed therapeutic current control systems as described herein.

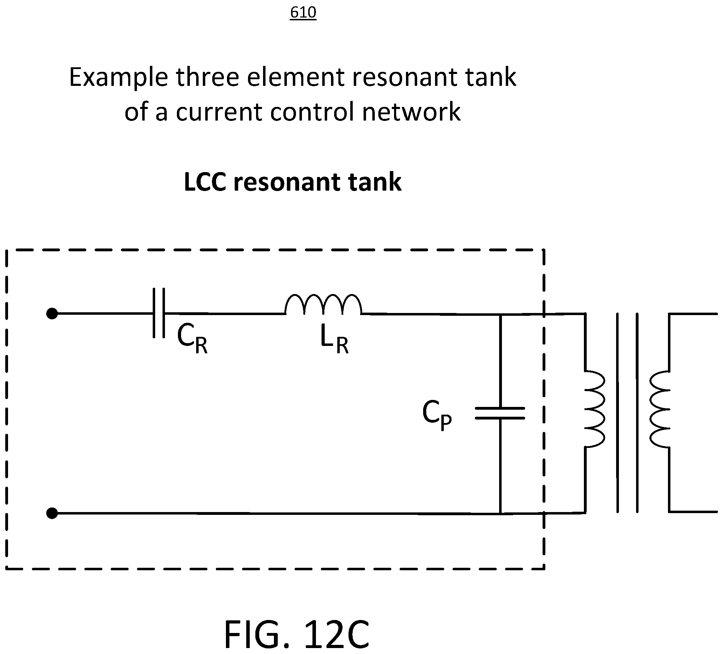

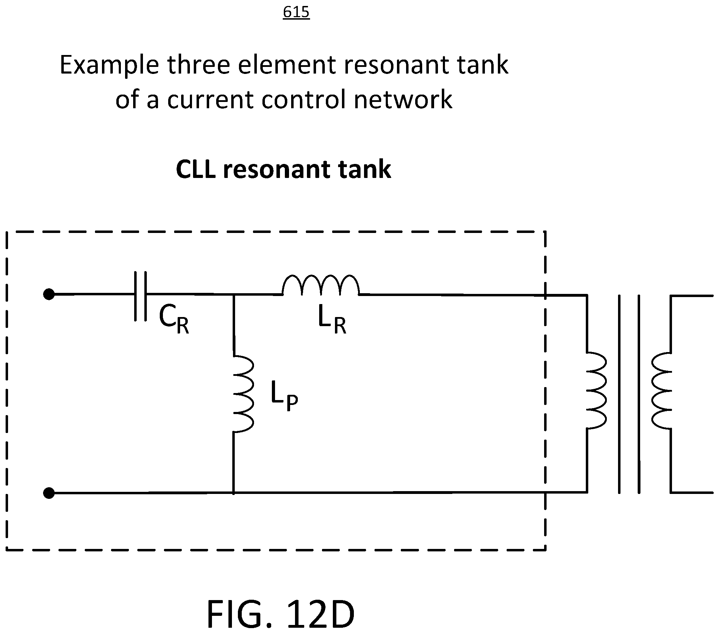

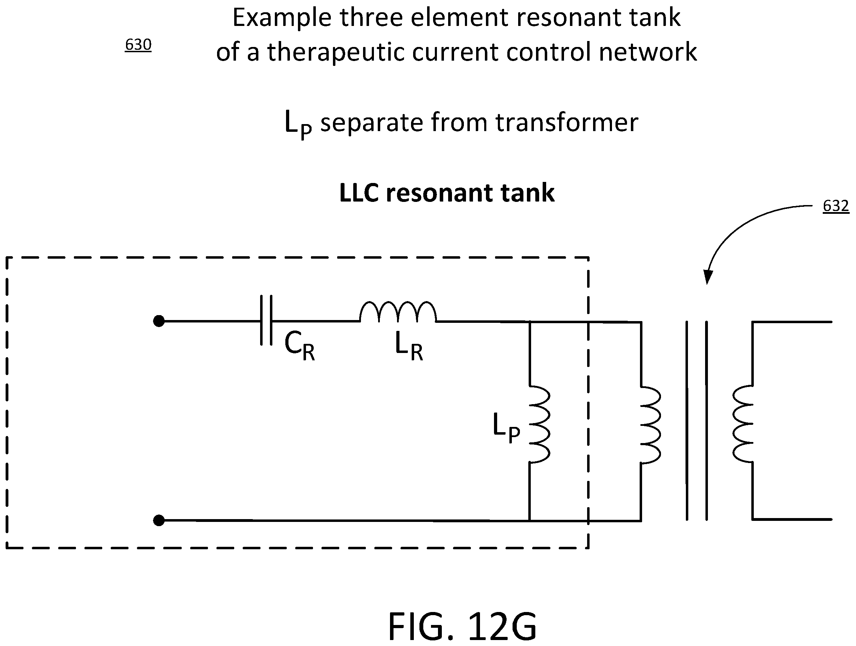

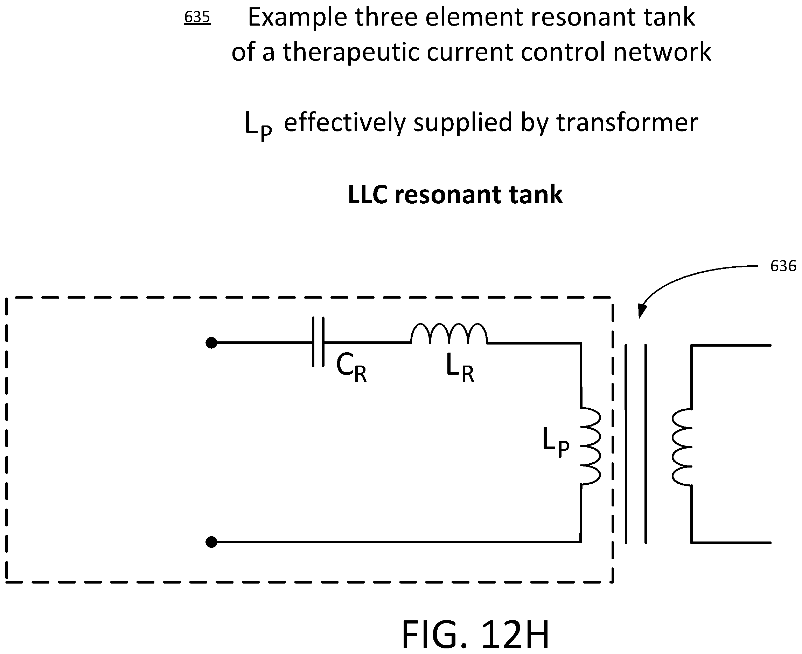

In various embodiments described herein, a variety of resonant tanks may be utilized. These include two-element, three-element and four-element resonant tanks. In various embodiments described herein, for some uses or ranges of uses, a resonant tank such as an LCC, CLL, LLC or LCLC resonant tank, for example, may be optimal or may be viewed as optimal. For example, FIG. 12C depicts an example LCC resonant tank implementation, FIG. 12D depicts an example CLL resonant tank implementation, FIGS. 12G and 12H depict example LLC resonant tank implementations, and FIG. 12V depicts an example LCLC resonant tank implementation. A particular resonant tank that is preferable or provides desired performance or efficiency in a therapeutic current control network according to an embodiment described herein may depend on a variety of factors. These factors may include, for example, physical, component related, functional or use related aspects or parameters, which may include particular practical, performance, or efficiency related requirements, priorities or preferences, for example. In some cases, an LCC resonant tank may be preferable, such as may include some applications involving a wide input or output voltage range.

In some cases, a CLL resonant tank may be preferable, such as may include some cases in which the resonant tank switching frequency is higher than the resonant frequency or frequencies.

In some cases, an LLC resonant tank may be preferable, such as may include some cases in which the LLC resonant tank may provide advantages including high efficiency, high power density, and relatively easy implementation of magnetic integration, among other potential advantages. Furthermore, in some embodiments, an LLC resonant tank based therapeutic current control network may have features or characteristics including the following. It may have a magnetics ratio, for example, in the range of between three to ten. A parallel to series inductance ratio ("K") may be selectable in such a way to have minimum circulating current while yet maintaining soft-switching. It may have an associated selectable quality factor, which may typically be between 0.1 and 1, that may directly affect the size of magnetics along with the selected resonant frequency, frequencies or frequency range. The quality factor may affect aspects such as Root-Mean-Squared (RMS) current, turn off current, range of relative frequency range, DC gain for regulation, as well as the selected K. In some embodiments, ZVS may be a function of K and quality factor. In some embodiments, selected K and quality factor may involve a balance or trade-off in certain ways. For example, a K or quality factor increase may be associated with lower primary RMS value, lower ZVS angle range, lower turn-off current, lower DC gain, higher size of magnetics and higher stress on an energy storage capacitor.

In some cases, an LCLC resonant tank may be preferable, such as may include some cases in which the LCLC resonant tank provides optimal performance by effectively homogenizing characteristics of an LLC resonant tank and an LCC resonant tank. Other resonant tanks, or other resonant electrical circuits, may be preferable in other cases.

A resonant tank may act as an electrical resonator, storing energy oscillating at the resonant tank's resonant frequency, frequencies or frequency range. In some embodiments described herein, a therapeutic current control network may include, connected in the following series: (1) an energy storage capacitor, (2) at least one current control switch, (3) a resonant tank, (4) a rectifier and (5) a filter. The topology created by elements (2)-(5) in the preceding list is commonly referred to as a resonant converter.

Parasitic capacitance and parasitic inductance refer to usually unwanted but unavoidable capacitance or inductance that exists between elements of an electronic circuit. According to some embodiments of the present disclosure, however, the parasitic inductance of a transformer positioned in the vicinity of a resonant tank may be intentionally utilized to provide capacitance or inductance for, and of, the resonant tank or other resonant electrical circuit. In such instances, the inductance provided by the transformer may effectively be part of the resonant tank, replacing the role of a resonant tank inductor.

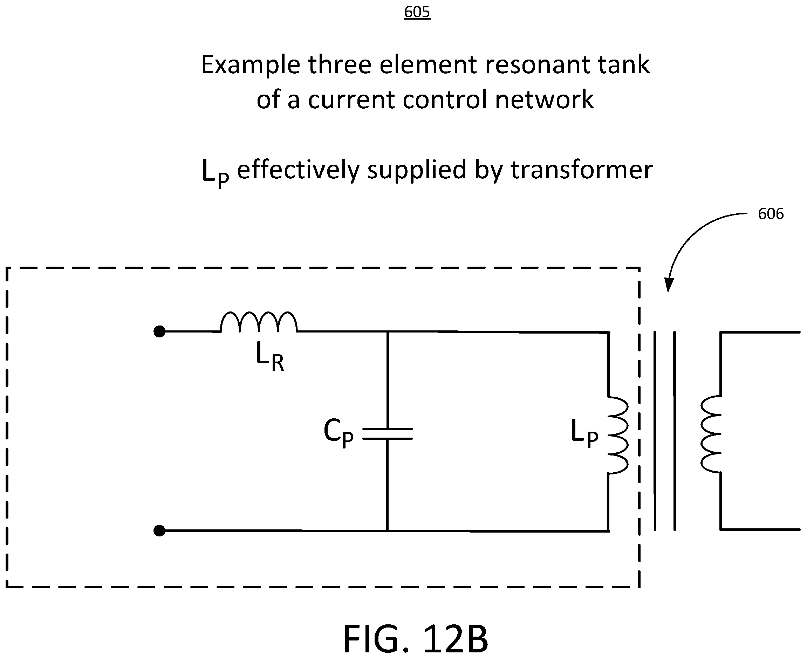

For example, FIG. 12A and 12B depict two embodiments 600, 605 of a three element resonant tank, which includes one inductor in series in the resonant tank ($L_R$) one inductor in parallel in the resonant tank ($L_P$) and one capacitor in parallel in the resonant tank ($C_P$). In the embodiment 600 depicted in FIG. 12A, the $L_P$ is separate from the depicted transformer 601. In the embodiment 605 depicted in FIG. 12B, however, the $L_P$ is effectively provided by the transformer 606. As another example, FIG. 12G and 12H depict two embodiments 630, 635 of an LLC resonant tank, which includes one inductor in series in the resonant tank ($L_R$) one inductor in parallel in the resonant tank ($L_P$) and one capacitor in series in the resonant tank ($C_R$). In the embodiment 630 depicted in FIG. 12G, the $L_P$ is separate from the depicted transformer 632. In the embodiment 635 depicted in FIG. 12H, however, the $L_P$ is effectively provided by the transformer 636. It is noted that, while not depicted in every resonant tank implementation shown in FIGS. 12A - 12V, in various embodiments, parasitic inductance can also be used to supply an $L_R$ in various resonant tanks or other resonant electrical circuits.

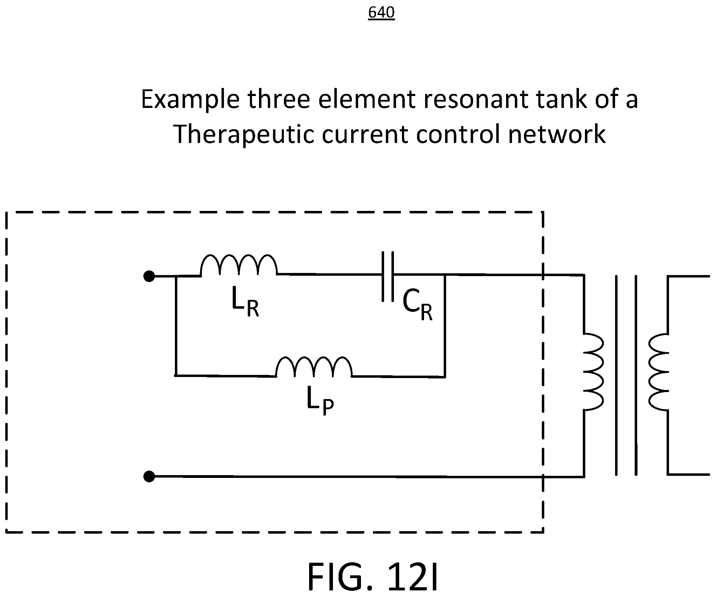

FIGS. 12C-12F, depicting embodiments, respectively, 610, 615, 620 and 625, and FIGS. 12I-12Q, depicting embodiments, respectively, 640, 645, 650, 655, 660, 665, 670, 675 and 680, provide additional examples of three element resonant tanks that may be used in some embodiments of a therapeutic current control network as described herein. FIGS. 12R - 12U, depicting embodiments 685, 690, 691 and 692, provide examples of two element resonant tanks that can be used in some embodiments of a therapeutic current control network as described herein. FIG. 12V depicts an embodiment 693 of a four element resonant tank that can be used in some embodiments of a therapeutic current control network as described herein.

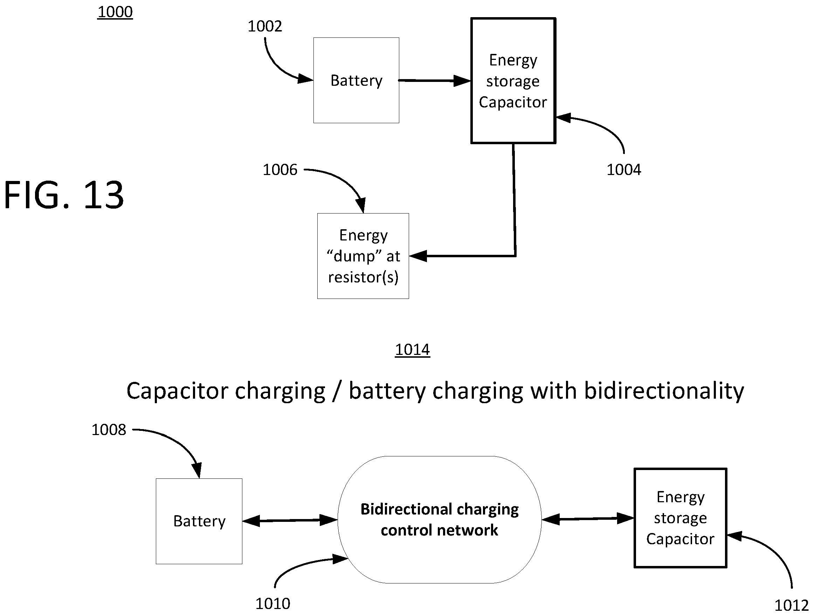

FIG. 13 depicts two flow diagrams illustrating different capacitor charging related methods 1000, 1014, such as may be used to charge an energy storage capacitor that may supply current for generation of an electrotherapeutic waveform to be delivered to a patient. In method 1000, a battery 1002 is used to charge an energy storage capacitor 1004. Excess or unused energy stored at the energy storage capacitor 1004 may be disposed of or dumped at one or more resistors 1006. Such excess energy can include, for example, excess energy following generation and delivery of an electrotherapeutic waveform to a patient. It can also include, for example, unused energy such as in a case in which the energy storage capacitor is charged for generation and delivery of an electrotherapeutic waveform to a patient but subsequently it is decided or determined not to go forward with the generation and delivery of the electrotherapeutic waveform to the patient. Such a decision or determination could be made, for instance, due to certain changed circumstances or changes in the patient's condition that would cause generation and delivery of the electrotherapeutic waveform to the patient to be currently not indicated, suboptimal, inadvisable or too risky. By contrast, in method 1014, a bidirectional charging control network 1010 facilitates not only charging of the energy storage capacitor 1012 by the battery 1008, not also enables return of excess or unused energy from the energy storage capacitor 1012 to the battery 1008 for storage at, and to charge, the battery 1008. The bidirectional charging control network 1010 may be implemented, for example, as the bidirectional charging control network 216 as depicted in FIG. 2C herein. Some embodiments of therapeutic current control systems utilize method 1014, increasing system efficiency, reducing heat production, and increasing battery life or the number of battery uses available before recharge of the battery 1008.

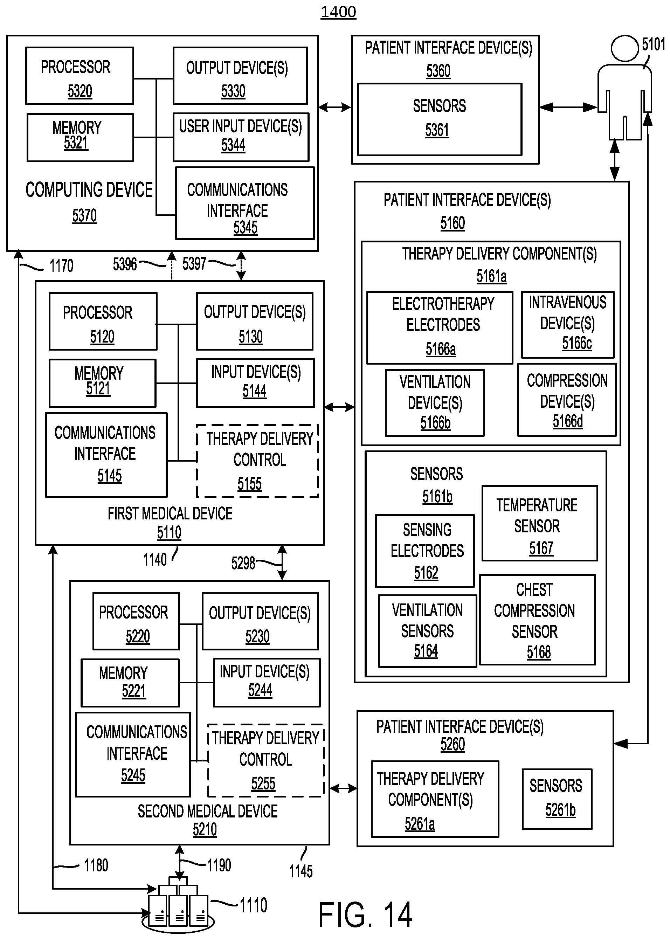

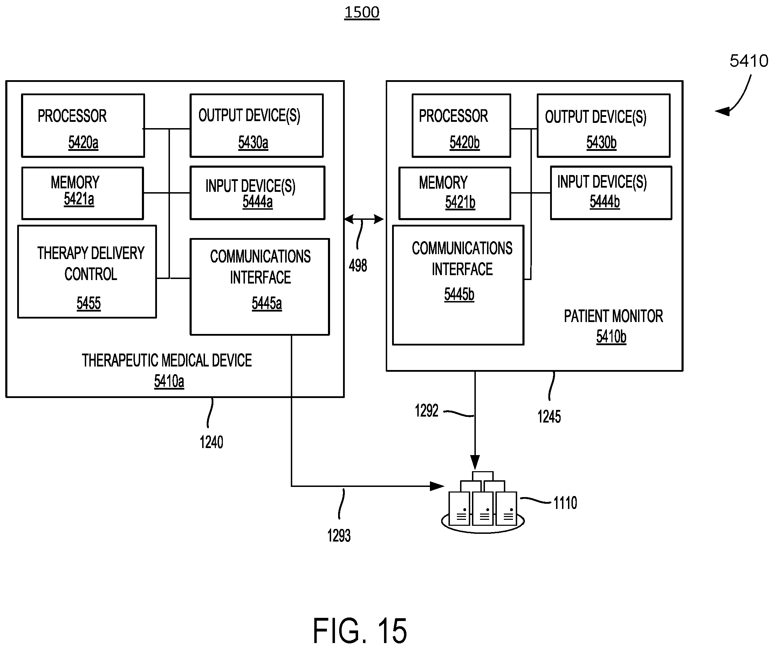

FIG. 14 is a schematic diagram illustrating components of a system 1400 of various devices including therapy delivery control elements that may incorporate aspects of embodiments of a therapeutic current control system. FIG. 15 is a schematic diagram illustrating components of a system, including a patient monitor and a therapeutic medical device including therapy deliver control elements that may incorporate aspects of embodiments of a therapeutic current control system. In FIGS. 14 and 15, medical devices can include such devices as defibrillators or pacemakers, which can incorporate embodiments described herein, such as a therapeutic current control system or a therapeutic current control network. Medical devices as shown in FIGS. 14 and 15 can further include portable, wearable or handheld varieties.

Referring to FIG. 14, examples of components of various devices discussed with regard to various figures herein, for example, are shown schematically. These devices may include medical device 5110, one or more additional medical device(s) 5210, one or more computing device(s) 5310, and one or more server(s) 1110. In an implementation, at least one of the medical devices 5110 and 5210 may be a therapeutic medical device configured to deliver medical therapy to the patient and may not be limited to patient monitoring and/or diagnostic care. The computing device 5370 may be adapted to function as a medical device. In an implementation, the computing device 5370 may not be a therapeutic medical device configured to deliver therapy to the patient. In such an implementation, the computing device 5370 may be limited to patient monitoring and/or diagnostic care.

One or more of the devices 5110, 5210, 5310, and 1110 may be communicatively coupled via communicative couplings 5298, 5396, 5397, 1170, 1180, and/or 1190. These communicative couplings may be each be a wired and/or a wireless communications link. The wired communications links may include a wired electrically coupling, an optical coupling via an optical cable, etc. The wireless communications link may include coupling via a radio frequency or other transmission media and/or via a network such as a local area network, an ad hoc network, a mesh network, a cellular and/or other communications network, a computer network, etc. The communications links as described herein may utilize protocols such as, for example, 802.11, ZigBee®, Bluetooth®, etc. The communications links may include near field communications which may be implemented via a communications RFID tag. The communications links may include one or more networks such as a local area network, a cellular network, a satellite network, and/or a computer network (e.g., an Internet Protocol (IP) network). In various implementations, the communicative couplings described herein may provide secure and/or authenticated communications channels. In an implementation, the devices described herein may encrypt and/or decrypt the data transmitted and/or received via the communicative couplings.

In FIG. 14, the components 5120, 5121, 5130, 5144, 5145, and 5155 are communicatively coupled (directly and/or indirectly) to each other for bi-directional communication. Similarly, the components 5220, 5221, 5230, 5244, 5245, and 5255 are communicatively coupled (directly and/or indirectly) to each other for bi-directional communication and the components 5320, 5321, 5330, 5344, and 5345 are communicatively coupled (directly and/or indirectly) to each other for bi-directional communication.

Although shown as separate entities in FIG. 14, the components 5120, 5121, 5145, and/or 5155 may be combined into one or more discrete components 5145 and/or 5155 may be part of the processor 5120. The processor 5120 and the memory 5121 may include and/or be coupled to associated circuitry in order to perform the functions described herein. Although shown as separate entities in FIG. 14, the components 5220, 5221, 5245, and/or 2555 may be combined into one or more discrete components 5245 and/or 5255 may be part of the processor 5220. The processor 5220 and the memory 5221 may include and/or be coupled to associated circuitry in order to perform the functions described herein. Although shown as separate entities in FIG. 14, the components 5320, 5321, and 5345 may be combined into one or more discrete components and component 5345 may be part of the processor 5320. The processor 5320 and the memory 5321 may include and/or be coupled to associated circuitry in order to perform the functions described herein.

The medical devices 5110 and/or 5210 may include the therapy delivery control module 5155 or 5255. For example, the therapy delivery control module 5155 and/or 5255 may be an electrotherapy delivery circuit that includes one or more capacitors configured to store electrical energy for a pacing pulse or a defibrillating pulse. The electrotherapy delivery circuit may further include resistors, additional capacitors, relays and/or switches, electrical bridges such as an H-bridge (e.g., including a plurality IGBTs), voltage measuring components, and/or current measuring components. As another example, the therapy delivery control module 5155 and/or 5255 may be a compression device electro-mechanical controller configured to control a mechanical compression device. As a further example, the therapy delivery control module 5155 and/or 5255 may be an electro-mechanical controller configured to control drug delivery, temperature management, ventilation, and/or other type of therapy delivery.

The medical device 5110 (e.g., a first medical device) may incorporate and/or be configured to couple to one or more patient interface device(s) 5160. The patient interface device(s) 5160 may include one or more therapy delivery component(s) 5161a and one or more sensor(s) 5161b. Similarly, the medical device 5210 (e.g., a second medical device) may incorporated and/or be configured to couple to one or more patient interface device(s) 5260. The patient interface device(s) 5260 may include one or more therapy delivery components 5261a and one or more sensors 5261b. The computing device 5370 may be adapted for medical use and may incorporate and/or be configured to couple to one or more patient interface device(s) 5360. The patient interface device(s) 5360 may include one or more sensors 5361. The therapy delivery component(s) 5261a may be substantially as described herein with regard to the therapy delivery component(s) 5161a. Similarly, the sensor(s) 5261b and 5361 may be substantially as described herein with regard to the sensor(s) 5161b.

The medical device 5210 may receive patient data in a manner substantially similar to that described above for the medical device 5110. The device 5210 may receive the patient data based on signals received from the therapy delivery component(s) 5261a and the sensor(s) 5261b. The sensor(s) 5261b may be substantially as described herein with regard to the sensor(s) 5161b.

The sensor(s) 5161b, 5261b, and 5361 may include sensing electrodes (e.g., the sensing electrodes 5162), ventilation and/or respiration sensors (e.g., the ventilation and/or respiration sensors 5164), temperature sensors (e.g., the temperature sensor 5167), chest compression sensors (e.g., the chest compression sensor 5168), etc. For example, the sensing electrodes may include cardiac sensing electrodes. The cardiac sensing electrodes may be conductive and/or capacitive electrodes configured to measure changes in a patient's electrophysiology to measure the patient's ECG information. The sensing electrodes may further measure the transthoracic impedance and/or a heart rate of the patient. The ventilation and/or respiration sensors may include spirometry sensors, flow sensors, pressure sensors, oxygen and/or carbon dioxide sensors such as, for example, one or more of pulse oximetry sensors, oxygenation sensors (e.g., muscle oxygenation/pH), O2 gas sensors and capnography sensors, impedance sensors, and combinations thereof. The temperature sensors may include an infrared thermometer, a contact thermometer, a remote thermometer, a liquid crystal thermometer, a thermocouple, a thermistor, etc. and may measure patient temperature internally and/or externally. The chest compression sensor may include one or more motion sensors including, for example, one or more accelerometers, one or more force sensors, one or more magnetic sensors, one or more velocity sensors, one or more displacement sensors, etc. The chest compression sensor may provide one or more signals indicative of the chest motion to the medical device 5110 and/or 5210 via a wired and/or wireless connection. The chest compression sensor may be, for example, but not limited to, a compression puck, a smart-phone, a hand-held device, a wearable device, etc. The chest compression sensor may be configured to detect chest motion imparted by a rescuer and/or an automated chest compression device (e.g., a belt system, a piston system, etc.). The chest compression sensor may provide signals indicative of chest compression data including displacement data, velocity data, release velocity data, acceleration data, force data, compression rate data, dwell time data, hold time data, blood flow data, blood pressure data, etc. In an implementation, the defibrillation and/or pacing electrodes may include or be configured to couple to the chest compression sensor.

In various implementations, the sensor(s) 5161b, 5261b and/or 5361 may include one or more sensor devices configured to provide sensor data that includes, for example, but not limited to electrocardiogram (ECG), blood pressure, heart rate, respiration rate, heart sounds, lung sounds, respiration sounds, end tidal $CO_2$, saturation of muscle oxygen ($SMO_2$), oxygen saturation (e.g., $SpO_2$ and/or $PaO_2$), cerebral blood flow, point of care laboratory measurements (e.g.,

lactate, glucose, etc.), temperature, electroencephalogram (EEG) signals, brain oxygen level, tissue pH, tissue fluid levels, images and/or videos via ultrasound, laryngoscopy, and/or other medical imaging techniques, near-infrared reflectance spectroscopy, pneumography, cardiography, and/or patient movement. Images and/or videos may be two-dimensional or three-dimensional.

The one or more therapy delivery components 5161a and 5261a may include electrotherapy electrodes (e.g., the electrotherapy electrodes 5166a), ventilation device(s) (e.g., the ventilation devices 5166b), intravenous device(s) (e.g., the intravenous devices 5166c), compression device(s) (e.g., the compression devices 5166d), etc. For example, the electrotherapy electrodes may include defibrillation electrodes, pacing electrodes, and combinations thereof. The ventilation devices may include a tube, a mask, an abdominal and/or chest compressor (e.g., a belt, a cuirass, etc.), etc. and combinations thereof. The intravenous devices may include drug delivery devices, fluid delivery devices, and combinations thereof. The compression devices may include mechanical compression devices such as abdominal compressors, chest compressors, belts, pistons, and combinations thereof. In various implementation, the therapy delivery component(s) 5161a and/or 5261a may be configured to provide sensor data and/or be coupled to and/or incorporate sensors. For example, the electrotherapy electrodes may provide sensor data such as transthoracic impedance, ECG, heart rate, etc. Further the electrotherapy electrodes may include and or be coupled to a chest compression sensor. As another example, the ventilation devices may be coupled to and/or incorporate flow sensors, gas species sensors (e.g., oxygen sensor, carbon dioxide sensor, etc.), etc. As a further example, the intravenous devices may be coupled to and/or incorporate temperature sensors, flow sensors, blood pressure sensors, etc. As yet another example, the compression devices may be coupled to and/or incorporate chest compression sensors, patient position sensors, etc. The therapy delivery control modules 5155 and 5255 may be configured to couple to and control the therapy delivery component(s) 5161a and 5261a, respectively.

The one or more sensor(s) 5161b, 5261b, and 5361 and/or the therapy delivery component(s) 5161a and 5261a may provide sensor data. The patient data provided at the operational interface and/or playback interface may include the sensor data. For example, the medical device 5110 (e.g., the first medical device) may process signals received from the sensor(s) 5161b and/or the therapy delivery component(s) 5161a to determine the sensor data. Similarly, the medical device 5210 may process signals received from the sensor(s) 5261b and/or the therapy delivery component(s) 5261a to determine the sensor data and the computing device 5370 may process signals received from the sensor(s) 5361 to determine the sensor data.

Referring to FIG. 15, examples 1500 of components of the modular therapeutic medical device/patient monitor are shown. The modular therapeutic medical device/patient monitor may include a therapeutic medical device and patient monitor configured to communicatively couple to one another. For example the therapeutic medical device may be a defibrillator and the modular therapeutic medical device/patient monitor may include a defibrillator and patient monitor configured to communicatively couple to one another Although shown as separate entities in FIG. 15, the components 5420a, 5421a, 5445a, and/or 5455 may be combined into one or more discrete components 445a and/or 455 may be part of the processor 420a. Similarly, the components 5420b, 5421b, and/or 5445b may be combined into one or more discrete components and component 5445b may be part of the processor 5420b. The processor 5420a and the memory 5421a may include and/or be coupled to associated circuitry in order to perform the functions described herein. Similarly, the processor 5420b and the memory 5421b may include and/or be coupled to associated circuitry in order to perform the functions described herein. The components 5420a, 5421a, 5430a, 5444a, 5445a, and 5455 are communicatively coupled (directly and/or indirectly) to each other for bi-directional communication. Similarly, the components 5420b, 5421b, 5430b, 5444b, and 5445b are communicatively coupled (directly and/or indirectly) to each other for bi-directional communication

The therapy delivery control module 5455 may be an electrotherapy delivery circuit substantially as described with regard to the therapy delivery control modules 155 and 5255. As another example, the therapy delivery control module 5455 may be a compression device electro-mechanical controller configured to control a mechanical compression device. As a further example, the therapy delivery control module 5455 may be an electro-mechanical controller configured to control drug delivery, temperature management, ventilation, and/or other type of therapy delivery.

The medical device 5110 may include a processor 5120, a memory 5121, one or more output devices 5130, one or more user input devices 5144, and a communications interface 5145. The medical device 5210 may include a processor 5220, a memory 5221, one or more output devices 5230, one or more user input devices 5244, and a communications interface 5245. The computing device 5370 may include a processor 5320, a memory 5321, one or more output devices 5330, one or more user input devices 5344, and a communications interface 5345. The therapeutic medical device 5410a may include a processor 5420a, a memory 5421a, one or more output devices 430a, one or more input devices 5444a, and a communications interface 5445a. The patient monitor 5410b may include a processor 5420b, a memory 5421b, one or more output devices 5430b, one or more input devices 5444b, and a communications interface 5445b.

The processors 5120, 5220, 5320, 5420a, and 5420b are physical processors (i.e., an integrated circuit configured to execute operations on the devices 5110, 5210, 5310, 5410a, and 5410b, respectively, as specified by software and/or firmware stored in a computer storage medium). The processors 5120, 5220, 5320, 5420a, and 5420b are operably coupled, respectively, to the memory 5121, the memory 5221, the memory 5321, the memory 5421a, and the memory 5421b. The processors 5120, 5220, 5320, 5420a, and 5420b may be intelligent hardware devices (for example, but not limited to, a central processing unit (CPU), a graphics processing unit (GPU), one or more microprocessors, a controller or microcontroller, an application specific integrated circuit (ASIC), a digital signal processor (DSP), etc.) designed to perform the functions described herein and operable to carry out instructions on the devices 5110, 5210, 5310, 5410a, and 5410b, respectively. Each of the processors 5120, 5220, 5320, 5420a, and 5420b may be one or more processors and may be implemented as a combination of hardware devices (e.g., a combination of DSP and a microprocessor, a plurality of microprocessors, one or more microprocessors in conjunction with a DSP core, or another such configuration). Each of the processors 5120, 5220, 5320, 5420a, and 5420b may include multiple separate physical entities that may be distributed in the devices 5110, 5210, 5310, 5410a, and 5410b respectively. Each of the processors 5120, 5220, 5320, 5420a, and 5420b is configured to execute processor-readable, processor-executable software code containing one or more instructions or code for controlling the processors 5120, 5220, 5320, 5420a, and 5420b to perform the functions as described herein. The processors 5120, 5220, 5320, 5420a, and/or 5420b may utilize various architectures including but not limited to a complex instruction set computer (CISC) processor, a reduced instruction set computer (RISC) processor, or a minimal instruction set computer (MISC). In various implementations, the processors 5120, 5220, 5320, 5420a and/or 5420b may be a single-threaded or a multi-threaded processor. The processors 5120, 5220, 5320, 5420a, and/or 5420b may be, for example, an Intel® Itanium® or Itanium 2® processor(s), AMD® Opteron®, Athlon MP® processor(s), a Motorola ® line of processor, or an ARM, Intel Pentium Mobile, Intel Core i5 Mobile, AMD A6 Series, AMD Phenom II Quad Core Mobile, or like devices.

The memories 5121, 5221, 5321, 5421a, and 5421b refer generally to a computer storage medium, including but not limited to RAM, ROM, FLASH, disc drives, fuse devices, and portable storage media, such as Universal Serial Bus (USB) flash drives, etc. Each of the memories 5121, 5221, 5321, 5421a, and 5421b may include, for example, random access memory (RAM), or another dynamic storage device(s) and may include read only memory (ROM) or another static storage device(s) such as programmable read only memory (PROM) chips for storing static information such as instructions for a coupled processor (e.g., one of the processors 5120, 5220, 5320, 5420a, and 5420b). The memories 5121, 5221, 5321, 5421a, and 5421b may include USB flash drives that may store operating systems and other applications. The USB flash drives may include input/output components, such as a wireless transmitter and/or USB connector that can be inserted into a USB port of another computing device. The memories 5121, 5221, 5321, 5421a, and/or 5421b may be long term, short term, or other memory associated with the respective device 5110, 5210, 5310, 5410a, and 5410b and are not to be limited to a particular type of memory or number of memories, or type of media upon which memory is stored. The memories 5121, 5221, 5321, 5421a, and/or 5421b include a non-transitory processor-readable storage medium (or media) that stores the processor-readable, processor-executable software code. The memories 5121, 5221, 5321, 5421a, and/or 5421b may store information and instructions. For example, the memories 5121, 5221, 5321, 5421a, and/or 5421b may include flash memory and/or another storage media may be used, including removable or dedicated memory in a mobile or portable device. As another example, hard disks such as the Adaptec® family of SCSI drives, an optical disc, an array of disks such as RAID (e.g. the Adaptec family of RAID drives), or another mass storage devices may be used. The memories 5121, 5221, 5321, 5421a, and/or 5421b may include removable storage media such as, for example, external hard-drives, floppy drives, flash drives, zip drives, compact disc - read only memory (CD-ROM), compact disc - re-writable (CD-RW), or digital video disk - read only memory (DVD-ROM).

The communications interfaces 5145, 5245, 5345, 5445a, and 5445b may transmit and/or receive information to and/or from one or more devices external to and communicatively coupled to the devices 5110, 5210, 5310, 5410a, and 5410b, respectively. The communications interfaces 5145, 5245, 5345, 5445a, and 5445b may transmit and/or receive the information via a wired and/or wireless communicative coupling (e.g., the couplings 5298, 5396, 5397, 5398a, 5398b, 5498, 1170, 1180, 1190, 1292, or 1293). The information may include information stored in at least one of the memories 5121, 5221, 5321, 5421a, and 5421b. The information may include, for example, but not limited to, resuscitative treatment information, physiological information, patient information, rescuer and/or caregiver information, location information, rescue and/or medical treatment center information, etc. The communications interfaces 5145, 5245, 5345, 5445a, and/or 5445b may enable short range and/or long range wireless communications capabilities which may include communications via near field communications, ZigBee®, Wi-Fi, Bluetooth®, satellite(s), radio waves, a computer network (e.g., the Internet), a cellular network, etc. The communications interfaces 5145, 5245, 5345, 5445a, and/or 5445b may enable communications via a network such a Local Area Network (LAN), Wide Area Network (WAN), a mesh network, an ad hoc network, or another network. The communications interfaces 5145, 5245, 5345, 5445a, and/or 5445b may include, for example, an RS-232 port for use with a modem based dialup connection, a copper or fiber 10/100/1000 Ethernet port, or a Bluetooth® or WiFi interface.

In an implementation, the communications interfaces 5145, 5245, 5345, 5445a, and/or 5445b may enable communication between one or more of the devices 5110, 5210, 5310, 5410a, and 410b with one or more servers 1110. For example, the one or more servers 1110 may be remote servers and may include a cloud server and/or a central facility server. In an implementation, the one or more servers 1110 may be associated with a medical provider (e.g., a hospital, a physician's office, a medical records office, an emergency services office, an emergency services vehicle, a dispatch center, etc.). In an implementation, the communications interface 5445b may enable the patient monitor 5410b to communicatively couple with multiple therapeutic medical device(s) 5410a and/or with another patient monitor. In an implementation, the therapeutic medical device 5410a may communicatively couple with the one or more servers 1110 via the patient monitor 5410b. For example, the communications interface 5445a may provide patient data and/or other information from the therapeutic medical device 5410a to the patient monitor 5410b via the communicative coupling 5498. The patient monitor 5410b may merge the received patient data and/or other information with patient data and/or other information collected by and/or generated at the patient monitor 5410b to create an integrated record. The patient monitor 4510b may provide the integrated record to the one or more servers 1110 via the communications interface 5445b and the communicative coupling 1292. Alternatively or additionally, the therapeutic medical device 5410a may provide patient data and/or other information to the one or more servers 1110 via the communications interface 5445a and the communicative coupling 1293. One or more of the communications interfaces 5145, 5245, 5345, 5445a, and 5445b in combination with one or more of the communicative couplings 5298, 5396, 5397, 5398a, 5398b, 5498, 1170, 1180, 1190, 1292, and 1293 may enable telemedicine communications and data sharing of real-time and/or historical patient data in support of the telemedicine communications.

The output device(s) 5130 and user input device(s) 5144 may be included in the medical device 5110 and/or coupled to the medical device 5110. Similarly, the output device(s) 5230 and the user input device(s) 5244 may be included in the medical device 5210 and/or coupled to the medical device 5210, the output device(s) 5330 and the user input device(s) 5344 may be included in the computing device 5370 and/or coupled to the computing device 5370, the output device(s) 5430a and the user input device(s) 5444a may be included in the therapeutic medical device 5410a and/or coupled to the therapeutic medical device 5410a, and the output device(s) 5430b and the user input device(s) 5444b may be included in the patient monitor 5410b and/or coupled to the patient monitor 5410b. For example, the output device(s) 5130, 5230, 5330, 5430a, and/or 5430b may include one or more of a display (e.g., the displays 5115, 5215, 5315, 5415a, and 5415b), a speaker, and a haptic device. The display may provide a graphical user interface (GUI). The display may be, for example, but not limited to, a liquid crystal display (LCD) and/or a light emitting diode (LED) display. In an implementation, the output device(s) 5130, 5230, 5330, 5430a, and/or 5430b may be input/output device(s) capable of capturing user input. For example, the display may be a touchscreen. The touchscreen may be, for example, a pressure sensitive touchscreen or a capacitive touchscreen. The touchscreen may capture user input provided via touchscreen gestures and/or provided via exertions of pressure on a particular area of the screen. Examples of touchscreen gestures provided herein with regard to user input may include pushing on the touchscreen to exert pressure that exceeds a particular threshold to indicate an input to a pressure sensitive touchscreen by the user. The touchscreen and the controlling processor (e.g., 5120, 5220, 5320, 5420a, and/or 5420b) may be configured recognize touchscreen gestures including, for example, but not limited to, tap, double tap, caliper gesture, drag and drop, slide, press and drag, hold and press, etc. In an implementation, the processors 5120, 5220, 5320, 5420a, and/or 5420b may control a respective display to provide visual representations of data captured by and/or received at the device 5110, 5210, 5310, 5410a, and/or 5410b. The visual representations may include still images and/or video images (e.g., animated images).

In an implementation, the output device(s) 5130, 5230, 5330, 5430a, and 5430b and/or the input device(s) 5144, 5244, 5344, 5444a, and 5444b may include wearable devices such as, for example, a heads-up display mounted onto eyeglasses, a face shield, a watch, and/or devices that may be integrated with other wearable communications devices, such as, for example, an ear bud or a Bluetooth® hands free phone adaptor. The processors 5120, 5220, 5320, 5420a, and 5420b may control the output devices 5130, 5230, 55330, 430a, and 5430b respectively, to provide information for the user. The information may include feedback (e.g., visible feedback, audible feedback, haptic feedback, numerical feedback, and graphical feedback) such as CPR feedback.

The one or more user input devices 5144, 5244, 5344, 5444a, and 5444b may include, for example, a keyboard, a mouse, joystick, trackball, or other pointing device, a microphone, a camera, etc. Further, the user input devices 5144, 5244, 5344, 5444a, and 5444b may be a touchscreen and/or another input/output device capable of providing information for the user and capturing information from the user. The touchscreen may be a pressure sensitive touchscreen

In an implementation, the user input devices 5144, 5244, 5344, 5444a, and/or 5444b are configured to capture information, such as, for example, patient medical history (e.g., medical record information including age, gender, weight, body mass index, family history of heart disease, cardiac diagnosis, co-morbidity, left ventricular ejection fraction, medications, previous medical treatments, and/or other physiological information), physical examination results, patient identification, caregiver identification, healthcare facility information, etc.

The processor, memory, communications, and input and/or output components described above are meant to exemplify some types of possibilities. In no way should the aforementioned examples limit the scope of the disclosure, as they are only exemplary embodiments of these components.

Various modifications and additions can be made to the exemplary embodiments discussed without departing from the scope of the present disclosure. For example, while the embodiments described above refer to particular features, the scope of the disclosure also includes embodiments having different combinations of features and embodiments that do not include all of the described features. Accordingly, the scope of the present disclosure is intended to embrace all such alternatives, modifications, and variations as fall within the scope of the claims, together with all equivalents thereof.

**Claims**

1. A system (250) for generating an electrotherapeutic pulse to be delivered to a patient (229), the system comprising:

    an energy storage capacitor (223) for providing electrotherapeutic current to the patient (229);
    a therapeutic current control network (238), electrically coupled to the energy storage capacitor (223), for controlling the electrotherapeutic current to be delivered to the patient (229), the therapeutic current control network (238) comprising a resonant electrical circuit (225) and at least one current control switch (224), wherein energy provided by the energy storage capacitor (223) flows through the resonant electrical circuit (225), wherein the resonant electrical circuit (225) is configured to boost a voltage of the electrotherapeutic pulse to be delivered to the patient (229); and
    a controller (233) electrically coupled to the energy storage capacitor (223) and the therapeutic current control network (238), wherein the controller (233) is configured to, in connection with the energy provided by the energy storage capacitor (223) that flows through the resonant electrical circuit (225), control operation of the at least one current control switch (224) of the therapeutic current control network (238) in delivering an electrotherapeutic waveform to the patient (229) to correspond with a specified waveform.

2. The system (250) of claim 1, comprising the at least one current control switch (224) comprising at least one MOSFET, wherein the control of the operation of the at least one current control switch (224) in delivering the electrotherapeutic waveform to the patient (229) to correspond with the specified waveform comprises controlling a switching frequency of the at least one current control switch (224).

3. The system (250) of claim 1 or claim 2, wherein the resonant electrical circuit (225) is connected with the patient (229) in a parallel configuration.

4. The system (250) of any of claims 1 to 3, wherein: the energy storage capacitor (223) and a first portion of the therapeutic current control network (238) comprising an inductor and are connected via a first node, the first portion of the therapeutic current control network (238) and a second portion of the therapeutic current control network (238) comprising the resonant electrical circuit (225) and a rectifier (226) are connected via a second node, and the second portion of the therapeutic current control network (238) and a third portion of the therapeutic current control network (238) comprising a capacitor and a patient load are connected via a third node.

5. The system (250) of claim 4, wherein the at least one current control switch (224) is connected with at least a portion of the resonant electrical circuit (225) in a series configuration.

6. The system (250) of any of claims 1 to 5, comprising at least one sensor (232) configured to sense at least one electrical parameter from which current flow to the patient (229) can be determined or estimated, wherein the controller (233) is configured to: process a signal associated with the sensed at least one electrical parameter; compare the processed signal with a second signal associated with the specified waveform; and control operation of the at least one current control switch (224) of the therapeutic current control network (238) in adjusting delivery of the electrotherapeutic waveform to the patient (229) to correspond with the specified waveform.

7. The system (250) of any of claims 1 to 6, comprising a battery (215) and a bidirectional charging control network (216), wherein: the battery (215) and the bidirectional charging control network (216) are connected via a first node and a second node, and the bidirectional charging control network (216) and the energy storage capacitor (223) are connected via a third node and a fourth node, wherein: the bidirectional charging control network (216) is for controlling energy flow from the battery (215) to the energy storage capacitor (223) for storage by the energy storage capacitor (223), and from the energy storage capacitor (223) to the battery (215) for storage by the battery (215).

8. The system (250) of claim 7, comprising a rectifier (219) is configured for use in conversion of alternating current to direct current and wherein the bidirectional charging control network (216) comprises a second resonant electrical circuit (218).

9. The system (250) of claim 8, wherein the bidirectional charging control network (216) comprises a second resonant electrical circuit (218).

10. The system (250) of any preceding claim, wherein the resonant electrical circuit (218, 225) comprises a resonant tank.

11. The system (250) of claim 10, wherein the resonant tank comprises at least one of: an LCC resonant tank, a CLL resonant tank, an LLC resonant tank and an LCLC resonant tank.

12. The system (250) of any preceding claim, wherein a computerized mobile device is configured to deliver the electrotherapeutic pulse to the patient (229), wherein the electrotherapeutic pulse has an energy of between 1 - 400 joules, optionally wherein the computerized mobile device has:

> an energy density, given by an energy of the electrotherapeutic pulse to be delivered to the patient (229) relative to a volume defined by a housing of the mobile device, of between 0.5 - 4.00 joules per cubic centimeter or a volume, defined by a housing of the mobile device, of between 100 - 1,200 cubic centimeters.

13. The system (250) of any preceding claim, wherein the electrotherapeutic waveform is for defibrillation or pacing.

14. The system (250) of any preceding claim, wherein the therapeutic current control network (238) comprises a patient relay circuit configured for use in control of allowing current flow through a patient load, wherein the patient relay circuit comprises at least one patient relay switch comprising a wide bandgap material.

15. The system (250) of any preceding claim, wherein a peak energy efficiency of the system in delivering the electrotherapeutic pulse to the patient (229) is at least 80 percent, wherein the electrotherapeutic pulse delivered to the patient (229) delivers an amount of energy to the patient (229) that is no more than 15 percent different than an amount of energy of the specified waveform and wherein the specified waveform is a biphasic rectilinear current waveform.

**Patentansprüche**

1. System (250) zum Erzeugen eines elektrotherapeutischen Impulses, der an einen Patienten (229) abzugeben ist, das System umfassend:

> einen Energiespeicherkondensator (223) zum Bereitstellen von elektrotherapeutischem Strom für den Patienten (229); ein therapeutisches Stromsteuerungsnetzwerk (238), das elektrisch mit dem Energiespeicherkondensator (223) gekoppelt ist, um den an den Patienten (229) abzugebenden elektrotherapeutischen Strom zu steuern, wobei das therapeutische Stromsteuerungsnetzwerk (238) eine elektrische Resonanzschaltung (225) und mindestens einen Stromsteuerungsschalter (224) umfasst, wobei die von dem Energiespeicherkondensator (223) bereitgestellte Energie durch die elektrische Resonanzschaltung (225) fließt, wobei die elektrische Resonanzschaltung (225) konfiguriert ist, um eine Spannung des an den Patienten (229) abzugebenden elektrotherapeutischen Impulses zu erhöhen; und eine Steuerung (233), die elektrisch mit dem Energiespeicherkondensator (223) und dem therapeutischen Stromsteuerungsnetzwerk (238) verbunden ist, wobei die Steuerung (233) konfiguriert ist, um in Verbindung mit der von dem Energiespeicherkondensator (223) bereitgestellten Energie, die durch die elektrische Resonanzschaltung (225) fließt, den Betrieb des mindestens einen Stromsteuerungsschalters (224) des therapeutischen Stromsteuerungsnetzwerks (238) zu steuern, um eine elektrotherapeutische Wellenform an den Patienten (229) zu liefern, die einer spezifizierten Wellenform entspricht.

2. System (250) nach Anspruch 1, umfassend den mindestens einen Stromsteuerungsschalter (224), der mindestens einen MOSFET umfasst, wobei die Steuerung des Betriebs des mindestens einen Stromsteuerungsschalters (224) bei der Abgabe der elektrotherapeutischen Wellenform an den Patienten (229), um der spezifizierten Wellenform zu entsprechen, die Steuerung einer Schaltfrequenz des mindestens einen Stromsteuerungsschalters (224) umfasst.

3. System (250) nach Anspruch 1 oder 2, wobei die elektrische Resonanzschaltung (225) mit dem Patienten (229) in einer Parallelkonfiguration verbunden ist.

4. System (250) nach einem der Ansprüche 1 bis 3, wobei: der Energiespeicherkondensator (223) und ein erster Abschnitt des therapeutischen Stromsteuerungsnetzwerks (238), der eine Induktivität umfasst, über einen ersten Knotenpunkt verbunden sind, der erste Abschnitt des therapeutischen Stromsteuerungsnetzwerks (238) und ein zweiter Abschnitt des therapeutischen Stromsteuerungsnetzwerks (238), der die elektrische Resonanzschaltung (225) und einen Gleichrichter (226) umfasst, über einen zweiten Knotenpunkt verbunden sind, und der zweite Abschnitt des therapeutischen Stromsteuerungsnetzwerks (238) und ein dritter Abschnitt des therapeutischen Stromsteuerungsnetzwerks (238), der einen Kondensator und eine Patientenlast umfasst, über einen dritten Knotenpunkt verbunden sind.

5. System (250) nach Anspruch 4, wobei der mindestens eine Stromsteuerungsschalter (224) mit mindestens einem Abschnitt der elektrischen Resonanzschaltung (225) in einer Reihenkonfiguration verbunden ist.

6. System (250) nach einem der Ansprüche 1 bis 5, umfassend mindestens einen Sensor (232), der konfiguriert ist, um mindestens einen elektrischen Parameter zu erfassen, aus dem der Stromfluss zu dem Patienten (229) bestimmt oder geschätzt werden kann, wobei die Steuerung (233) konfiguriert ist zum: Verarbeiten eines Signals, das mit dem erfassten mindestens einen elektrischen Parameter verbunden ist; Vergleichen des verarbeiteten Signals mit einem zweiten Signal, das mit der spezifizierten Wellenform verbunden ist; und Steuern des Betriebs des mindestens einen Stromsteuerungsschalters (224) des therapeutischen Stromsteuerungsnetzwerks (238) beim Einstellen der Abgabe der elektrotherapeutischen Wellenform an den Patienten (229), um der spezifizierten Wellenform zu entsprechen.

7. System (250) nach einem der Ansprüche 1 bis 6, umfassend eine Batterie (215) und ein bidirektionales Ladesteuernetzwerk (216), wobei: die Batterie (215) und das bidirektionale Ladesteuernetzwerk (216) über einen ersten Knoten und einen zweiten Knoten verbunden sind, und das bidirektionale Ladesteuernetzwerk (216) und der Energiespeicherkondensator (223) über einen dritten Knoten und einen vierten Knoten verbunden sind, wobei: das bidirektionale Ladesteuernetzwerk (216) zum Steuern des Energieflusses von der Batterie (215) zu dem Energiespeicherkondensator (223) zur Speicherung durch den Energiespeicherkondensator (223) und von dem Energiespeicherkondensator (223) zu der Batterie (215) zur Speicherung durch die Batterie (215) dient.

8. System (250) nach Anspruch 7, wobei ein Gleichrichter (219) zur Verwendung bei der Umwandlung von Wechselstrom in Gleichstrom konfiguriert ist und wobei das bidirektionale Ladesteuernetzwerk (216) eine zweite elektrische Resonanzschaltung (218) umfasst.

9. System (250) nach Anspruch 8, wobei das bidirektionale Ladesteuernetzwerk (216) eine zweite elektrische Resonanzschaltung (218) umfasst.

10. System (250) nach einem der vorstehenden Ansprüche, wobei die elektrische Resonanzschaltung (218, 225) einen Resonanztank umfasst.

11. System (250) nach Anspruch 10, wobei der Resonanztank mindestens eines von Folgendem umfasst: einen LCC-Resonanztank, einen CLL-Resonanztank, einen LLC-Resonanztank und einen LCLC-Resonanztank.

12. System (250) nach einem der vorstehenden Ansprüche, wobei eine computergestützte mobile Vorrichtung konfiguriert ist, um den elektrotherapeutischen Impuls an den Patienten (229) abzugeben, wobei der elektrotherapeutische Impuls eine Energie zwischen 1-400 Joule aufweist, wobei optional die computergestützte mobile Vorrichtung Folgendes aufweist:

eine Energiedichte, angegeben durch die Energie des an den Patienten (229) abzugebenden elektrotherapeutischen Impulses, bezogen auf ein durch ein Gehäuse der mobilen Vorrichtung definiertes Volumen, zwischen 0,5-4,00 Joule pro Kubikzentimeter oder
ein durch ein Gehäuse der mobilen Vorrichtung definiertes Volumen zwischen 100-1.200 Kubikzentimeter.

13. System (250) nach einem der vorstehenden Ansprüche, wobei die elektrotherapeutische Wellenform zur Defibrillation oder Stimulation dient.

14. System (250) nach einem der vorstehenden Ansprüche, wobei das therapeutische Stromsteuerungsnetzwerk (238) eine Patientenrelaisschaltung umfasst, die konfiguriert ist, um den Stromfluss durch eine Patientenlast zu steuern, wobei die Patientenrelaisschaltung mindestens einen Patientenrelaisschalter umfasst, der ein Material mit breiter Bandlücke umfasst.

15. System (250) nach einem der vorstehenden Ansprüche, wobei eine Spitzenenergieeffizienz des Systems bei der Abgabe des elektrotherapeutischen Impulses an den Patienten (229) mindestens 80 Prozent beträgt, wobei der an den Patienten (229) abgegebene elektrotherapeutische Impuls eine Energiemenge an den Patienten (229) abgibt, die sich um nicht mehr als 15 Prozent von einer Energiemenge der spezifizierten Wellenform unterscheidet, und wobei die spezifizierte Wellenform eine biphasische, geradlinige Stromwellenform ist.

**Revendications**

1. Système (250) de génération d'une impulsion électrothérapeutique destinée à être administrée à un patient (229), le système comprenant :

   un condensateur de stockage d'énergie (223) pour fournir un courant électrothérapeutique au patient (229) ; un réseau de commande du courant thérapeutique (238), couplé électriquement au condensateur de stockage d'énergie (223), pour commander le courant électrothérapeutique à administrer au patient (229), le réseau de commande du courant thérapeutique (238) comprenant un circuit électrique résonant (225) et au moins un commutateur de commande de courant (224), dans lequel l'énergie fournie par le condensateur de stockage d'énergie (223) circule à travers le circuit électrique résonant (225), dans lequel le circuit électrique résonant (225) est configuré pour amplifier la tension de l'impulsion électrothérapeutique à administrer au patient (229) ; et un dispositif de commande (233) couplé électriquement au condensateur de stockage d'énergie (223) et au réseau de commande du courant thérapeutique (238), dans lequel le dispositif de commande (233) est configuré pour, en relation avec l'énergie fournie par le condensateur de stockage d'énergie (223) qui circule à travers le circuit électrique résonant (225), commander le fonctionnement de l'au moins un commutateur de commande de courant (224) du réseau de commande du courant thérapeutique (238) pour administrer une forme d'onde électrothérapeutique au patient (229) afin de correspondre à une forme d'onde spécifiée.

2. Système (250) selon la revendication 1, comprenant l'au moins un commutateur de commande de courant (224) comprenant au moins un MOSFET, dans lequel la commande du fonctionnement de l'au moins un commutateur de commande de courant (224) pour adminsitrer la forme d'onde électrothérapeutique au patient (229) afin de correspondre à la forme d'onde spécifiée comprend la commande d'une fréquence de commutation de l'au moins un commutateur de commande de courant (224).

3. Système (250) selon la revendication 1 ou la revendication 2, dans lequel le circuit électrique résonant (225) est relié au patient (229) dans une configuration parallèle.

4. Système (250) selon l'une quelconque des revendications 1 à 3, dans lequel : le condensateur de stockage d'énergie (223) et une première partie du réseau de commande du sont reliésapeutique (238) comprenant une inductance sont reliés via un premier nœud, la première partie du réseau de commande du courant thérapeutique (238) et une deuxième partie du réseau de commande du courant thérapeutique (238) comprenant le circuit électrique résonant (225) et un redresseur (226) sont connectés via un deuxième nœud, et la deuxième partie du réseau de commande du courant thérapeutique (238) et une troisième partie du réseau de commande du courant thérapeutique (238) comprenant un condensateur et une charge de patient sont reliés via un troisième nœud.

5. Système (250) selon la revendication 4, dans lequel l'au moins un commutateur de commande de courant (224) est relié à au moins une partie du circuit électrique résonant (225) dans une configuration en série.

6. Système (250) selon l'une quelconque des revendications 1 à 5, comprenant au moins un capteur (232) configuré pour détecter au moins un paramètre électrique à partir duquel le flux de courant vers le patient (229) peut être déterminé ou estimé, dans lequel le dispositif de commande (233) est configuré pour : traiter un signal associé à l'au moins un paramètre électrique détecté ; comparer le signal traité avec un second signal associé à la forme d'onde spécifiée ; et commander le fonctionnement de l'au moins un commutateur de commande de courant (224) du réseau de commande du courant thérapeutique (238) en ajustant l'administration de la forme d'onde électrothérapeutique au patient (229) pour correspondre à la forme d'onde spécifiée.

7. Système (250) selon l'une quelconque des revendications 1 à 6, comprenant une batterie (215) et un réseau de commande de charge bidirectionnel (216), dans lequel : la batterie (215) et le réseau de commande de charge bidirectionnel (216) sont reliés via un premier nœud et un deuxième nœud, et le réseau de commande de charge bidirectionnel (216) et le condensateur de stockage d'énergie (223) sont reliés via un troisième nœud et un quatrième nœud, dans lequel : le réseau de commande de charge bidirectionnel (216) sert à commander le flux d'énergie de la batterie (215) vers le condensateur de stockage d'énergie (223) pour le stockage par le condensateur de stockage d'énergie (223), et du condensateur de stockage d'énergie (223) vers la batterie (215) pour le stockage par la batterie (215).

8.  Système (250) selon la revendication 7, comprenant un redresseur (219), qui est configuré pour être utilisé dans la conversion d'un courant alternatif en un courant continu et dans lequel le réseau de commande de charge bidirectionnel (216) comprend un second circuit électrique résonant (218).

9.  Système (250) selon la revendication 8, dans lequel le réseau de commande de charge bidirectionnel (216) comprend un second circuit électrique résonant (218).

10. Système (250) selon une quelconque revendication précédente, dans lequel le circuit électrique résonant (218, 225) comprend un réservoir résonant.

11. Système (250) selon la revendication 10, dans lequel le réservoir résonant comprend au moins un des éléments suivants : un réservoir résonant LCC, un réservoir résonant CLL, un réservoir résonant LLC et un réservoir résonant LCLC.

12. Système (250) selon une quelconque revendication précédente, dans lequel un dispositif mobile informatisé est configuré pour administrer l'impulsion électrothérapeutique au patient (229), dans lequel l'impulsion électrothérapeutique a une énergie comprise entre 1 et 400 joules, éventuellement dans lequel le dispositif mobile informatisé a :

    une densité d'énergie, donnée par l'énergie de l'impulsion électrothérapeutique à administrer au patient (229) par rapport à un volume défini par le boîtier du dispositif mobile, comprise entre 0,5 et 4,00 joules par centimètre cube ou
    un volume, défini par le boîtier du mobile, compris entre 100 et 1 200 centimètres cubes.

13. Système (250) selon une quelconque revendication précédente, dans lequel la forme d'onde électrothérapeutique est destinée à la défibrillation ou à la stimulation.

14. Système (250) selon une quelconque revendication précédente, dans lequel le réseau de commande du courant thérapeutique (238) comprend un circuit de relais patient configuré pour être utilisé dans la commande de la circulation du courant à travers une charge de patient, dans lequel le circuit de relais patient comprend au moins un commutateur de relais patient comprenant un matériau à large bande interdite.

15. Système (250) selon une quelconque revendication précédente, dans lequel l'efficacité énergétique maximale du système dans l'administration de l'impulsion électrothérapeutique au patient (229) est d'au moins 80 pour cent, dans lequel l'impulsion électrothérapeutique administrée au patient (229) administre une quantité d'énergie au patient (229) qui n'est pas différente de plus de 15 pour cent d'une quantité d'énergie de la forme d'onde spécifiée et dans lequel la forme d'onde spécifiée est une forme d'onde de courant rectiligne biphasique.

FIG. 1A

100

Network
101

102
Defibrillator

106

109

110

108

105

104

103
Therapeutic current
control system

107

EP 4 440 690 B1

110

111 Network

112 Handheld defibrillator

116

114

115

117

113

Therapeutic current control system

118

FIG. 1B

**Therapeutic current control system**

286

281

| Power supply (may include a bidirectional charging control network) |

Therapeutic current control network

282

283

| Current control switch(es) | Resonant tank |

287

| Patient |

284

| Specified current waveform (e.g., for matching/generating) |

285

| Controller |

FIG. 2A

EP 4 440 690 B1

**Therapeutic current control system**

200

201 — Power Supply

202

203 — Current control switch(es)

204 — Resonant tank

205 — Rectifier

206 — Filter

Therapeutic current control network

207 — Patient

208 — Electrical parameter sensor(s)

216 — Closed loop current control

212 — Controller

209 — Signal(s) indicative of current output to patient

214 — Specified current waveform (for matching/generating)

**FIG. 2B**

250

215

Battery · **Therapeutic current control system**

216

217

**Bidirectional charging
control network (BCCN)** 222

218 219

| BCCN
Switch
network | Resonant
tank | Rectifier | Filter |

**FIG. 2C**

223

Energy
storage
capacitor

Soft-switching,
wide bandgap
material
switches

238

**Therapeutic
current control
network**

224 225 227 228

226

| Current
control
switch(es) | Resonant
tank | Rectifier | Filter | Polarity
control |

229 232

**Patient** ¦ Current,
voltage
sensors

233

**Controller**

234

FPGA/DSP

**Specified
current
waveform**

235

236

Analog to
Digital
Converter
(ADC)

239

Closed loop
current
control

Signals corresponding
with current and
voltage output to
patient

237

500

Charge energy storage capacitor to
provide electrotherapy current energy

502

504

**Closed loop current control during delivery of
electrotherapeutic waveform to patient**

506

Controller compares signal associated with
desired waveform with signal associated with
waveform being delivered

508

Based at least in part on comparison,
controller controls operation of current
control switch(es) to adjust waveform being
delivered to match desired waveform

# FIG. 3

**Therapeutic current control system**

401
Therapeutic current
control network

402

Power
Supply

404

405

Resonant
tank
(parallel
or series
config)

Current
control
switch(es)

Mode
control
switch(es)

Rectifier

Filter

408

Patient

Electrical
parameter
sensor(s)

411

Specified
current waveform
(for matching/generating)

Controller

Closed loop
current
control

Signal(s) indicative of
current output to
patient

# FIG. 4A

EP 4 440 690 B1

Therapeutic current control system 415

Resonant tank based therapeutic current control network 428

Energy storage capacitor 416

C5

High side switch 417a

417 Current control switches

Nb1 Nb2

M1

D3

M2 417b Low side switch

Timing control

414 Controller

L1 418a

C1 418b

418 Resonant tank

D4

Nb3 427a

W1

Nb4

W2 427b

427 Mode control switches

Series/Parallel mode switch (shown in parallel mode)

Nb5

Nb6

Rectification and filtering

L2 Nb7

D1

C2

D2 Nb8 L3

C3

422 Filter

421 Rectifier (D1 and D2)

Nb9

Nb10

Z1

Elec param sensor 429

Current sense

R1

Patient 426

Z2

Z3

Z4

425 Polarity control

425 Polarity control

FIG. 4B

52

FIG. 4C

EP 4 440 690 B1

FIG. 4D

EP 4 440 690 B1

FIG. 5A

**Therapeutic current control system**

515

502 — Therapeutic current control network

504 — Resonant tank (parallel config)

Current control switch(es)

Rectifier

Filter

Power Supply

Controller

Specified current waveform (for matching/generating)

Signal(s) indicative of current output to patient

Closed loop current control

507

Patient

Electrical parameter sensor(s)

Therapeutic current control system —530

Resonant tank based **therapeutic current control network** —531

Energy storage capacitor 521

High side switch

Na1    Na2

523 Current control switches

M1    D3

Timing control

D4

522 Controller

M2

Low side switch

Resonant converter defibrillation waveform generator

Na3

L1    C1

Na4

524 Resonant tank

525

Rectification and filtering 526

Na5    Na7

D1    L2    C2

D2 Na6 L3

C3    Na8

526 Rectifier (D1 and D2)

527 Filter

Z1    Elec param sensor 528    Z2

Current sense    R1

Patient    532

Z3    Z4

529 Polarity control    529 Polarity control

FIG. 5B

EP 4 440 690 B1

Patient voltage delivered from Parallel Resonant Converter

FIG. 6

EP 4 440 690 B1

FIG. 7

EP 4 440 690 B1

FIG. 8

FIG. 8A

**900 Therapeutic current control system**

901

Therapeutic current control network

903 — Filter/energy storage inductor

904 — Resonant tank

905 — Current control switch(es)

906 — Rectifier

907 — Filter capacitor

908 — Patient

909 — Electrical parameter sensor(s)

902 — Power Supply

912 — Closed loop current control

911 — Controller

910 — Specified current waveform (for matching/generating)

Signal(s) indicative of current output to patient

FIG. 9A

FIG. 9B

EP 4 440 690 B1

## 930

One complete
switching duty cycle

**931**

**932**
**"On" period**
of switching
duty cycle

**934** Current control **switch S turns on**

**935** Current in Lr increases until **current in Lr > current in Lf**

**936** Current in Lr continues increasing, now sourced by Cr

**937** When voltage on Cr < voltage on Cap, current in Lf increases and energy stored in Cr and transferred to Lr

**938** When voltage on Cr = 0: current in Lr decreases, energy in Lr transfers to Lf and Cr, and voltage on Cr becomes negative.

**939** When Lr = 0, **switch S turns off**

**933**
**"Off" period**
of switching
duty cycle

**940** At switch S turn off:
voltage on Cr is negative, and |voltage on Cr| approx. = |voltage on Cap|,
voltage on Lf = almost 2X voltage on Cap
current in Lf is increasing

**941** Energy in Cr transfers to Lf, then energy in Lf transfers back to Cr, while voltage on Cr increases until:
**voltage on Cr = output voltage to patient load**

**942** Remaining energy in the filter inductor (Lf) transfers to the patient load

## FIG. 10

FIG. 11

600

Example three element resonant tank
of a therapeutic current control network

$L_P$ separate from transformer

FIG. 12A

EP 4 440 690 B1

605

# Example three element resonant tank
# of a current control network

$L_P$ effectively supplied by transformer

606

EP 4 440 690 B1

$L_R$

$C_P$

$L_P$

# FIG. 12B

Example three element resonant tank
of a current control network

**LCC resonant tank**

$C_R$  $L_R$  $C_P$

FIG. 12C

EP 4 440 690 B1

615

# Example three element resonant tank
# of a current control network

## CLL resonant tank

FIG. 12D

Example three element resonant tank of a
Therapeutic current control network

FIG. 12E

EP 4 440 690 B1

Example three element resonant tank of a
Therapeutic current control network

FIG. 12F

EP 4 440 690 B1

EP 4 440 690 B1

Example three element resonant tank
of a therapeutic current control network

630

$L_P$ separate from transformer

**LLC resonant tank**

632

$C_R$   $L_R$   $L_P$

## FIG. 12G

635 Example three element resonant tank of a therapeutic current control network

$L_P$ effectively supplied by transformer

**LLC resonant tank**

636

$C_R$   $L_R$   $L_P$

FIG. 12H

Example three element resonant tank of a
Therapeutic current control network

FIG. 12I

EP 4 440 690 B1

645

Example three element resonant tank of a
Therapeutic current control network

FIG. 12J

EP 4 440 690 B1

Example three element resonant tank of a
Therapeutic current control network

FIG. 12K

EP 4 440 690 B1

Example three element resonant tank of a
Therapeutic current control network

## FIG. 12L

EP 4 440 690 B1

660

Example three element resonant tank of a
Therapeutic current control network

FIG. 12M

665

Example three element resonant tank of a
Therapeutic current control network

FIG. 12N

EP 4 440 690 B1

670

Example three element resonant tank of a
Therapeutic current control network

FIG. 12O

EP 4 440 690 B1

675

Example three element resonant tank of a
Therapeutic current control network

FIG. 12P

680

Example three element resonant tank of a
Therapeutic current control network

FIG. 12Q

EP 4 440 690 B1

685

Example two element resonant tank of a
therapeutic current control network

$C_R$

$L_R$

FIG. 12R

690

# Example two element resonant tank of a therapeutic current control network

# FIG. 12S

EP 4 440 690 B1

691

Example two element resonant tank of a
therapeutic current control network

$L_R$

$C_R$

FIG. 12T

Example two element resonant tank of a
therapeutic current control network

FIG. 12U

693

An example of a four element of a therapeutic
current control network

**LCLC resonant tank**

FIG. 12V

EP 4 440 690 B1

## FIG. 13

1000

1002

Battery

Energy storage Capacitor

1004

1006

Energy "dump" at resistor(s)

EP 4 440 690 B1

1014

Capacitor charging / battery charging with bidirectionality

1008

Battery

Bidirectional charging control network

Energy storage Capacitor

1010

1012

1400

FIG. 14

FIG. 15

**REFERENCES CITED IN THE DESCRIPTION**

**Patent documents cited in the description**

- WO 2021242882 A1